# EUROPEAN PATENT APPLICATION

(11) **EP 2 535 406 A1**
(43) Date of publication of application: **19.12.2012**
(21) Application number: 12170602.2
(22) Date of filing: 23.07.2008
(51) Int. Cl.: C12N 9/22, C12N 15/90, A01K 67/00, A61K 38/46, A61K 48/00

(54) **Meganuclease variants cleaving a DNA target sequence from the human hemoglobin beta gene and uses thereof**

(30) Priority: 23.07.2007 WO PCT/IB2007/003169; 06.06.2008 WO PCT/IB2008/002524
(62) Divisional of application: 08807213.7
(71) Applicant: Cellectis, 75013 Paris (FR)
(72) Inventor: Perez-Michaut, Christophe, 75016 Paris (FR)
(74) Representative: Thomas, Dean

(57) **Abstract**

An I*-Cre*I variant, wherein one of the two I-*Cre*I monomers has at least two substitutions, one in each of the two functional subdomains of the LAGLIDADG core domain situated respectively from positions 26 to 40 and 44 to 77 of I-*Cre*I, said variant being able to cleave a DNA target sequence from the human beta globin gene. Use of said variant and derived products for the prevention and the treatment of pathological conditions caused by a mutation in the human beta globin gene (sickle cell disease, beta-thalassemia).

## Description

The invention relates to a meganuclease variant cleaving a DNA target sequence from the human hemoglobin beta gene, to a vector encoding said variant, to a cell, an animal or a plant modified by said vector and to the use of said meganuclease variant and derived products for genome therapy *ex vivo* (gene cell therapy), and genome engineering.

Hemoglobin (haemoglobin or Hb) molecules are responsible for carrying oxygen via the red blood cells, from the lungs to various parts of the body for use in cellular respiration. Mature haemoglobin (normal adult haemoglobin or Hb A) is a tetrameric protein composed of two types of proteins: two alpha chains and two beta chains. The haemoglobin beta chain is often called beta globin (β-globin). Each protein subunit of hemoglobin carries an iron-containing molecule called heme which is responsible for the oxygen binding. A complete hemoglobin protein is then capable of carrying four oxygen molecules at a time.

The gene which codes for the haemoglobin beta chain (HBB gene or beta globin (β-globin) gene) is located on the short (p) arm of chromosome 11 at position 15.5 (11p15.5). It is composed of 3 exons that are distributed over 1606 base pairs of genomic DNA (Accession number GenBank NC_000011.8 or NT_009237.17). The mRNA (626-bp; accession number GenBank NM_00518.4) is translated into the 147-amino acid sequence of the HBB polypeptide chain [Accession number GenBank NP_00509.1; Online Mendelian Inheritance in Man, OMIM (TM). Johns Hopkins University, Baltimore, MD. MIM No.141900 (November 6, 2001)].

Hundreds of alterations have been identified in the HBB gene. Some of these changes result in the production of abnormal forms of hemoglobin, often designated by letters of the alphabet and sometimes also by a name. These abnormal forms of haemoglobin cause sickle cell diseases (sickle cell syndromes or sickle cell anemia syndromes) that include sickle cell anemia (HbSS), hemoglobin SC (HbSC) and hemoglobin SE (HbSE), (Park, K.W., Int. Anesthesiol. Clin., 2004, 42, 77-93; Stuart, M.J. and Nagel R.L., Lancet, 2004, 364, 1343-1360). Other conditions, called beta thalassemias are caused by many genetic mutations that abolish or reduce production of the beta globin subunit of hemoglobin. Hemoglobin sickle-beta thalassemia (HbSBetaThal) is caused when mutations that produce hemoglobin S and beta thalassemia occur together.

Sickle cell diseases are a group of inherited hemoglobin disorders characterized by chronic hemolytic anemia, an increased susceptibility to infections, organ damage, and vascular occlusion causing both acute and chronic pain (Ashley-Koch et al., American Journal of Epidemiology, 2000, 151, 839-845; The metabolic & molecular bases of inherited disease, 8th Ed., Scriver, C.R., c2001: p4571-4636, McGraw-Hill, New York*).* It is estimated that 70,000 Americans of different ethnic backgrounds have sickle cell disease, and sickle cell syndromes are present in 1 in 400 African Americans. Populations from the Mediterranean basin, the Middle East, and India have also a high frequency of the disease.

The most common beta haemoglobin variant resulting in sickle cell disease is hemoglobin S or HbS which involves the substitution of the glutamic acid at position 6 of the beta globin chain with a valine (E6V).

In sickle cell anemia (HbSS), a common form of sickle cell disease, hemoglobin S or HbS replaces both beta hemoglobin subunits in haemoglobin (Ashley-Koch et al., Am. J. Epidemiol. 2000, 151, 839-845; Sickle cell disease, Sergeant G., Oxford University Press: Oxford, 1985*).* The mutation causes the abnormal HbS subunits to stick together and form long, rigid molecules. The rigid HbS molecules bend red blood cells into a sickle shape which die prematurely. Therefore the mutation leads to a shortage of red blood cells (anemia). The sickle-shaped cells can also block small blood vessels, causing pain and organ damage.

In other types of sickle cell disease, just one beta hemoglobin subunit is replaced with hemoglobin S. The other beta hemoglobin subunit is replaced with a different abnormal variant, such as hemoglobin C or hemoglobin E. For example, in the hemoglobin SC (HbSC) disease, the beta hemoglobin subunits are replaced by hemoglobin S and hemoglobin C. The severity of this disorder is quite variable, but it can be as severe as sickle cell anemia. Hemoglobin C (HbC) is more common in people of West African descent than in other populations and results from the substitution of the glutamic acid at position 6 by a lysine (E6K). Hemoglobin E (HbE) is a variant of hemoglobin found predominantly in the people of Southeast Asia. The mutated protein results from the substitution of the glutamic acid at position 26 by a lysine (E26K).

Defects in HBB are the cause of beta-thalassemia (Thein SL., Br. J. Haematol., 2004, 124, 264-274). It occurs mostly in Mediterranean and Southeast Asian populations. More than 200 HBB mutations that cause beta thalassemia have been identified. Most of the mutations involve a change in a single nucleotide within the HBB gene, but insertion or deletion has also been reported. The hallmark of beta-thalassemia is an imbalance in globin-chain production in the adult HbA molecule. Absence of beta chain causes beta(0)-thalassemia, while reduced amounts of detectable beta globin causes beta(+)-thalassemia. In the severe forms of beta-thalassemia, the excess alpha globin chains accumulate in the developing erythroid precursors in the marrow. Their deposition leads to a vast increase in erythroid apoptosis that in turn causes ineffective erythropoiesis and severe microcytic hypochromic anemia.

The most common problem for sickle cell patients is the pain episode mainly in the extremities, back, chest, and abdomen (Dwarakanath, G.K. and Warfield C., Hosp. Pract., 1986, 64b; Fields H.L. and Levine J.D., West. J. Med. 1984, 347, 141-143; Vichinski et al., Am. J. Ped. Hematol. Oncol., 1982, 4, 328). The exact cause of the intense pain is unknown. It could be caused by the inflammatory response resulting from the obstruction and sludging of blood flow produced by sickled erythrocytes. Pain episodes are usually managed by the use of analgesic. However, despite the pain, the most serious live threatening complications are bacterial infection and splenic sequestration crisis. Bacterial infection is one of the main causes of death in patients with sickle cell disease (Barrett-Connor, E., Medicine, 1971, 50, 97-112), especially when children are under three years old. As splenic function is decreased or absent in sickle cell anemia, the patients have a high risk of infection within encapsulated organisms such as *Streptococcus pneumoniae, Haemophilus influenzae, Salmonella, Meningococcus,* and others [Powars et al., JAMA, 1981, 245, 1839-1842]. Thus, prophylactic penicillin is always instituted in the young child (Gaston et al., N. Engl. J. Med., 1986, 314, 1593-99). Splenic sequestration crisis is one of the most serious complications and is second cause of death in infants with sickle cell disease. This event usually occurs between the ages of four months and three years. During sequestration episodes sickle cells are trapped in the spleen, causing rapid fall in the hemoglobin level and enlargement of the spleen and others complications. The only treatment today consists of aggressive blood transfusions. Emergent splenectomy is occasionally required (Vichinsky et al., N Engl. J. Med., 1995, 222, 206-213; Haberkern et al., Blood, 1995, 86, 142a).

Daily administration of oral anti-sickling agent like hydroxyurea (Hydrea) is the first effective treatment documented to provide significant benefits in sickle cell disease (Steinberg MH., Scientific World Journal, 2002, 2, 1706-1728; Mehanna A.S., Curr. Med. Chem., 2001, 8, 79-88; Charache et al., N. Engl. J. Med., 1995, 332, 1317-1322). However, the long term benefits of hydroxyurea and toxicities are unknown. Thus, today, blood transfusions are still the best acute treatment for life threatening complications.

A second strategy would be the bone marrow transplantation. It has been used to successfully convert 27 patients in the United States from hemoglobin SS to normal AA or AS (depending on the phenotype of the related marrow donor). However, bone marrow transplantation is still an experimental therapy limited to patients who have enough complications from sickle cell disease to warrant the risk of death (10% mortality) and long-term complications from bone marrow transplantation and who also have a sibling with an identical HLA match (Walters et al., New Engl. J. Med., 1996, 355, 369-376; Walters et al., Biol. Blood Marrow Transplant., 1996, 2, 100-104).

Allogenic hematopoietic stem cell (HSC) transplantation is curative, but this therapeutic option is not available to the majority of patients. The transfer of a regulated β-globin gene in autologous HCSs thus represents a highly attractive alternative treatment (Tisdale J. and Sadelain, M., Semin. Hematol., 2001, 38, 382-392).

HBB could appear to be an excellent candidate for gene therapy: the gene is very small and has been characterized extensively; the disorder is recessively inherited and affects blood cells. However, initial attempt in 1980 failed, largely because of inefficient gene transfer and poor expression of the introduced β-globin genes. Although, much more knowledge was gained about HBB gene expression, there have been no subsequent gene therapy attempts. This is due to the problem of the very tight control of gene expression required into the desired cells. Indeed, the amount of β-globin protein produced must be equal to the amount of α-globin, since the imbalance between β-globin and α-globin chains would result in an α-thalassemia phenotype (Sadelain M. et al., Best. Pract. Res. Clin. Haematol., 2004, 17, 517-534). Nevertheless, *ex vivo* gene therapy seems to be very attractive, and tremendous achievement has been reached, at least in small animals as mice. Different humanized Sickle Cell Anemia (SCA) mouse models have been obtained by knock-out of the endogenous α- and β-globin genes and knock-in of the human α-, βS- and γ-globin gene (Paszty et al., Science 1997, 278, 876-878; Ryan et al., Science, 1997, 278, 873-876; Chang et al., Proc. Nat. Acad. Sci. USA, 1998, 95, 14886-14890), the sickle cell mice recapitulating the major features found in human sickle cell disease. The sickle cell phenotype could be reversed by integration of a lentivirus-borne human β-globin gene into bone marrow cells (May et al., Blood, 2002, 99, 1902-1908; Pawliuk et al., Science, 2001, 294, 2368-2371), providing a first step towards true human gene therapy.

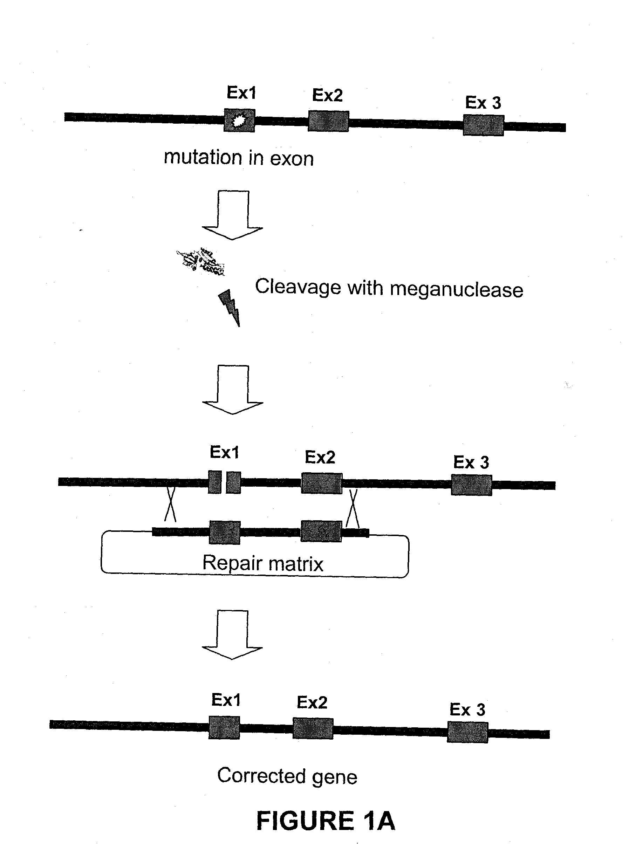

Targeted homologous recombination is another alternative that should bypass the problems raised by current approaches. Current gene therapy strategies are based on a complementation approach, wherein randomly inserted but functional extra copy of the gene provide for the function of the mutated endogenous copy. In contrast, homologous recombination should allow for the precise correction of mutations *in situ* (Figure 1A). Homologous recombination (HR), is a very conserved DNA maintenance pathway involved in the repair of DNA double-strand breaks (DSBs) and other DNA lesions (Rothstein, Methods Enzymol., 1983, 101, 202-211; Paques et al., Microbiol Mol Biol Rev, 1999, 63, 349-404; Sung et al., Nat. Rev. Mol. Cell. Biol., 2006, 7, 739-750) but it also underlies many biological phenomenon, such as the meiotic reassortiment of alleles in meiosis (Roeder, Genes Dev., 1997, 11, 2600-2621), mating type interconversion in yeast (Haber, Annu. Rev. Genet., 1998, 32, 561-599), and the "homing" of class I introns and inteins to novel alleles. HR usually promotes the exchange of genetic information between endogenous sequences, but in gene targeting experiments, it is used to promote exchange between an endogenous chromosomal sequence and an exogenous DNA construct. Basically, a DNA sharing homology with the targeted sequence was introduced into the cell's nucleus, and the endogenous homologous recombination machinery provides for the next steps (Figure 1A).

Homologous gene targeting strategies have been used to knock out endogenous genes (Capecchi, M.R., Science, 1989, 244, 1288-1292, Smithies, O., Nature Medicine, 2001, 7, 1083-1086) or knock-in exogenous sequences in the chromosome. It can as well be used for gene correction, and in principle, for the correction of mutations linked with monogenic diseases. However, this application is in fact difficult, due to the low efficiency of the process (10⁻⁶ to 10⁻⁹ of transfected cells). Recently, the gene targeting approach was used to correct the human β-S-globin gene in ES cells from model SCA mice (Chang et al., Proc. Nat. Acad. Sci. USA, 2006, 103, 1036-1040; Wu et al., Blood, 2006, 108, 1183-1188). However, classical selection scheme had to be used, due to the intrinsic low efficiency of the method.

In the last decade, several methods have been developed to enhance this yield. For example, chimeraplasty (de Semir, Nadal et al. 2003) and Small Fragment Homologous Replacement (Goncz, Colosimo et al. 2001; Bruscia, Sangiuolo et al. 2002; Sangiuolo, Bruscia et al. 2002; De Semir and Aran 2003) have both been used to try to correct CFTR mutations with various levels of success.

Another strategy to enhance the efficiency of recombination is to deliver a DNA double-strand break in the targeted locus, using meganucleases. Meganucleases are by definition sequence-specific endonucleases recognizing large sequences (Thierry, A. and B. Dujon, Nucleic Acids Res., 1992, 20, 5625-5631). They can cleave unique sites in living cells, thereby enhancing gene targeting by 1000-fold or more in the vicinity of the cleavage site (Puchta et al., Nucleic Acids Res., 1993, 21, 5034-5040 ; Rouet et al., Mol. Cell. Biol., 1994, 14, 8096-8106 ; Choulika et al., Mol. Cell. Biol., 1995, 15, 1968-1973; Puchta et al., Proc. Natl. Acad. Sci. U.S.A., 1996, 93, 5055-5060 ; Sargent et al., Mol. Cell. Biol., 1997, 17, 267-277; Cohen-Tannoudji et al., Mol. Cell. Biol., 1998, 18, 1444-1448; Donoho, et al., Mol. Cell. Biol., 1998, 18, 4070-4078; Elliott et al., Mol. Cell. Biol., 1998, 18, 93-101). Such meganucleases could be used to correct mutation responsible for monogenic inherited diseases.

The most accurate way to correct a genetic defect is to use a repair matrix with a non mutated copy of the gene, resulting in a reversion of the mutation. However, the efficiency of gene correction decreases as the distance between the mutation and the DSB grows, with a five-fold decrease by 200 bp of distance. Therefore, a given meganuclease can be used to correct only mutations in the vicinity of its DNA target.

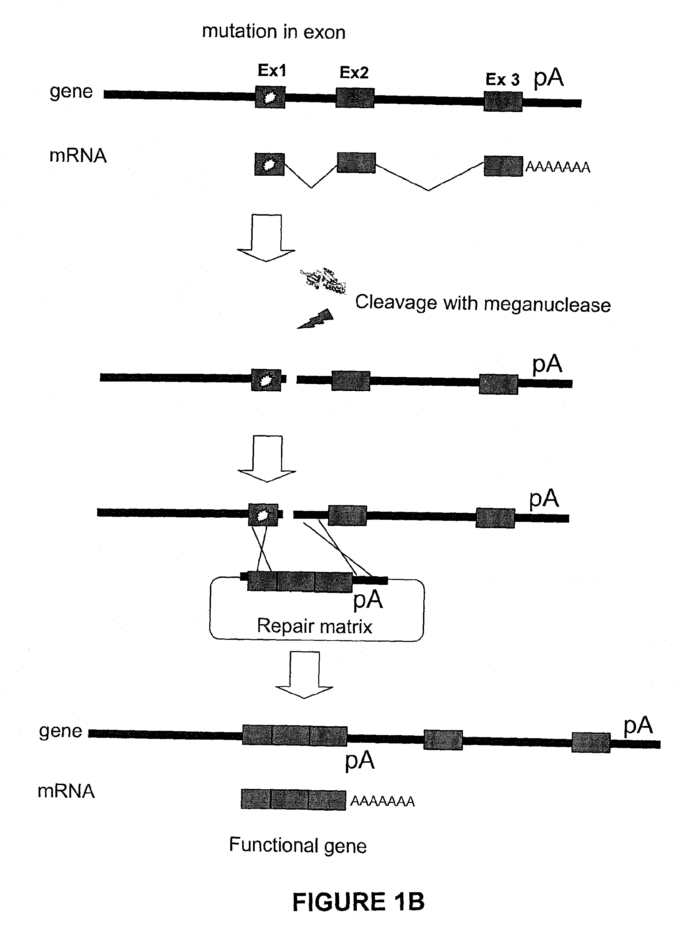

An alternative, termed "exon knock-in" is featured in Figure 1B. In this case, a meganuclease cleaving in the 5' part of the gene can be used to knock-in functional exonic sequences upstream of the deleterious mutation. Although this method places the transgene in its regular location, it also results in exons duplication, which impact on the long range remains to be evaluated. In addition, should naturally cis-acting elements be placed in an intron downstream of the cleavage, their immediate environment would be modified and their proper function would also need to be explored. However, this method has a tremendous advantage: a single meganuclease could be used for many different patients.

However, although several hundreds of natural meganucleases, also referred to as "homing endonucleases" have been identified (Chevalier, B.S. and B.L. Stoddard, Nucleic Acids Res., 2001, 29, 3757-3774), the repertoire of cleavable sequences is too limited to address the complexity of the genomes, and there is usually no cleavable site in a chosen gene. For example, there is no cleavage site for a known natural meganuclease in human HBB gene. Theoretically, the making of artificial sequence specific endonucleases with chosen specificities could alleviate this limit. Therefore, the making of meganucleases with tailored specificities is under intense investigation.

Recently, fusion of Zinc-Finger Proteins (ZFPs) with the catalytic domain of the *Fok*I, a class IIS restriction endonuclease, were used to make functional sequence-specific endonucleases (Smith et al., Nucleic Acids Res., 1999, 27, 674-681; Bibikova et al., Mol. Cell. Biol., 2001, 21, 289-297; Bibikova et al., Genetics, 2002, 161, 1169-1175; Bibikova et al., Science, 2003, 300, 764; Porteus, M.H. and D. Baltimore, Science, 2003, 300, 763-; Alwin et al., Mol. Ther., 2005, 12, 610-617; Urnov et al., Nature, 2005, 435, 646-651; Porteus, M.H., Mol. Ther., 2006, 13, 438-446). Such nucleases could recently be used for the engineering of the IL2RG gene in human cells from the lymphoid lineage (Urnov et al., Nature, 2005, 435, 646-651).

The binding specificity of Cys2-His2 type Zinc-Finger Proteins, is easy to manipulate, probably because they represent a simple (specificity driven by essentially four residues per finger), and modular system (Pabo et al., Annu. Rev. Biochem., 2001, 70, 313-340; Jamieson et al., Nat. Rev. Drug Discov., 2003, 2, 361-368). Studies from the Pabo laboratories resulted in a large repertoire of novel artificial ZFPs, able to bind most G/ANNG/ANNG/ANN sequences (Rebar, E.J. and C.O. Pabo, Science, 1994, 263, 671-673; Kim, J.S. and C.O. Pabo, Proc. Natl. Acad. Sci. U S A, 1998, 95, 2812-2817), Klug (Choo, Y. and A. Klug, Proc. Natl. Acad. Sci. USA, 1994, 91, 11163-11167; Isalan M. and A. Klug, Nat. Biotechnol., 2001, 19, 656-660) and Barbas (Choo, Y. and A. Klug, Proc. Natl. Acad. Sci. USA, 1994, 91, 11163-11167; Isalan M. and A. Klug, Nat. Biotechnol., 2001, 19, 656-660).

Nevertheless, ZFPs might have their limitations, especially for applications requiring a very high level of specificity, such as therapeutic applications. It was recently shown that *FokI* nuclease activity in fusion acts with either one recognition site or with two sites separated by varied distances via a DNA loop including in the presence of some DNA-binding defective mutants of *FokI* (Catto et al., Nucleic Acids Res., 2006, 34, 1711-1720). Thus, specificity might be very degenerate, as illustrated by toxicity in mammalian cells and Drosophila (Bibikova et al., Genetics, 2002, 161, 1169-1175; Bibikova et al., Science, 2003, 300, 764-.).

In the wild, meganucleases are essentially represented by homing endonucleases. Homing Endonucleases (HEs) are a widespread family of natural meganucleases including hundreds of proteins families (Chevalier, B.S. and B.L. Stoddard, Nucleic Acids Res., 2001, 29, 3757-3774). These proteins are encoded by mobile genetic elements which propagate by a process called "homing": the endonuclease cleaves a cognate allele from which the mobile element is absent, thereby stimulating a homologous recombination event that duplicates the mobile DNA into the recipient locus. Given their exceptional cleavage properties in terms of efficacy and specificity, they could represent ideal scaffold to derive novel, highly specific endonucleases.

HEs belong to four major families. The LAGLIDADG family, named after a conserved peptidic motif involved in the catalytic center, is the most widespread and the best characterized group. Seven structures are now available. Whereas most proteins from this family are monomeric and display two LAGLIDADG motifs, a few ones have only one motif, but dimerize to cleave palindromic or pseudo-palindromic target sequences.

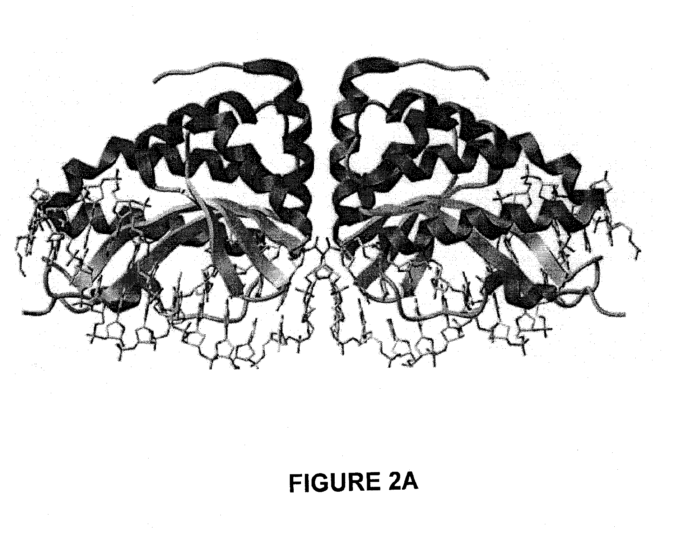

Although the LAGLIDADG peptide is the only conserved region among members of the family, these proteins share a very similar architecture (Figure 2A). The catalytic core is flanked by two DNA-binding domains with a perfect twofold symmetry for homodimers such as I-*Cre*I (Chevalier, et al., Nat. Struct. Biol., 2001, 8, 312-316) and I-*Mso*I (Chevalier et al., J. Mol. Biol., 2003, 329, 253-269) and with a pseudo symmetry for monomers such as I-*Sce*I (Moure et al., J. Mol. Biol., 2003, 334, 685-69, I-*DmoI* (Silva et al., J. Mol. Biol., 1999, 286, 1123-1136) or I-Ani*I* (Bolduc et al., Genes Dev., 2003, 17, 2875-2888). Both monomers, or both domains (for monomeric proteins) contribute to the catalytic core, organized around divalent cations. Just above the catalytic core, the two LAGLIDADG peptides play also an essential role in the dimerization interface. DNA binding depends on two typical saddle-shaped ββαββ folds, sitting on the DNA major groove. Other domains can be found, for example in inteins such as PI-*Pfu*I (Ichiyanagi et al., J. Mol. Biol., 2000, 300, 889-901) and PI-*Sce*I (Moure et al., Nat. Struct. Biol., 2002, 9, 764-770), which protein splicing domain is also involved in DNA binding.

The making of functional chimeric meganucleases, by fusing the N-terminal I-*Dmo*I domain with an I*-Cre*I monomer (Chevalier et al., Mol. Cell., 2002, 10, 895-905; Epinat et al., Nucleic Acids Res, 2003, 31, 2952-62; International PCT Applications WO 03/078619 and WO 2004/031346) have demonstrasted the plasticity of LAGLIDADG proteins.

Besides, different groups have used a semi-rational approach to locally alter the specificity of the I-*Cre*I (Seligman et al., Genetics, 1997, 147, 1653-1664; Sussman et al., J. Mol. Biol., 2004, 342, 31-41; International PCT Applications WO 2006/097784 and WO 2006/097853; Arnould et al., J. Mol. Biol., 2006, 355, 443-458; Rosen et al., Nucleic Acids Res., 2006, 34, 4791-4800 ; Smith et al. Nucleic Acids Res., 2006, 34, e149), I-*Sce*I (Doyon et al., J. Am. Chem. Soc., 2006, 128, 2477-2484), PI-*Sce*I (Gimble et al., J. Mol. Biol., 2003, 334, 993-1008) and I-*Mso*I (Ashworth et al., Nature, 2006, 441, 656-659).

In addition, hundreds of I-*Cre*I derivatives with locally altered specificity were engineered by combining the semi-rational approach and High Throughput Screening:
- Residues Q44, R68 and R70 or Q44, R68, D75 and 177 of I*-Cre*I were mutagenized and a collection of variants with altered specificity at positions ± 3 to 5 of the DNA target 5NNN DNA target) were identified by screening (International PCT Applications WO 2006/097784 and WO 2006/097853; Arnould et al., J. Mol. Biol., 2006, 355, 443-458; Smith et al., Nucleic Acids Res., 2006, 34, e149).
- Residues K28, N30 and Q38, N30, Y33 and Q38 or K28, Y33, Q38 and S40 of I-*Cre*I were mutagenized and a collection of variants with altered specificity at positions ± 8 to 10 of the DNA target (10NNN DNA target) were identified by screening (Smith et al., Nucleic Acids Res., 2006, 34, e149; International PCT Applications WO 2007/060495 and WO 2007/049156).

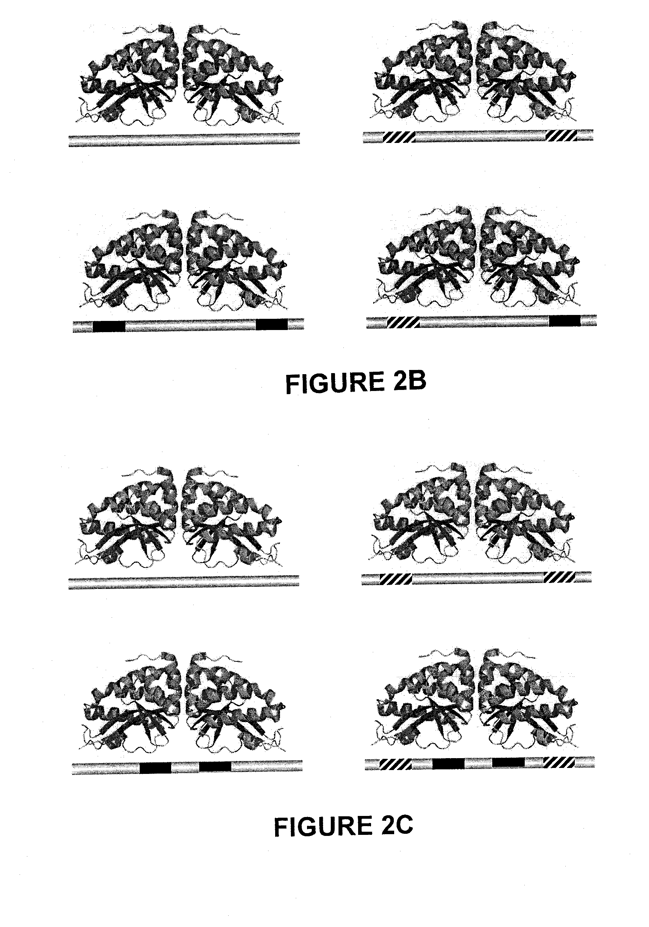

Two different variants were combined and assembled in a functional heterodimeric endonuclease able to cleave a chimeric target resulting from the fusion of a different half of each variant DNA target sequence (Arnould et al., precited; International PCT Applications WO 2006/097854 and WO 2007/034262), as illustrated on figure 2B. Interestingly, the novel proteins had kept proper folding and stability, high activity, and a narrow specificity.

Furthermore, residues 28 to 40 and 44 to 77 of I-CreI were shown to form two separable functional subdomains, able to bind distinct parts of a homing endonuclease half-site (Smith et al. Nucleic Acids Res., 2006, 34, e149; International PCT Applications WO 2007/049095 and WO 2007/057781).

The combination of mutations from the two subdomains of I-CreI within the same monomer allowed the design of novel chimeric molecules (homodimers) able to cleave a palindromic combined DNA target sequence comprising the nucleotides at positions ± 3 to 5 and ± 8 to 10 which are bound by each subdomain (Smith et al., Nucleic Acids Res., 2006, 34, e149; International PCT Applications WO 2007/060495 and WO 2007/049156), as illustrated on figure 2C.

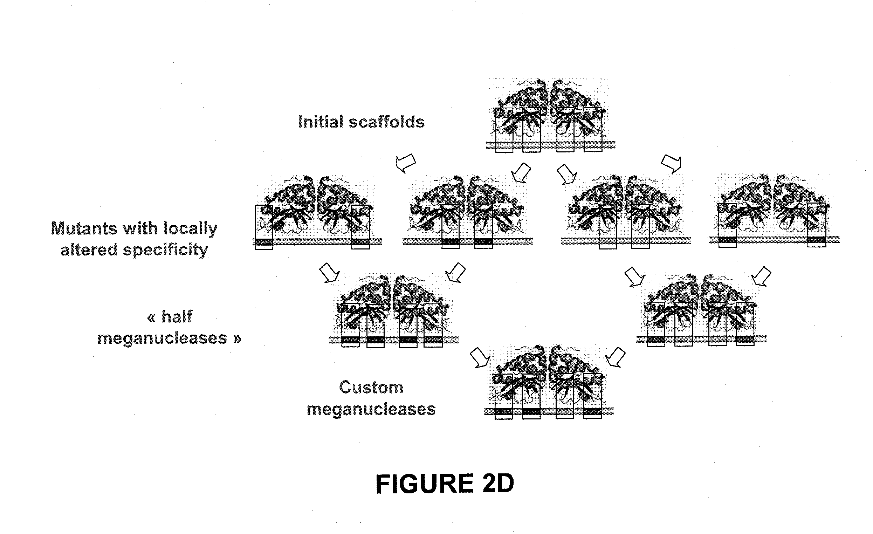

The combination of the two former steps allows a larger combinatorial approach, involving four different subdomains. The different subdomains can be modified separately and combined to obtain an entirely redesigned meganuclease variant (heterodimer or single-chain molecule) with chosen specificity, as illustrated on figure 2D. In a first step, couples of novel meganucleases are combined in new molecules ("half-meganucleases") cleaving palindromic targets derived from the target one wants to cleave. Then, the combination of such "half-meganuclease" can result in an heterodimeric species cleaving the target of interest. The assembly of four set of mutations into heterodimeric endonucleases cleaving a model target sequence or a sequence from the human RAG1and XPC genes have been described in Smith et al. (Nucleic Acids Res., 2006, 34, e149) and Arnould et al., (J. Mol. Biol., Epub 10 May, 2007), respectively.

These variants can be used to cleave genuine chromosomal sequences and have paved the way for novel perspectives in several fields, including gene therapy.

However, even though the base-pairs ±1 and ±2 do not display any contact with the protein, it has been shown that these positions are not devoid of content information (Chevalier et al., J. Mol. Biol., 2003, 329, 253-269), especially for the base-pair ±1 and could be a source of additional substrate specificity (Argast et al., J. Mol. Biol., 1998, 280, 345-353; Jurica et al., Mol. Cell., 1998, 2, 469-476; Chevalier, B.S. and B.L. Stoddard, Nucleic Acids Res., 2001, 29, 3757-3774). *In vitro* selection of cleavable I-*Cre*I target (Argast *et al.,* precited) randomly mutagenized, revealed the importance of these four base-pairs on protein binding and cleavage activity. It has been suggested that the network of ordered water molecules found in the active site was important for positioning the DNA target (Chevalier et al., Biochemistry, 2004, 43, 14015-14026). In addition, the extensive conformational changes that appear in this region upon I-*Cre*I binding suggest that the four central nucleotides could contribute to the substrate specificity, possibly by sequence dependent conformational preferences (Chevalier *et al.,* 2003, precited).

Thus, it was not clear if mutants identified on 10NNN and 5NNN DNA targets as homodimers cleaving a palindromic sequence with the four central nucleotides being gtac, would allow the design of new endonucleases that would cleave targets containing changes in the four central nucleotides.

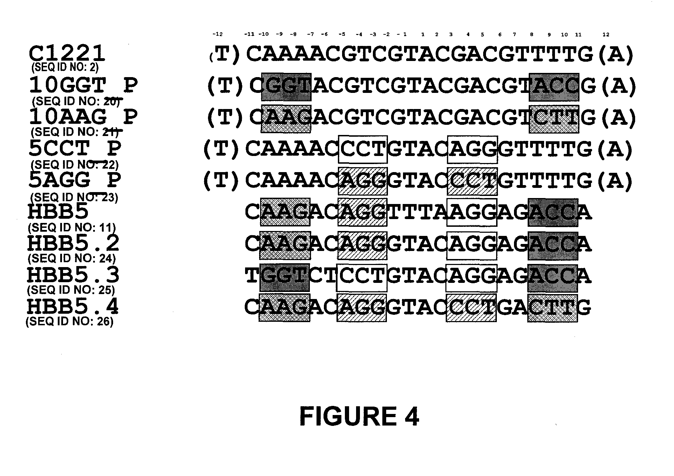

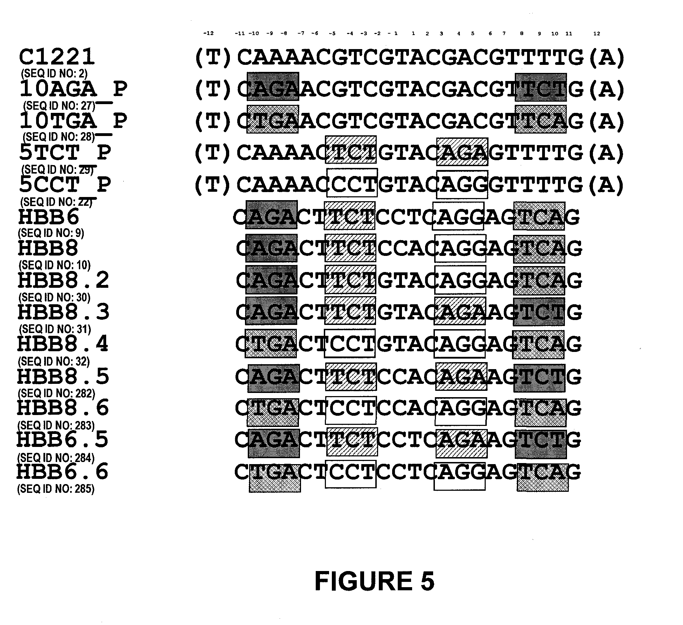

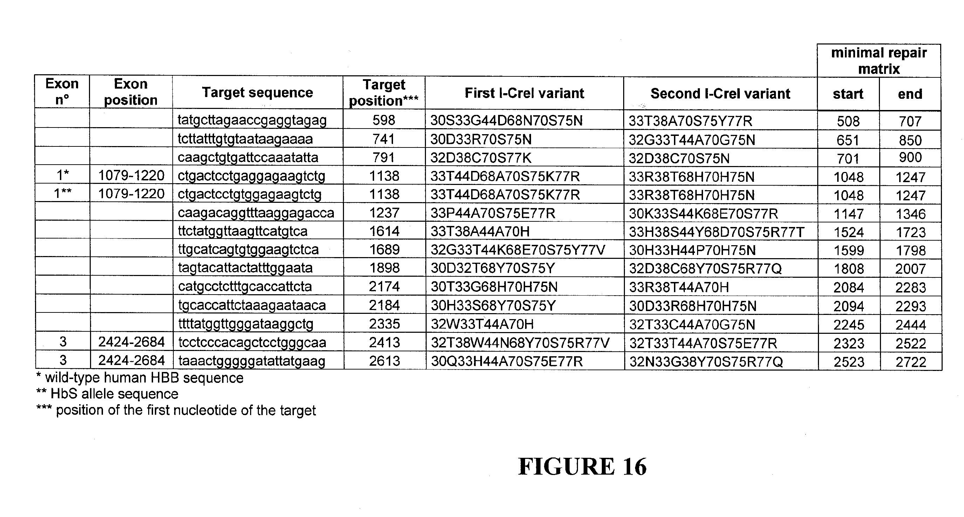

The Inventors have identified a series of DNA targets in the human beta globin gene that could be cleaved by I-*Cre*I variants (Table I and Figure 16). The combinatorial approach described in figure 2D was used to entirely redesign the DNA binding domain of the I-*Cre*I protein and thereby engineer novel meganucleases with fully engineered specificity, to cleave two DNA targets (HBB5 and HBB8) from the human HBB gene which differs from the I-*Cre*I C1221 22 bp palindromic site by 16 nucleotides including three (positions -2, +1, +2) out of the four central nucleotides (HBB5; Figure 4) and 15 nucleotides including 2 (positions +1, +2) out of the four central nucleotides (HBB8; Figure 5), respectively.

Even though the combined variants were initially identified towards nucleotides 10NNN and 5NNN respectively, and a strong impact of the four central nucleotides of the target on the activity of the engineered meganuclease was observed, functional meganucleases with a profound change in specificity were selected. Furthermore, the activity of the engineered protein could be significantly improved by random mutagenesis and screening, to compare with the activity of the I*-Cre*I protein.

The I-*Cre*I variants which are able to cleave a genomic DNA target from the human HBB gene can be used for genome therapy of sickle cell diseases and beta thalassemia and genome engineering at the beta globin locus (animal models and recombinant cells generation).

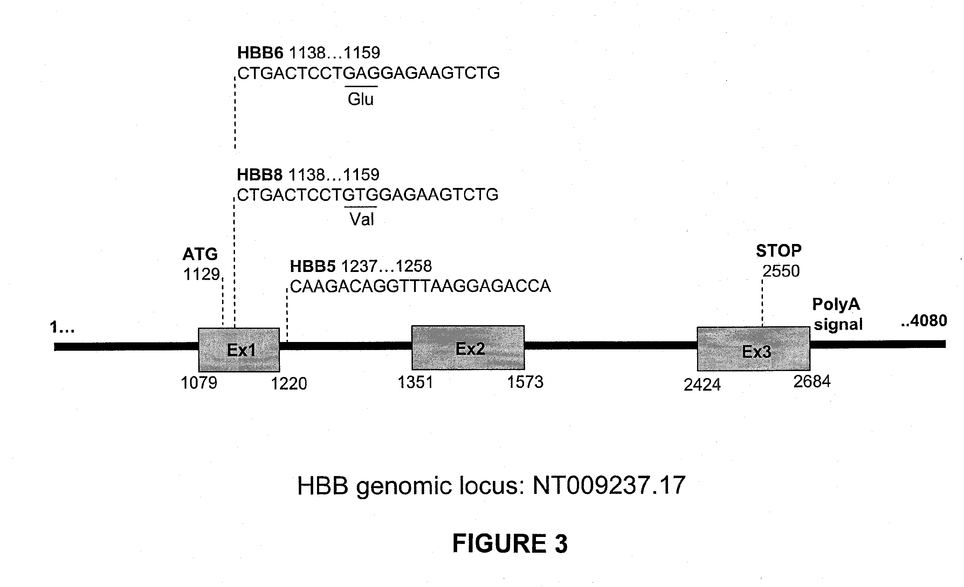

For example, the DNA target called HBB6, situated in the coding Exon 1 of HBB (positions 1138 to 1159 of SEQ ID NO: 4; Figure 3), overlaps the glutamic acid codon whose mutation is responsible for sickle cell anemia and HbSC and is close to the glutamic acid codon whose mutation is responsible for HbSE. In the HbS allele, which differs from the wild-type gene by an A to T transversion at position 1148 that changes the glutamic acid codon (GAG; positions 1147 to 1149 of SEQ ID NO: 4) to a valine codon (GTG), the HBB6 sequence is replaced with HBB8. The DNA target called HBB5 is located in the beginning of the intron 1 of HBB gene (positions 1237 to 1258 of SEQ ID NO: 4; Figure 3), close to the mutated glutamic acid codons responsible for sickle cell anemia (GTG codon at positions 1147 to 1149; E6V mutation), HbSC (AAG codon at positions 1147 to 1149; E6K mutation) and HbSE (AAG codon at positions 1207 to 1209; E6K mutation).

The meganucleases cleaving HBB8 or HBB5 could be used to correct the E6V mutation. Meganucleases cleaving HBB5 could also be used to correct other mutations at position 6 of the beta globin chain (HbC: E6K). In addition, meganucleases cleaving HBB5 or HBB6 could be used to correct any other amino acid mutation in the vicinity of the cleavage site (E26K). Since the efficiency of gene correction decreases when the distance to the DSB increases (Elliott et al., Mol. Cell. Biol., 1998, 18, 93-101), this strategy would be most efficient with mutations located within 500 bp of the cleavage site. Alternatively, the same meganucleases could be used to knock-in exonic sequences that would restore a functional HBB gene at the HBB locus (Figure 1B). This strategy could be used for any mutation downstream of the cleavage site.

The invention relates to an I-*Cre*I variant wherein at least one of the two I-CreI monomers has at least two substitutions, one in each of the two functional subdomains of the LAGLIDADG core domain situated respectively from positions 26 to 40 and 44 to 77 of I-*Cre*I, and is able to cleave a DNA target sequence from the human beta globin gene.

The cleavage activity of the variant according to the invention may be measured by any well-known, *in vitro* or *in vivo* cleavage assay, such as those described in the International PCT Application WO 2004/067736; Epinat et al., Nucleic Acids Res., 2003, 31, 2952-2962; Chames et al., Nucleic Acids Res., 2005, 33, e178 and Arnould et al., J. Mol. Biol., 2006, 355, 443-458. For example, the cleavage activity of the variant of the invention may be measured by a direct repeat recombination assay, in yeast or mammalian cells, using a reporter vector. The reporter vector comprises two truncated, non-functional copies of a reporter gene (direct repeats) and the genomic DNA target sequence within the intervening sequence, cloned in a yeast or a mammalian expression vector. Expression of the variant results in a functional endonuclease which is able to cleave the genomic DNA target sequence. This cleavage induces homologous recombination between the direct repeats, resulting in a functional reporter gene, whose expression can be monitored by appropriate assay.

### Definitions

- The admitted numerals of positions of both HBB and meganuclease variants omit counting the first methionine; this explains the discrepancy observed between the usually used numeral annotation and the effective positions observed in the herewith attached sequence listing; more precisely:
   . as regards HBB numeration, it is in agreement with the numeration given in the OMIM^{™} database; therefore, the mutations E6K and E26K correspond in the reference sequence (GenBank NP_00509.1 or SEQ ID NO:150 of the herewith attached sequence listing) to mutations in positions 7 and 27 respectively; and
   . as regards meganuclease numeration, it is in agreement with I-*Cre*I pdb accession code Ig9y.
- Amino acid residues in a polypeptide sequence are designated herein according to the one-letter code, in which, for example, Q means Gln or Glutamine residue, R means Arg or Arginine residue and D means Asp or Aspartic acid residue.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "meganuclease", is intended an endonuclease having a double-stranded DNA target sequence of 12 to 45 bp. Said meganuclease is either a dimeric enzyme, wherein each domain is on a monomer or a monomeric enzyme comprising the two domains on a single polypeptide.
- by "meganuclease domain" is intended the region which interacts with one half of the DNA target of a meganuclease and is able to associate with the other domain of the same meganuclease which interacts with the other half of the DNA target to form a functional meganuclease able to cleave said DNA target.
- by "meganuclease variant" or "variant" is intented a meganuclease obtained by replacement of at least one residue in the amino acid sequence of the wild-type meganuclease (natural meganuclease) with a different amino acid.
- by "functional variant" is intended a variant which is able to cleave a DNA target sequence, preferably said target is a new target which is not cleaved by the parent meganuclease. For example, such variants have amino acid variation at positions contacting the DNA target sequence or interacting directly or indirectly with said DNA target.
- by "I-*Cre*I" is intended the wild-type I-*Cre*I having the sequence SWISSPROT P05725 or pdb accession code 1g9y, corresponding respectively to the sequence SEQ ID NO: 1 and SEQ ID NO: 178 in the sequence listing.
- by "I-*Cre*I variant with novel specificity" is intended a variant having a pattern of cleaved targets different from that of the parent meganuclease. The terms "novel specificity", "modified specificity", "novel cleavage specificity", "novel substrate specificity" which are equivalent and used indifferently, refer to the specificity of the variant towards the nucleotides of the DNA target sequence.
- by "I-*Cre*I site" is intended a 22 to 24 bp double-stranded DNA sequence which is cleaved by I-*Cre*I. I-*Cre*I sites include the wild-type (natural) non-palindromic I-*Cre*I homing site and the derived palindromic sequences such as the sequence 5'- t-₁₂c₁₁ a-₁₀a-₉a-₈a-₇c-₆g-₅t₋₅c-₃g-₂t₋1ₐ+₁c+₂g+₃a+₄c+₅g+₆t+₇t+₈t+₉t+₁₀g+₁₁a+₁₂ (SEQ ID NO : 2), also called C1221 (Figure 4).
- by "domain" or "core domain" is intended the "LAGLIDADG homing endonuclease core domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁β₂β₃β₄) folded in an antiparallel beta-sheet which interacts with one half of the DNA target. This domain is able to associate with another LAGLIDADG homing endonuclease core domain which interacts with the other half of the DNA target to form a functional endonuclease able to cleave said DNA target. For example, in the case of the dimeric homing endonuclease I-CreI (163 amino acids), the LAGLIDADG homing endonuclease core domain corresponds to the residues 6 to 94.
- by "subdomain" is intended the region of a LAGLIDADG homing endonuclease core domain which interacts with a distinct part of a homing endonuclease DNA target half-site.
- by "beta-hairpin" is intended two consecutive beta-strands of the antiparallel beta-sheet of a LAGLIDADG homing endonuclease core domain (β₁β₂ or β₃β₄) which are connected by a loop or a turn,
- by "single-chain meganuclease", "single-chain chimeric meganuclease", "single-chain meganuclease derivative", "single-chain chimeric meganuclease derivative" or "single-chain derivative" is intended a meganuclease comprising two LAGLIDADG homing endonuclease domains or core domains linked by a peptidic spacer. The single-chain meganuclease is able to cleave a chimeric DNA target sequence comprising one different half of each parent meganuclease target sequence.
- by "DNA target", "DNA target sequence", "target sequence" , "target-site", "target" , "site"; "site of interest"; "recognition site", "recognition sequence", "homing recognition site", "homing site", "cleavage site" is intended a 20 to 24 bp double-stranded palindromic, partially palindromic (pseudo-palindromic) or non-palindromic polynucleotide sequence that is recognized and cleaved by a LAGLIDADG homing endonuclease such as *I-CreI,* or a variant, or a single-chain chimeric meganuclease derived from *I-CreI.* These terms refer to a distinct DNA location, preferably a genomic location, at which a double stranded break (cleavage) is to be induced by the meganuclease. The DNA target is defined by the 5' to 3' sequence of one strand of the double-stranded polynucleotide, as indicated above for C1221. Cleavage of the DNA target occurs at the nucleotides at positions +2 and -2, respectively for the sense and the antisense strand. Unless otherwiwe indicated, the position at which cleavage of the DNA target by an I-Cre I meganuclease variant occurs, corresponds to the cleavage site on the sense strand of the DNA target.
- by "DNA target half-site", "half cleavage site" or half-site" is intended the portion of the DNA target which is bound by each LAGLIDADG homing endonuclease core domain.
- by "chimeric DNA target" or "hybrid DNA target" is intended the fusion of a different half of two parent meganuclease target sequences. In addition at least one half of said target may comprise the combination of nucleotides which are bound by at least two separate subdomains (combined DNA target).
- by "beta globin gene" or "HBB gene" is intended the human beta globin gene located on the short (p) arm of chromosome 11 at position 15.5 (11p15.5). The invention includes the normal (wild-type HBB) and the mutated HBB genes (mutant HBB; HBB allele), in particular the mutants responsible for sickle cell diseases (HbS allele, βS-globin gene or sickle globin gene) and beta-thalassemias. The HBB gene (1606 bp) corresponds to positions 4033937 to 4035542 on the reverse complemented strand of the sequence accession number GenBank NT_009237.17. It comprises three exons (Exon 1: positions 1 to 142; Exon 2: positions 273 to 495; Exon 3: positions 1346 to 1606). The ORF which is from position 51 (Exon 1) to positions 1474 (Exon 3), is flanked by two untranslated regions. The wild-type HBB sequence (SEQ ID NO: 3) is included in a 4080 bp fragment (SEQ ID NO: 4) corresponding to positions 4032541 to 4036620 on the reverse complemented strand of the sequence accession number NT_009237.17. The HBB gene sequence corresponds to positions 1079 to 2684 of SEQ ID NO: 4, Exon 1 to positions 1079 to 1220, Exon 2 to positions 1351 to 1573, Exon 3 to positions 2424 to 2684 and the ORF to positions 1129 to 2552 (Figure 3). The HBB mRNA corresponds to the sequence accession number GenBank NM_00518.4 (SEQ ID NO: 149). The beta globin protein corresponds to the sequence accession number GenBank NP_00509.1 (SEQ ID NO: 150).
- by "DNA target sequence from the beta globin gene", "genomic DNA target sequence", " genomic DNA cleavage site", "genomic DNA target" or "genomic target" is intended a 20 to 24 bp sequence of the beta globin gene of a mammal which is recognized and cleaved by a meganuclease variant or a single-chain chimeric meganuclease derivative.
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99 %.
- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and others such as for example: cows, pigs and horses.
- by mutation is intended the substitution, deletion, insertion of one or more nucleotides/amino acids in a polynucleotide (cDNA, gene) or a polypeptide sequence. Said mutation can affect the coding sequence of a gene or its regulatory sequence. It may also affect the structure of the genomic sequence or the structure/stability of the encoded mRNA.

The variant according to the present invention may be an homodimer or an heterodimer. Preferably, both monomers of the heterodimer are mutated at positions 26 to 40 and/or 44 to 77. More preferably, both monomers have different substitutions both at positions 26 to 40 and 44 to 77 of I*-Cre*I.

In a preferred embodiment of said variant, said substitution(s) in the subdomain situated from positions 44 to 77 of I-*Cre*I are at positions 44, 68, 70, 75 and/or 77.

In another preferred embodiment of said variant, said substitution(s) in the subdomain situated from positions 26 to 40 of I-*Cre*I are at positions 26, 28, 30, 32, 33, 38 and/or 40.

In another preferred embodiment of said variant, it comprises one or more mutations at positions of other amino acid residues that contact the DNA target sequence or interact with the DNA backbone or with the nucleotide bases, directly or via a water molecule; these residues are well-known in the art (Jurica et al., Molecular Cell., 1998, 2, 469-476; Chevalier et al., J. Mol. Biol., 2003, 329, 253-269). In particular, additional substitutions may be introduced at positions contacting the phosphate backbone, for example in the final C-terminal loop (positions 137 to 143; Prieto et al., Nucleic Acids Res., Epub 22 April 2007). Preferably said residues are involved in binding and cleavage of said DNA cleavage site. More preferably, said residues are at positions 138, 139, 142 or 143 of I*-Cre*I. Two residues may be mutated in one variant provided that each mutation is in a different pair of residues chosen from the pair of residues at positions 138 and 139 and the pair of residues at positions 142 and 143. The mutations which are introduced modify the interaction(s) of said amino acid(s) of the final C-terminal loop with the phosphate backbone of the I-*Cre*I site. Preferably, the residue at position 138 or 139 is substituted by an hydrophobic amino acid to avoid the formation of hydrogen bonds with the phosphate backbone of the DNA cleavage site. For example, the residue at position 138 is substituted by an alanine or the residue at position 139 is substituted by a methionine. The residue at position 142 or 143 is advantageously substituted by a small amino acid, for example a glycine, to decrease the size of the side chains of these amino acid residues. More, preferably, said substitution in the final C-terminal loop modifies the specificity of the variant towards the nucleotide at positions ± 1 to 2, ± 6 to 7 and/or ± 11 to 12 of the I-CreI site.

In another preferred embodiment of said variant, it comprises one or more additional mutations that improve the binding and/or the cleavage properties of the variant towards the DNA target sequence from the human beta globin gene.

The additional residues which are mutated may be on the entire I-CreI sequence, and in particular in the C-terminal half of I-*Cre*I (positions 80 to 163). Both I-*Cre*I monomers are advantageously mutated; the mutation(s) in each monomer may be identical or different. For example, the variant comprises one or more additional substitutions at positions: 4, 7, 12, 16, 19, 24, 34, 43, 49, 54, 58, 60, 64, 66, 79, 80, 81, 82, 85, 86, 87, 92, 93, 94, 96, 99, 100, 103, 105, 109, 111, 117, 120, 121, 125, 129, 131, 132, 139, 140, 147, 151, 152, 155, 159, 160, 161, 162 and 163. Said substitutions are advantageously selected from the group consisting of: K4E, K7R, Y12H, F16L, G19S, G19A, 124V, K34R, F43L, T49A, F54L, L58Q, D60N, D60G, V64I, Y66H, S79G, E80G, E80K, I81T, K82R, K82E, H85R, N86S, F87L, Q92R, P93A, F94L, K96R, Q99R, K100R, N103S, V105A, V1051,1109V, Q111H, E117G, D120G, K121R, K121E, V125A, V129A, Q131R, I132V, K139R, T140A, T147A, V151M, L152Q, L155P, K159R, K159E, K160E, S161P, S162P and P163S. Preferably, the variant comprises at least one substitution selected from the group consisting of: G19S, F54L, E80K, F87L, V105A and I132V. More preferably, the variant comprises at least the G19S and E80K substitutions or the F87L and E80K substitutions.

According to a more preferred embodiment of said variant, said additional mutation further impairs the formation of a functional homodimer. More preferably, said mutation is the G19S mutation. The G19S mutation is advantageously introduced in one of the two monomers of an heterodimeric I-CreI variant, so as to obtain a meganuclease having enhanced cleavage activity and enhanced cleavage specificity. In addition, to enhance the cleavage specificity further, the other monomer may carry a distinct mutation that impairs the formation of a functional homodimer or favors the formation of the heterodimer.

In another preferred embodiment of said variant, said substitutions are replacement of the initial amino acids with amino acids selected from the group consisting of: A, D, E, G, H, K, N, P, Q, R, S, T, Y, C, V, L and W.

The variant of the invention may be derived from the wil-type I-CreI (SEQ ID NO: 1 or SEQ ID NO: 178) or an I-CreI scaffold protein having at least 85 % identity, preferably at least 90 % identity, more preferably at least 95 % identity with SEQ ID NO: 178, such as the scaffold SEQ ID NO: 5 (167 amino acids) having the insertion of an alanine at position 2, the D75N substitution, and the insertion of AAD at the C-terminus (positions 164 to 166) of the I-*Cre*I sequence.

In addition, the variants of the invention may include one or more residues inserted at the NH₂ terminus and/or COOH terminus of the sequence. For example, a tag (epitope or polyhistidine sequence) is introduced at the NH₂ terminus and/or COOH terminus; said tag is useful for the detection and/or the purification of said variant. For example, the variant may have the AAD or ATD sequence inserted at its C-terminus.

The variant may also comprise a nuclear localization signal (NLS); said NLS is useful for the importation of said variant into the cell nucleus. The NLS may be inserted just after the first methionine of the variant or just after an N-terminal tag.

The variant according to the present invention may be an homodimer which is able to cleave a palindromic or pseudo-palindromic DNA target sequence.

Alternatively, said variant is an heterodimer, resulting from the association of a first and a second monomer having different substitutions at positions 26 to 40 and 44 to 77 of I-*Cre*I, said heterodimer being able to cleave a non-palindromic DNA target sequence from the human beta globin gene.

Each monomer (first monomer and second monomer) of the heterodimeric variant according to the present invention may be named with a letter code, after the eleven residues at positions 28, 30, 32, 33, 38, 40, 44, 68 and 70, 75 and 77 and the additional residues which are mutated, as indicated above. For example, KTSHRS/KYSDT + 120G or 28K30T32S33H38R40S /44K68Y70S75D77T + 120G stands for I*-Cre*I K28, T30, S32 , H33 , R38, S40/ K44, Y68, S70, D75, T77 and G120.

The DNA target sequence which is cleaved by said variant may be in an exon or in an intron of the human beta globin gene.

In another preferred embodiment of said variant, said DNA target sequence is selected from the group consisting of the sequences SEQ ID NO: 6 to 19 (Figure 16 and TableI).

**Table I: Human HBB gene target sequences**

| **SEQ ID NO:** | **Target sequence** | **Target position*** | **Target location** |
|---|---|---|---|
| **6** | tatgcttagaaccgaggtagag | 598 | **5'flanking sequence** |
| **7** | tcttatttgtgtaataaqaaaa | 741 | **5'flanking sequence** |
| **8** | caagctgtgattccaaatatta | 791 | **5'flanking sequence** |
| **9** | ctgactcctgaggagaagtctg | 1138 | **Exon 1 (wild type sequence)** |
| **10** | ctgactcctgtggagaagtctg | 1138 | **Exon 1 (mutated sequence)** |
| **11** | caagacaggtttaaggagacca | 1237 | **Intron** 1 |
| **12** | ttctatggttaagttcatgtca | 1614 | **Intron 2** |
| **13** | ttgcatcagtgtggaagtctca | 1689 | **Intron 2** |
| **14** | tagtacattactatttggaata | 1898 | **Intron 2** |
| **95** | catgcctctttgcaccattcta | 2174 | **Intron 2** |
| **16** | tgcaccattctaaagaataaca | 2184 | **Intron 2** |
| **17** | ttttatggttggataaggctg | 2335 | **Intron 2** |
| **18** | tcctcccacagctcctaggcaa | 2413 | **Intron 2** |
| **19** | taaactgggggatattatgaag | 2613 | **Exon 3** |

| | | | |
|---|---|---|---|
| * the indicated position which is that of the first nucleotide of the target is indicated by reference to the sequence SEQ ID NO: 4 | | | |

More preferably, the monomers of the I-*Cre*I variant have at least the following substitutions, respectively for the first and the second I-*Cre*I monomer:
* N30S, Y33G, Q44D, R68N, R70S and D75N (first monomer), and Y33T, Q38A, R70S, D75Y and I77R (second monomer); this variant cleaves the HBB target SEQ ID NO: 6 that is located in the human HBB gene 5' flanking sequence (figure 16 and Table I),
* N30D, Y33R, R70S and D75N (first monomer), and S32G, Y33T, Q44A, R70G and D75N (second monomer); this variant cleaves the HBB target SEQ ID NO: 7 that is located in the human HBB gene 5' flanking sequence (figure 16 and Table I),
* S32D, Q38C, R70S and I77K (first monomer), and S32D, Q38C, R70S and D75N (second monomer); this variant cleaves the HBB target SEQ ID NO: 8 that is located in the human HBB gene 5' flanking sequence (figure 16 and Table I),
* Y33T, Q44D, R68A, R70S, D75K and I77R (first monomer), and Y33R, Q38T, R68H, R70H and D75N (second monomer); this variant cleaves both HBB6 (SEQ ID NO: 9) and HBB8 (SEQ ID NO: 10) targets (figures 3 and 16 and Table I). The HBB6 and HBB8 targets are located in Exon 1 and overlap respectively, the normal (GAG; glutamic acid, E6) and mutated (GTG; A to T transversion resulting in a glutamic acid to valine substitution (E6V) causing sickle cell anemia) codon at position 6 of the human beta globin chain.
* The following variants (figure 16 and Tables XVI, XVIII, XX and XXI) cleave the HBB8 (SEQ ID NO: 10) target (figures 3 and 16 and Table I), and do not cleave, or cleave with a lower efficiency, the HBB6 target. The HBB8 target is located in Exon 1 and overlaps the mutated codon at position 6 of the human beta globin chain that is responsible for sickle cell anemia:
   - a first monomer having K at position 28, N at position 30, S, E or T at position 32, T, C, S or V at position 33, S, Q, R, H at position 38, S or Q at position 40, K, R, D, N or H at position 44, Y, A or E at position 68, S at position 70, D, K or N at position 75, and R or Q at position 77. Preferably, the residues at positions 28, 30, 32, 33, 38 and 40 are selected from the group consisting of: KNSTSS, KNSSSS, KNETQS, KNTCQS, KNSCQS, KNSTRQ, KNSVRQ, KNSVHQ, KNSCHS, KNSSRS, KNSTQS and KNSVHS, and the residues at positions 44, 68, 70, 75 and 77 are selected from the group consisting of:KASDR, RYSNQ, HYSNR, NESNR and DASKR. More preferably, the first monomer is selected from the group consisting of: KNSTSS/RYSNQ, KNTCQS/RYSNQ, KNTCQS/HYSNR, KNSCHS/RYSNQ, KNSVHS/RYSNQ, KNSTRQ/NESNR, KNETQS/DASKR, KNTCQS/DASKR, KNSCHS/DASKR, KNSCQS/DASKR, KNSSSS/KASDR, KNSSRS/DASKR, KNSTRQ/DASKR, KNSVRQ/DASKR, KNSTQS/DASKR and KNSVHQ/DASKR. The first monomer comprises advantageously at least one first additional mutation selected from the group consisting of G19S, F54L, E80K, F87L, V105A and I132V, and eventually a second additional mutation selected from the group consisting of: K7R, K121R and Q131R. Examples of such variants are presented in Table XVI (SEQ ID NO: 104, 105, 111, 113, 114, 118, 121), Table XX (SEQ ID NO: 257 to 276), Table XXI (SEQ ID NO: 286) and figure 16 (SEQ ID NO: 126).
   - a second monomer having K at position 28, N, T or Q at position 30, T or S at position 32, G, H, T, C, Y or R at position 33, K, R, T or Q at position 38, S at position 40, Q or K at position 44, A, S, H or Y at position 68, S or H at position 70, N at position 75, and R, Q, Y or I at position 77. Preferably, the residues at positions 28, 30, 32, 33, 38 and 40 are selected from the group consisting of: KNSGKS, KTSHRS, KNSTRS, KNSRTS, KNSGRS, KNSCRS, KQTYRS and KTSRQS, and the residues at positions 44, 68, 70, 75 and 77 are selected from the group consisting of: QASNR, KYSNY, KYSNQ, QYSNR, KYSNR, KYSNI, QHHNI and KSSNY. More preferably, the second monomer is selected from the group consisting of: KNSGKS/QASNR, KTSHRS/KYSNY, KNSTRS/KYSNY, KNSGRS/KYSNY, KNSCRS/KYSNQ, KNSGKS/KYSNY, KNSGKS/QYSNR, KNSGKS/KYSNR, KNSGKS/KYSNQ, KQTYRS/KYSNI, KQTYRS/KYSNY, KQTYRS/QASNR, KQTYRS/KYSNQ, KNSRTS/QHHNI and KTSRQS/KSSNY. The second monomer comprises advantageously at least one first additional mutation selected from the group consisting of : G19S, F54L, E80K, F87L, V105A and 1132V, and eventually a second additional mutation selected from the group consisting of K4E, Y12H, L58Q, D60N, V64I, H85R, P93A, E117G, V129A and L152Q. Examples of such variants are presented in Table XVI (SEQ ID NO: 82, 84, 85, 88, 94, 103), Table XVIII (SEQ ID NO: 231 to 255), Table XXI (SEQ ID NO: 287 to 291) and figure 16 (SEQ ID NO: 139.

Examples of such variants which cleave the HBB8 target and do not cleave, or cleave with a lower efficiency, the HBB6 target are presented in Tables XVI, XVIII, XX and XXI. For example, the variants presented in Table XVI have the following substitutions:
- Y33T, Q38S, Q44R, R68Y, R70S, D75N and I77Q (KNSTSS/RYSNQ) or S32E, Y33T, Q44D, R68A, R70S, D75K and I77R (KNETQS/DASKR; first monomer), and Y33C, Q38R, Q44K, R68Y, R70S, D75N and I77Q (KNSCRS/KYSNQ; second monomer),
- Y33C, Q38H, Q44R, R68Y, R70S, D75N and I77Q (KNSCHS/RYSNQ) or S32T, Y33C, Q44D, R68A, R70S, D75K and I77R (KNTCQS/DASKR; first monomer), and a second monomer selected from the group consisting of: Y33G, Q38K, R68A, R70S, D75N and I77R (KNSGKS/QASNR); N30T, Y33H, Q38R, Q44K, R68Y, R70S, D75N and I77Y (KTSHRS/KYSNY); Y33T, Q38R, Q44K, R68Y, R70S, D75N and I77Y (KNSTRS/KYSNY); Y33G, Q38R, Q44K, R68Y, R70S, D75N and I77Y (KNSGRS/KYSNY); Y33G, Q38K, Q44K, R68Y, R70S, D75N and I77Y (KNSGKS/KYSNY),
- Y33T, Q38R, S40Q, Q44N, R68E, R70S, D75N and I77R (KNSTRQ/NESNR) or Y33S, Q38S, Q44K, R68A, R70S, and I77R (KNSSSS/KASDR; first monomer), and N30T, Y33H, Q38R, Q44K, R68Y, R70S, D75N and I77Y (KTSHRS/KYSNY; second monomer),
- S32T, Y33C, Q44R, R68Y, R70S, D75N and I77Q (KNTCQS/RYSNQ; first monomer), and Y33G, Q38K, R68A, R70S, D75N and I77R (KNSGKS/QASNR; second monomer);

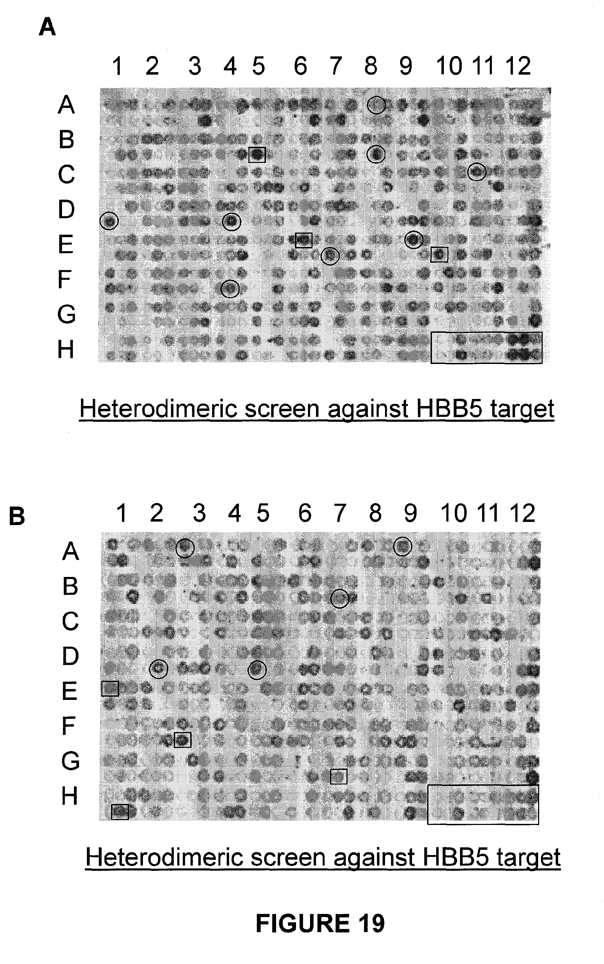

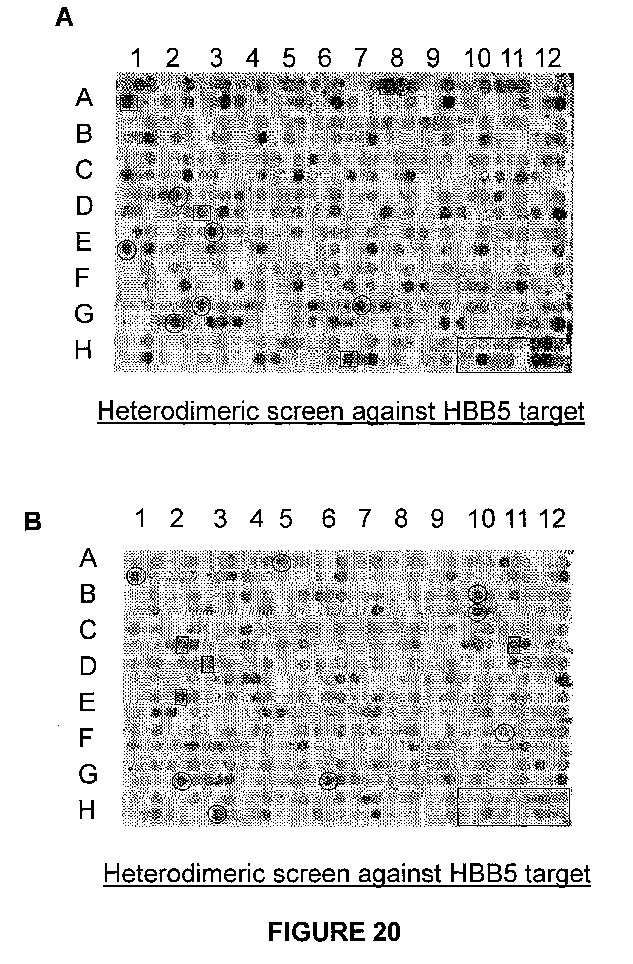

Preferred variants are those presented in Table XXI:
- KNSVHQ/DASKR+87L+131R (first monomer) and KNSGKS/KYSNY+19S+117G, KQTYRS/KYSNY+19S+64I, KNSGKS/KYSNY+19S+132V, KQTYRS/KYSNY+54L or KQTYRS/KYSNQ+19S (second monomer),
- KNSTRQ/DASKR+19S (first monomer) and KQTYRS/KYSNY+105A+152Q, KQTYRS/KYSNY+54L, KQTYRS/QASNR+87L or KQTYRS/KYSNI+19S (second monomer),
- KNSVRQ/DASKR+19S (first monomer) and KQTYRS/QASNR+87L+58Q, KQTYRS/KYSNY+105A+152Q, KNSGKS/KYSNY+132V, KQTYRS/KYSNY+54L or KQTYRS/QASNR+87L (second monomer),
- KNSVHQ/DASKR+87L (first monomer) and KNSGKS/KYSNY+132V, KNSGKS/KYSNY+19S+117G, KNSGKS/KYSNY+19S+132V, KQTYRS/KYSNY+54L, or KQTYRS/KYSNI+19S (second monomer).
   * The following variants (figure 16 and Tables V, VII, IX, XI and XII) cleave the HBB5 target (SEQ ID NO: 11) which is located in Intron 1, in the vicinity of the mutated codons responsible for three major sickle cell diseases: sickle cell anemia (E6V), HbSC (E6V and E6K) and HbSE (E6V and E26K; figure 16 and Table I):
- a first monomer having K at position 28, N at position 30, T, S, P or D at position 32, Y or P at position 33, Q at position 38, S at position 40, A at position 44, R at position 68, S at position 70, E at position 75 and R at position 77. These variants correspond to KNTYQS/ARSER, KNDYQS/ARSER, KNPYQS/ARSER, KNSPQS/ARSER and KNSYQS/ARSER. The first monomer comprises advantageously at least one first additional mutation selected from the group consisting of G19S, E80K, F87L, V105A and I132V. More preferably, the first monomer comprises at least the G19S and E80K substitutions or the F87L and E80K substitutions, and eventually additional mutations selected from the group consisting of: I24V, F43L, K82R, H85R, K96R, K100R, V105I, I109V, K121E, V129A, K139R, L155P, K159R, K160E and S161P. Examples of such variants are presented in Table V (SEQ ID NO: 79), Table IX (SEQ ID NO: 179 to 186), Table XI (SEQ ID NO: 209 to 220), Table XII (SEQID NO: 281), figure 16 (SEQ ID NO: 127), figures 19B and 20B (SEQ ID NO: 292 to 297).
- a second monomer having K at position 28, T, N, K or S at position 30, S or P at position 32, H, S or R at position 33, H, Q, K or R at position 38, S at position 40, K or R at position 44, Y , S or E at position 68, S at position 70, S, D or N at position 75 and T, R I or Q at position 77. Preferably, the residues at positions 28, 30, 32, 33, 38 and 40 are selected from the group consisting of: KTSRHS, KTSHRS, KNSRRS, KNSSKS, KNSRRS, KSSHRS, KKSSQS and KSPHRS, and the residues at positions 44, 68, 70, 75 and 77 are selected from the group consisting of: KSSNI, KYSDT, KYSDR, KYSNQ, RYSNQ and KESNR. More preferably, the second monomer is selected from the group consisting of: KTSRHS/KSSNI, KTSHRS/KYSDT, KNSRRS/KYSDT; KNSRRS/KYSDR, KNSRRS/KYSNQ, KNSRRS/RYSNQ, KSSHRS/KYSDT, KNSSKS/KYSNQ, KNSSKS/KYSDR, KNSRRS/KYSNQ, KSSHRS/KYSDQ, KSPHRS/KYSDT, KSSHRS/KYSNQ and KKSSQS/KESNR. The second monomer comprises advantageously at least one first additional mutation selected from the group consisting of G19S, E80K, F87L, V105A and I132V, and eventually a second additional mutation selected from the group consisting of: K4E, K7R, F16L, K34R, T49A, D60G, Y66H, E80G, I81T, K82R, K82E, N86S, Q92R, F94L, Q99R, K100R, N103S, Q111H, D120G, V125A, K139R, T140A, T147A, V151M, K159E, K159R, K160E, S162P and P163S. Examples of such variants are presented in Table V (SEQ ID NO: 45 and 56), Table VII (SEQ ID NO: 164 to 177), Table XI (SEQ ID NO: 197 to 208), Table XII (SEQ ID NO: 277 to 280) and figures 16 (SEQ ID NO: 140), 17A (SEQ ID NO: 298 to 301), 19A (SEQ ID NO: 302 to 304) and 20A (SEQ ID NO: 305, 306).

Examples of such variants which cleave the HBB5 target are presented in figures 16, 17, 19, 20 and Tables V, VII, IX, XI and XII and illustrated by the following sequences: Y33P, Q44A, R70S, D75E and I77R (KNSPQS/ARSER; first monomer) and N30K, Y33S, Q44K, R68E, R70S and I77R (KKSSQS/KESNR; second monomer); S32T, Q44A, R70S, D75E, I77R, E80K and K96R (KNTYQS/ARSER+80K+96R; first monomer), and N30T, Y33H, Q338R, Q44K, R68Y, R70S, and I77T (KTSHRS/KYSDT) or Y33R, Q38R, Q44K, R68Y, R70S and I77T (KNSRRS/KYSDT; second monomer).

Preferred variants are those presented in Table XII: KNTYQS/ARSER+19S+80K+85R+87L+96R+139R (first monomer) and KSPHRS/KYSDT+81 T or KTSHRS/KYSDT+66H+82R+86S+99R+132V+139R+140A (second monomer).
* Y33T, Q38A, Q44A and R70H (first monomer), and Y33H, Q38S, Q44Y, R68D, R70S, D75R and I77T (second monomer); this variant cleaves the HBB target SEQ ID NO: 12 that is located in Intron 2 (figure 16 and Table I),
* S32G, Y33T, Q44K, R68E, R70S, D75Y and I77V (first monomer), and N30H, Y33H, Q44P, R70H and D75N (second monomer); this variant cleaves the HBB target SEQ ID NO: 13 that is located in Intron 2 (figure 16 and Table I),
* N30D, S32T, R68Y, R70S and D75Y (first monomer), and S32D, Q38C, R68Y, R70S, D75R and I77Q (second monomer); this variant cleaves the HBB target SEQ ID NO: 14 that is located in Intron 2 (figure 16 and Table I),
* N30T, Y33G, R68H, R70H and D75N (first monomer), and Y33R, Q38T, Q44A and R70H (second monomer); this variant cleaves the HBB target SEQ ID NO: 15 that is located in Intron 2 is located (figure 16 and Table I),
* N30H, Y33S, R68Y, R70S and D75Y (first monomer), and N30D, Y33R, R68H, R70H and D75N (second monomer); this variant cleaves the HBB target SEQ ID NO: 16 that is located in Intron 2 (figure 16 and Table I),
* S32W, Y33T, Q44A and R70H (first monomer), and S32T, Y33C, Q44A, R70G and D75N (second monomer); this variant cleaves the HBB target SEQ ID NO: 17 that is located in Intron 2 (figure 16 and Table I),
* S32T, Q38W, Q44N, R68Y, R70S, D75R and I77V (first monomer), and S32T, Y33T, Q44A, R70S, D75E and I77R (second monomer); this variant cleaves the HBB target SEQ ID NO: 18 that is located in Intron 2 (figure 16 and Table I), and
* N30Q, Y33H, Q44A, R70S, D75E and I77R (first monomer), and S32N, Y33G, Q38Y, R70S, D75R and I77Q (second monomer); this variant cleaves the HBB target SEQ ID NO: 19 that is located in Exon 3 (figure 16 and Table I).

The heterodimeric variant as defined above may have only the amino acid substitutions as indicated above. In this case, the positions which are not indicated are not mutated and thus correspond to the wild-type I-*Cre*I (SEQ ID NO: 1 or 178) or I-*Cre*I N75 scaffold (SEQ ID NO: 5) sequence, respectively. Examples of such heterodimeric I-*Cre*I variants cleaving the HBB DNA targets of Table I (nucleotide sequences SEQ ID NO: 6 to 19) include the variants consisting of a first and a second monomer corresponding to the following pairs of sequences: SEQ ID NO: 123 to 135 (first monomer) and SEQ ID NO: 136 to 148, respectively (second monomer; Figure 16). The variants consisting of a first and a second monomer corresponding to the following pairs of sequences (Tables V, VII, IX, XI and XII), cleave the HBB5 target: SEQ ID NO: 79 (first monomer) and any of SEQ ID NO: 45, 56, 164 to 177, 298 to 301 (second monomer); SEQ ID NO: 179 to 186 (first monomer) and SEQ ID NO: 57 (second monomer); SEQ ID NO: 179 (first monomer) and any of SEQ ID NO: 197 to 208, 302 to 306 (second monomer); SEQ ID NO: 209 to 220, 292 to 297 (first monomer) and SEQ ID NO: 164 (second monomer); SEQ ID NO: 213, 214, 281 (first monomer) and SEQ ID NO: 277, 278, 279 or 280 (second monomer). In addition the variants consisting of a first and a second monomer corresponding to the following pairs of sequences (Tables XVI, XVIII, XX and XXI), cleave the HBB8 target: SEQ ID NO: 104 or 114 (first monomer) and SEQ ID NO: 82 (second monomer); SEQ ID NO: 105 or 118 (first monomer) and any of SEQ ID NO: 84, 85, 88, 94 and 103 (second monomer); SEQ ID NO: 113 or 111 (first monomer) and SEQ ID NO: 103 (second monomer); SEQ ID NO: 121 (first monomer) and SEQ ID NO: 85 (second monomer); SEQ ID NO: 118 (first monomer) and SEQ ID NO: 231 to 255 (second monomer); SEQ ID NO: 257 to 274 (first monomer) and SEQ ID NO: 103 (second monomer); SEQ ID NO: 286 (first monomer) and any of SEQ ID NO: 250, 236, 287, 288 and 289 (second monomer); SEQ ID NO: 269 (first monomer) and any of SEQ ID NO: 290, 236, 237 and 248 (second monomer); SEQ ID NO: 273 (first monomer) and any of SEQ ID NO: 291, 290, 242, 236 and 237 (second monomer); SEQ ID NO: 261 (first monomer) and any of SEQ ID NO: 242, 289, 287, 236 and 248 (second monomer).

Alternatively, the heterodimeric variant may consist of an *I-CreI* sequence comprising the amino acid substitutions as defined above. In the latter case, the positions which are not indicated may comprise additional mutations, for example one or more additional mutations as defined above.

The invention encompasses I-*Cre*I variants having at least 85 % identity, preferably at least 90 % identity, more preferably at least 95 % (96 %, 97 %, 98 %, 99 %) identity with the sequences as defined above, said variant being able to cleave a DNA target from the human beta globin gene.

The heterodimeric variant is advantageously an obligate heterodimer variant having at least one pair of mutations interesting corresponding residues of the first and the second monomers which make an intermolecular interaction between the two I-*Cre*I monomers, wherein the first mutation of said pair(s) is in the first monomer and the second mutation of said pair(s) is in the second monomer and said pair(s) of mutations prevent the formation of functional homodimers from each monomer and allow the formation of a functional heterodimer, able to cleave the genomic DNA target from the human beta globin gene.

To form an obligate heterodimer, the monomers have advantageously at least one of the following pairs of mutations, respectively for the first and the second monomer:
a) the substitution of the glutamic acid at position 8 with a basic amino acid, preferably an arginine (first monomer) and the substitution of the lysine at position 7 with an acidic amino acid, preferably a glutamic acid (second monomer) ; the first monomer may further comprise the substitution of at least one of the lysine residues at positions 7 and 96, by an arginine,
b) the substitution of the glutamic acid at position 61 with a basic amino acid, preferably an arginine (first monomer) and the substitution of the lysine at position 96 with an acidic amino acid, preferably a glutamic acid (second monomer) ; the first monomer may further comprise the substitution of at least one of the lysine residues at positions 7 and 96, by an arginine,
c) the substitution of the leucine at position 97 with an aromatic amino acid, preferably a phenylalanine (first monomer) and the substitution of the phenylalanine at position 54 with a small amino acid, preferably a glycine (second monomer); the first monomer may further comprise the substitution of the phenylalanine at position 54 by a tryptophane and the second monomer may further comprise the substitution of the leucine at position 58 or lysine at position 57, by a methionine, and
d) the substitution of the aspartic acid at position 137 with a basic amino acid, preferably an arginine (first monomer) and the substitution of the arginine at position 51 with an acidic amino acid, preferably a glutamic acid (second monomer).

For example, the first monomer may have the mutation D137R and the second monomer, the mutation R51D. The obligate heterodimer meganuclease comprises advantageously, at least two pairs of mutations as defined in a), b) c) or d), above; one of the pairs of mutation is advantageously as defined in c) or d). Preferably, one monomer comprises the substitution of the lysine residues at positions 7 and 96 by an acidic amino acid (aspartic acid (D) or glutamic acid (E)), preferably an aspartic acid (K7E and K96E) and the other monomer comprises the substitution of the glutamic acid residues at positions 8 and 61 by a basic amino acid (arginine (R) or lysine (K); for example, E8K and E61R). More preferably, the obligate heterodimer meganuclease, comprises three pairs of mutations as defined in a), b) and c), above. The obligate heterodimer meganuclease consists advantageously of (i) E8R, E8K or E8H, E61R, E61K or E61H and L97F, L97W or L97Y; (ii) K7R, E8R, E61R, K96R and L97F, or (iii) K7R, E8R, F54W, E61R, K96R and L97F and a second monomer (B) having at least the mutations (iv) K7E or K7D, F54G or F54A and K96D or K96E; (v) K7E, F54G, L58M and K96E, or (vi) K7E, F54G, K57M and K96E. For example, the first monomer may have the mutations K7R, E8R or E8K, E61R, K96R and L97F or K7R, E8R or E8K, F54W, E61R, K96R and L97F and the second monomer, the mutations K7E, F54G, L58M and K96E or K7E, F54G, K57M and K96E. The obligate heterodimer may comprise at least one NLS and/or one tag as defined above; said NLS and/or tag may be in the first and/or the second monomer

The subject-matter of the present invention is also a single-chain chimeric meganuclease (fusion protein) derived from an I-*Cre*I variant as defined above. The single-chain meganuclease may comprise two *I-CreI* monomers, two I-*Cre*I core domains (positions 6 to 94 of I-*Cre*I) or a combination of both. Preferably, the two monomers /core domains or the combination of both, are connected by a peptidic linker.

The subject-matter of the present invention is also a polynucleotide fragment encoding a variant or a single-chain chimeric meganuclease as defined above; said polynucleotide may encode one monomer of an homodimeric or heterodimeric variant, or two domains/monomers of a single-chain chimeric meganuclease.

The subject-matter of the present invention is also a recombinant vector for the expression of a variant or a single-chain meganuclease according to the invention. The recombinant vector comprises at least one polynucleotide fragment encoding a variant or a single-chain meganuclease, as defined above. In a preferred embodiment, said vector comprises two different polynucleotide fragments, each encoding one of the monomers of an heterodimeric variant.

A vector which can be used in the present invention includes, but is not limited to, a viral vector, a plasmid, a RNA vector or a linear or circular DNA or RNA molecule which may consist of a chromosomal, non chromosomal, semisynthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).

Preferred vectors include lentiviral vectors, and particularly self inactivacting lentiviral vectors.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for *S*. *cerevisiae*; tetracycline, rifampicin or ampicillin resistance in *E*. *coli.*

Preferably said vectors are expression vectors, wherein the sequence(s) encoding the variant/single-chain meganuclease of the invention is placed under control of appropriate transcriptional and translational control elements to permit production or synthesis of said variant. Therefore, said polynucleotide is comprised in an expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is an heterodimer, the two polynucleotides encoding each of the monomers are included in one vector which is able to drive the expression of both polynucleotides, simultaneously. Suitable promoters include tissue specific and/or inducible promoters. Examples of inducible promoters are: eukaryotic metallothionine promoter which is induced by increased levels of heavy metals, prokaryotic lacZ promoter which is induced in response to isopropyl-β-D-thiogalacto-pyranoside (IPTG) and eukaryotic heat shock promoter which is induced by increased temperature. Examples of tissue specific promoters are skeletal muscle creatine kinase, prostate-specific antigen (PSA), α-antitrypsin protease, human surfactant (SP) A and B proteins, β-casein and acidic whey protein genes.

According to another advantageous embodiment of said vector, it includes a targeting construct comprising sequences sharing homologies with the region surrounding the genomic DNA cleavage site as defined above.

For instance, said sequence sharing homologies with the regions surrounding the genomic DNA cleavage site of the variant is a fragment of the human beta globin gene comprising positions: 508 to 707, 651 to 850, 701 to 900, 1048 to 1247, 1147 to 1346, 1524 to 1723, 1599 to 1798, 1808 to 2007, 2084 to 2283, 2094 to 2293, 2245 to 2444, 2323 to 2522 and 2523 to 2722 of SEQ ID NO: 4.

Alternatively, the vector coding for an *I-CreI* variant/single-chain meganuclease and the vector comprising the targeting construct are different vectors.

More preferably, the targeting DNA construct comprises:
a) sequences sharing homologies with the region surrounding the genomic DNA cleavage site as defined above, and
b) a sequence to be introduced, flanked by sequences as in a) or included in sequences as in a).

Preferably, homologous sequences of at least 50 bp, preferably more than 100 bp and more preferably more than 200 bp are used. Therefore, the targeting DNA construct is preferably from 200 pb to 6000 pb, more preferably from 1000 pb to 2000 pb. Indeed, shared DNA homologies are located in regions flanking upstream and downstream the site of the break and the DNA sequence to be introduced should be located between the two arms. The sequence to be introduced is preferably a sequence which repairs a mutation in the gene of interest (gene correction or recovery of a functional gene), for the purpose of genome therapy. Alternatively, it can be any other sequence used to alter the chromosomal DNA in some specific way including a sequence used to modify a specific sequence, to attenuate or activate the gene of interest, to inactivate or delete the gene of interest or part thereof, to introduce a mutation into a site of interest or to introduce an exogenous gene or part thereof. Such chromosomal DNA alterations are used for genome engineering (animal models/human recombinant cell lines). The targeting construct comprises advantageously a positive selection marker between the two homology arms and eventually a negative selection marker upstream of the first homology arm or downstream of the second homology arm. The marker(s) allow(s) the selection of cells having inserted the sequence of interest by homologous recombination at the target site.

For example, figure 16 indicates the targets from the HBB gene, variants which are able to cleave said targets and the minimal matrix for repairing the cleavage at each target site.

For correcting the HBB gene, cleavage of the gene occurs in the vicinity of the mutation, preferably, within 500 bp of the mutation (Figure 1A). The targeting construct comprises a HBB gene fragment which has at least 200 bp of homologous sequence flanking the target site (minimal repair matrix) for repairing the cleavage, and includes a sequence encoding a portion of wild-type beta-globin corresponding to the region of the mutation for repairing the mutation (Figure 1A). Consequently, the targeting construct for gene correction comprises or consists of the minimal repair matrix; it is preferably from 200 pb to 6000 pb, more preferably from 1000 pb to 2000 pb. Preferably, when the cleavage site of the variant overlaps with the mutation (HBB8 target for example) the repair matrix includes a modified cleavage site that is not cleaved by the variant which is used to induce said cleavage in the HBB gene and a sequence encoding wild-type human beta globin that does not change the open reading frame of the human beta globin protein.

For example, for correcting the E6V, E6K or E26K mutations in the HBB gene found in sickle cell diseases (HbSS, HbSC and HbSE, respectively), as indicated in figure 3, the heterodimeric variants cleaving the HBB5 DNA target as defined above, may be used with a targeting construct comprising at least positions 1147 to 1346 of SEQ ID NO: 4 for efficient repair of both the DNA double-strand break and the mutation. Preferred variants are those presented in Table XII: KNTYQS/ARSER+19S+80K+85R+87L+96R+139R (first monomer; SEQ ID NO: 214, for example)) and KSPHRS/KYSDT+81T (SEQ ID NO: 277, for example) or KTSHRS/KYSDT+66H+82R+86S+99R+132V+139R+140A (SEQ ID NO: 279, for example; second monomer).

In addition, for correcting the E6V mutation in the HBB gene found in sickle cell anemia (HbSS), as indicated in figure 3, the heterodimeric variants cleaving the HBB8 DNA target as defined above may be used with a targeting construct comprising at least positions 1048 to 1247 of SEQ ID NO: 4 for efficient repair of both the DNA double-strand break and the mutation. Preferred variants are those presented in Table XXI:
- KNSVHQ/DASKR+87L+131R (SEQ ID NO: 286, for example; first monomer) and KNSGKS/KYSNY+19S+117G (SEQ ID NO: 286, for example), KQTYRS/KYSNY+19S+64I (SEQ ID NO: 288, for example), KNSGKS/KYSNY+19S+132V (SEQ ID NO: 287, for example), KQTYRS/KYSNY+54L (SEQ ID NO: 226, for example) or KQTYRS/KYSNQ+19S (SEQ ID NO: 250, for example, second monomer),
- KNSTRQ/DASKR+19S (SEQ ID NO: 269, for example) first monomer) and KQTYRS/KYSNY+105A+152Q (SEQ ID NO: 290, for example), KQTYRS/KYSNY+54L (SEQ ID NO: 236, for example), KQTYRS/QASNR+87L (SEQ ID NO: 237, for example) or KQTYRS/KYSNI+19S (SEQ ID NO: 248, for example; second monomer),
- KNSVRQ/DASKR+19S (SEQ ID NO: 273, for example) first monomer) and KQTYRS/QASNR+87L+58Q (SEQ ID NO: 291, for example), KQTYRS/KYSNY+105A+152Q (SEQ ID NO: 290, for example), KNSGKS/KYSNY+132V (SEQ ID NO: 242, for example), KQTYRS/KYSNY+54L (SEQ ID NO: 236, for example) or KQTYRS/QASNR+87L (SEQ ID NO: 248, for example; second monomer),
- KNSVHQ/DASKR+87L (SEQ ID NO: 261, for example) first monomer) and KNSGKS/KYSNY+132V (SEQ ID NO: 242, for example), KNSGKS/KYSNY+19S+117G (SEQ ID NO: 289, for example), KNSGKS/KYSNY+19S+132V (SEQ ID NO: 287, for example), KQTYRS/KYSNY+54L (SEQ ID NO: 236, for example) or KQTYRS/KYSNI+19S ((SEQ ID NO: 248, for example; second monomer).

Furthermore, for correcting the E26K mutation in the HBB gene found in HbSE, the following variant cleaving the HBB6 DNA target may be used with a targeting construct comprising at least positions 1048 to 1247 of SEQ ID NO: 4 for efficient repair of both the DNA double-strand break and the mutation:
- first monomer: Y33T, Q44D, R68A, R70S, D75K and I77R (SEQ ID NO: 126, for example), and second monomer: Y33R, Q38T, R68H, R70H and D75N (SEQ ID NO: 139, for example).

Alternatively, for restoring a functional gene (Figure 1B), cleavage of the gene occurs upstream of a mutation. Preferably said mutation is the first known mutation in the sequence of the gene, so that all the downstream mutations of the gene can be corrected simultaneously. Therefore, cleavage occurs preferably upstream of the HBB gene, for example at position 597 (HBB target SEQ ID NO: 6), 740 (HBB target SEQ ID NO: 7) or 790 (HBB target SEQ ID NO: 8) of SEQ ID NO: 4. The heterodimeric variant as indicated in figure 16 may be used:
* position 597: first monomer: N30S, Y33G, Q44D, R68N, R70S and D75N (SEQ ID NO: 123, for example), and second monomer: Y33T, Q38A, R70S, D75Y and I77R (SEQ ID NO: 136, for example),
* position 597: first monomer: N30D, Y33R, R70S and D75N (SEQ ID NO: 124, for example), and second monomer: S32G, Y33T, Q44A, R70G and D75N (SEQ ID NO: 137, for example),
* position 790: first monomer: S32D, Q38C, R70S and I77K (SEQ ID NO: 125, for example, and second monomer: S32D, Q38C, R70S and D75N (SEQ ID NO: 138, for example).

The targeting construct comprises the exons downstream of the cleavage site fused in frame (as in the cDNA) and with a polyadenylation site to stop transcription in 3'. The sequence to be introduced (exon knock-in construct) is flanked by introns or exons sequences surrounding the cleavage site, so as to allow the transcription of the engineered gene (exon knock-in gene) into a mRNA able to code for a functional protein (Figure 1B). For example, the exon knock-in construct is flanked by sequences upstream and downstream of the cleavage site, from a minimal repair matrix as defined above.

For making knock-in animals/cells, the targeting DNA construct comprises: a HBB gene fragment which has at least 200 bp of homologous sequence flanking the target site for repairing the cleavage, the sequence of an exogeneous gene of interest, and eventually a selection marker, such as the HPRT gene.

For the insertion of a sequence, DNA homologies are generally located in regions directly upstream and downstream to the site of the break (sequences immediately adjacent to the break; minimal repair matrix). However, when the insertion is associated with a deletion of ORF sequences flanking the cleavage site, shared DNA homologies are located in regions upstream and downstream the region of the deletion.

The modification(s) in the human HBB gene are introduced in human cells, for the purpose of human genome therapy or the making of human recombinant cell lines. However they may also be introduced in humanized cells wherein the endogenous HBB gene has been deleted (knock-out) and a normal or mutated human HBB gene has been introduced anywhere in the genome (transgenic) or specifically at the endogenous HBB locus (knock-in), for the purpose of making animal models of inherited hemoglobin disorders or studying the correction of the mutation by meganuclease-induced homologous recombination. Mice models of inherited hemoglobin disorders are well-known in the art and include the SCA mice, for example (Paszty et al., Science 1997, 278, 876-878; Ryan et al., Science, 1997, 278, 873-876; Chang et al., Proc. Nat. Acad. Sci. USA, 1998, 95, 14886-14890).

The subject-matter of the present invention is also a targeting DNA construct as defined above.

The subject-matter of the present invention is also a composition characterized in that it comprises at least one meganuclease as defined above (variant or single-chain derived chimeric meganuclease) and/or at least one expression vector encoding said meganuclease, as defined above.

In a preferred embodiment of said composition, it comprises a targeting DNA construct, as defined above.

Preferably, said targeting DNA construct is either included in a recombinant vector or it is included in an expression vector comprising the polynucleotide(s) encoding the meganuclease according to the invention.

The subject-matter of the present invention is also the use of at least one meganuclease and/or one expression vector, as defined above, for the preparation of a medicament for preventing, improving or curing a pathological condition caused by a mutation in the beta globin gene as defined above, in an individual in need thereof.

Preferably said pathological condition is selected from the group consisting of: sickle cell diseases (sickle cell anemia, haemoglobin SC, haemoglobin SE) and beta-thalassemias.

The use of the meganuclease may comprise at least the step of (a) inducing in somatic tissue(s) of the donor/ individual a double stranded cleavage at a site of interest of the beta globin gene comprising at least one recognition and cleavage site of said meganuclease by contacting said cleavage site with said meganuclease, and (b) introducing into said somatic tissue(s) a targeting DNA, wherein said targeting DNA comprises (1) DNA sharing homologies to the region surrounding the cleavage site and (2) DNA which repairs the beta globin gene upon recombination between the targeting DNA and the chromosomal DNA, as defined above. The targeting DNA is introduced into the somatic tissues(s) under conditions appropriate for introduction of the targeting DNA into the site of interest. The targeting construct may comprise sequences for deleting the human beta globin gene and eventually the sequence of an exogenous gene of interest (gene replacement).

According to the present invention, said double-stranded cleavage may be induced, *ex vivo* by introduction of said meganuclease into somatic cells (hematopietic stem cells) from the diseased individual and then transplantation of the modified cells back into the diseased individual.

The subject-matter of the present invention is also a method for preventing, improving or curing a pathological condition caused by a mutation in the beta-globin gene, in an individual in need thereof, said method comprising at least the step of administering to said individual a composition as defined above, by any means.

The subject-matter of the present invention is further the use of a meganuclease as defined above, one or two polynucleotide(s), preferably included in expression vector(s), for genome engineering of the human beta globin gene for non-therapeutic purposes. The human beta globin gene may be the human endogenous HBB gene (human HBB gene locus; human recombinant cells generation) or a transgene that has been inserted in an animal, for example a mouse (animal models of inherited hemoglobin disorders).

According to an advantageous embodiment of said use, it is for inducing a double-strand break in a site of interest of the beta globin gene comprising a genomic DNA target sequence, thereby inducing a DNA recombination event, a DNA loss or cell death.

According to the invention, said double-strand break is for: repairing a specific sequence in the human beta globin gene, modifying a specific sequence in the human beta globin gene, restoring a functional human beta globin gene in place of a mutated one, attenuating or activating the human beta globin gene, introducing a mutation into a site of interest of the human beta globin gene, introducing an exogenous gene or a part thereof, inactivating or deleting the human beta globin gene or a part thereof, translocating a chromosomal arm, or leaving the DNA unrepaired and degraded.

According to another advantageous embodiment of said use, said variant, polynucleotide(s), vector, are associated with a targeting DNA construct as defined above.

In a first embodiment of the use of the meganuclease according to the present invention, it comprises at least the following steps: 1) introducing a double-strand break at a site of interest of the human beta globin gene comprising at least one recognition and cleavage site of said meganuclease, by contacting said cleavage site with said meganuclease; 2) providing a targeting DNA construct comprising the sequence to be introduced flanked by sequences sharing homologies to the targeted locus. Said meganuclease can be provided directly to the cell or through an expression vector comprising the polynucleotide sequence encoding said meganuclease and suitable for its expression in the used cell. This strategy is used to introduce a DNA sequence at the target site, for example to generate knock-in or transgenic animals, or recombinant human cell lines that can be used for protein production, gene function studies, drug development (drug screening) or as inherited hemoglobin disorders model (study of the disease and of the correction of the mutations by meganuclease-induced homologous recombination).

In a second embodiment of the use of the meganuclease according to the present invention, it comprises at least the following steps: 1) introducing a double-strand break at a site of interest of the human beta globin gene comprising at least one recognition and cleavage site of said meganuclease, by contacting said cleavage site with said meganuclease; 2) maintaining said broken genomic locus under conditions appropriate for homologous recombination with chromosomal DNA sharing homologies to regions surrounding the cleavage site.

In a third embodiment of the use of the meganuclease according to the present invention, it comprises at least the following steps: 1) introducing a double-strand break at a site of interest of the human beta globin gene comprising at least one recognition and cleavage site of said meganuclease, by contacting said cleavage site with said meganuclease; 2) maintaining said broken genomic locus under conditions appropriate for repair of the double-strand break by non-homologous end joining.

The subject-matter of the present invention is also a method for making a modified mouse (knock-in mouse) derived from a humanized mouse comprising a normal/mutated human HBB gene, comprising at least the steps of:
(a) introducing into a pluripotent precursor cell or an embryo of said humanized mouse, a meganuclease, as defined above, so as to induce a double strand cleavage at a site of interest of the human HBB gene comprising a DNA recognition and cleavage site of said meganuclease; and simultaneously or consecutively,
(b) introducing into the mouse precursor cell or embryo of step (a) a targeting DNA, wherein said targeting DNA comprises (1) DNA sharing homologies to the region surrounding the cleavage site and (2) DNA which repairs the site of interest upon recombination between the targeting DNA and the chromosomal DNA, so as to generate a genomically modified mouse precursor cell or embryo having repaired the site of interest by homologous recombination,
(c) developping the genomically modified mouse precursor cell or embryo of step (b) into a chimeric mouse, and
(d) deriving a transgenic mouse from the chimeric mouse of step (c).

Preferably, step (c) comprises the introduction of the genomically modified precursor cell generated in step (b) into blastocysts so as to generate chimeric mice.

The subject-matter of the present invention is also a method for making a recombinant human cell, comprising at least the steps of:
(a) introducing into a human cell, a meganuclease, as defined above, so as to into induce a double stranded cleavage at a site of interest of the human globin gene comprising a DNA recognition and cleavage site for said meganuclease; and simultaneously or consecutively,
(b) introducing into the cell of step (a), a targeting DNA, wherein said targeting DNA comprises (1) DNA sharing homologies to the region surrounding the cleavage site and (2) DNA which repairs the site of interest upon recombination between the targeting DNA and the chromosomal DNA, so as to generate a recombinant human cell having repaired the site of interest by homologous recombination,
(c) isolating the recombinant human cell of step (b), by any appropriate mean.

The targeting DNA is introduced into the cell under conditions appropriate for introduction of the targeting DNA into the site of interest.

In a preferred embodiment, said targeting DNA construct is inserted in a vector.

The cells which are modified may be any cells of interest. For making knock-in/transgenic mice, the cells are pluripotent precursor cells such as embryo-derived stem (ES) cells, which are well-kown in the art. For making recombinant human cell lines, the cells may advantageously be PerC6 (Fallaux et al., Hum. Gene Ther. 9, 1909-1917, 1998) or HEK293 (ATCC # CRL-1573) cells. Said meganuclease can be provided directly to the cell or through an expression vector comprising the polynucleotide sequence encoding said meganuclease and suitable for its expression in the used cell.

For making human recombinant cell lines/transgenic animals expressing an heterologous protein of interest, the targeting DNA comprises a sequence encoding the product of interest (protein or RNA), and eventually a marker gene, flanked by sequences upstream and downstream the cleavage site, as defined above, so as to generate genomically modified cells (human cell) having integrated the exogenous sequence of interest in the human beta globin gene, by homologous recombination.

The sequence of interest may be any gene coding for a certain protein/peptide of interest, included but not limited to: reporter genes, receptors, signaling molecules, transcription factors, pharmaceutically active proteins and peptides, disease causing gene products and toxins. The sequence may also encode an RNA molecule of interest including for example a siRNA.

The expression of the exogenous sequence may be driven, either by the endogenous human beta globin promoter or by an heterologous promoter, preferably a ubiquitous or tissue specific promoter, either constitutive or inducible, as defined above. In addition, the expression of the sequence of interest may be conditional; the expression may be induced by a site-specific recombinase (Cre, FLP...).

Thus, the sequence of interest is inserted in an appropriate cassette that may comprise an heterologous promoter operatively linked to said gene of interest and one or more functional sequences including but mot limited to (selectable) marker genes, recombinase recognition sites, polyadenylation signals, splice acceptor sequences, introns, tags for protein detection and enhancers.

For making animal models of inherited-hemoglobin disorders, the targeting DNA comprises the correct/mutated human HBB gene sequence, flanked by sequences upstream and downstream the cleavage site, so as to generate animals having corrected the mutation in the HBB gene or animals having inserted a mutated HBB gene (sickle globin gene, for example).

The meganuclease can be used either as a polypeptide or as a polynucleotide construct encoding said polypeptide. It is introduced into mouse cells, by any convenient means well-known to those in the art, which are appropriate for the particular cell type, alone or in association with either at least an appropriate vehicle or carrier and/or with the targeting DNA.

According to an advantageous embodiment of the uses according to the invention, the meganuclease (polypeptide) is associated with:
- liposomes, polyethyleneimine (PEI); in such a case said association is administered and therefore introduced into somatic target cells.
- membrane translocating peptides (Bonetta, The Scientist, 2002, 16, 38; Ford et al., Gene Ther., 2001, 8, 1-4; Wadia and Dowdy, Curr. Opin. Biotechnol., 2002, 13, 52-56); in such a case, the sequence of the variant/single-chain meganuclease is fused with the sequence of a membrane translocating peptide (fusion protein).

According to another advantageous embodiment of the uses according to the invention, the meganuclease (polynucleotide encoding said meganuclease) and/or the targeting DNA is inserted in a vector. Vectors comprising targeting DNA and/or nucleic acid encoding a meganuclease can be introduced into a cell by a variety of methods (e.g., injection, direct uptake, projectile bombardment, liposomes, electroporation). Meganucleases can be stably or transiently expressed into cells using expression vectors. Techniques of expression in eukaryotic cells are well known to those in the art. (See Current Protocols in Human Genetics: Chapter 12 "Vectors For Gene Therapy" & Chapter 13 "Delivery Systems for Gene Therapy"). Optionally, it may be preferable to incorporate a nuclear localization signal into the recombinant protein to be sure that it is expressed within the nucleus.

Once in a cell, the meganuclease and if present, the vector comprising targeting DNA and/or nucleic acid encoding a meganuclease are imported or translocated by the cell from the cytoplasm to the site of action in the nucleus.

For purposes of therapy, the meganucleases and a pharmaceutically acceptable excipient are administered in a therapeutically effective amount. Such a combination is said to be administered in a "therapeutically effective amount" if the amount administered is physiologically significant. An agent is physiologically significant if its presence results in a detectable change in the physiology of the recipient. In the present context, an agent is physiologically significant if its presence results in a decrease in the severity of one or more symptoms of the targeted disease and in a genome correction of the lesion or abnormality.

In one embodiment of the uses according to the present invention, the meganuclease is substantially non-immunogenic, i.e., engender little or no adverse immunological response. A variety of methods for ameliorating or eliminating deleterious immunological reactions of this sort can be used in accordance with the invention. In a preferred embodiment, the meganuclease is substantially free of N-formyl methionine. Another way to avoid unwanted immunological reactions is to conjugate meganucleases to polyethylene glycol ("PEG") or polypropylene glycol ("PPG") (preferably of 500 to 20,000 daltons average molecular weight (MW)). Conjugation with PEG or PPG, as described by Davis et al. (US 4,179,337) for example, can provide non-immunogenic, physiologically active, water soluble endonuclease conjugates with anti-viral activity. Similar methods also using a polyethylene--polypropylene glycol copolymer are described in Saifer et al. (US 5,006,333).

The invention also concerns a prokaryotic or eukaryotic host cell which is modified by a polynucleotide or a vector as defined above, preferably an expression vector.

The invention also concerns a non-human transgenic animal or a transgenic plant, characterized in that all or part of their cells are modified by a polynucleotide or a vector as defined above.

As used herein, a cell refers to a prokaryotic cell, such as a bacterial cell, or an eukaryotic cell, such as an animal, plant or yeast cell.

The subject-matter of the present invention is also the use of at least one meganuclease variant, as defined above, as a scaffold for making other meganucleases. For example a third round of mutagenesis and selection/screening can be performed on said variants, for the purpose of making novel, third generation meganucleases.

The different uses of the meganuclease and the methods of using said meganuclease according to the present invention include the use of the *I-CreI* variant, the single-chain chimeric meganuclease derived from said variant, the polynucleotide(s), vector, cell, transgenic plant or non-human transgenic mammal encoding said variant or single-chain chimeric meganuclease, as defined above.

The I-*Cre*I variant according to the invention may be obtained by a method for engineering I-*Cre*I variants able to cleave a genomic DNA target sequence from the human beta globin gene, comprising at least the steps of:
(a) constructing a first series of *I-CreI* variants having at least one substitution in a first functional subdomain of the LAGLIDADG core domain situated from positions 26 to 40 *of I-CreI,*
(b) constructing a second series of I-*Cre*I variants having at least one substitution in a second functional subdomain of the LAGLIDADG core domain situated from positions 44 to 77 of I-*Cre*I,
(c) selecting and/or screening the variants from the first series of step (a) which are able to cleave a mutant I-*Cre*I site wherein (i) the nucleotide triplet at positions -10 to -8 of the I-*Cre*I site has been replaced with the nucleotide triplet which is present at positions -10 to -8 of said genomic target and (ii) the nucleotide triplet at positions +8 to +10 has been replaced with the reverse complementary sequence of the nucleotide triplet which is present at positions -10 to -8 of said genomic target,
(d) selecting and/or screening the variants from the second series of step (b) which are able to cleave a mutant I-*Cre*I site wherein (i) the nucleotide triplet at positions -5 to -3 of the I-*Cre*I site has been replaced with the nucleotide triplet which is present at positions -5 to -3 of said genomic target and (ii) the nucleotide triplet at positions +3 to +5 has been replaced with the reverse complementary sequence of the nucleotide triplet which is present at positions -5 to -3 of said genomic target,
(e) selecting and/or screening the variants from the first series of step (a) which are able to cleave a mutant *I-CreI* site wherein (i) the nucleotide triplet at positions +8 to +10 of the I-*Cre*I site has been replaced with the nucleotide triplet which is present at positions +8 to +10 of said genomic target and (ii) the nucleotide triplet at positions -10 to -8 has been replaced with the reverse complementary sequence of the nucleotide triplet which is present at positions +8 to +10 of said genomic target,
(f) selecting and/or screening the variants from the second series of step (b) which are able to cleave a mutant *I-CreI* site wherein (i) the nucleotide triplet at positions +3 to +5 of the *I-CreI* site has been replaced with the nucleotide triplet which is present at positions +3 to +5 of said genomic target and (ii) the nucleotide triplet at positions -5 to -3 has been replaced with the reverse complementary sequence of the nucleotide triplet which is present at positions +3 to +5 of said genomic target,
(g) combining in a single variant, the mutation(s) at positions 26 to 40 and 44 to 77 of two variants from step (c) and step (d), to obtain a novel homodimeric I-*Cre*I variant which cleaves a sequence wherein (i) the nucleotide triplet at positions -10 to -8 is identical to the nucleotide triplet which is present at positions -10 to -8 of said genomic target, (ii) the nucleotide triplet at positions +8 to +10 is identical to the reverse complementary sequence of the nucleotide triplet which is present at positions -10 to -8 of said genomic target, (iii) the nucleotide triplet at positions -5 to -3 is identical to the nucleotide triplet which is present at positions -5 to -3 of said genomic target and (iv) the nucleotide triplet at positions +3 to +5 is identical to the reverse complementary sequence of the nucleotide triplet which is present at positions -5 to -3 of said genomic target, and/or
(h) combining in a single variant, the mutation(s) at positions 26 to 40 and 44 to 77 of two variants from step (e) and step (f), to obtain a novel homodimeric I-*Cre*I variant which cleaves a sequence wherein (i) the nucleotide triplet at positions +3 to +5 is identical to the nucleotide triplet which is present at positions +3 to +5 of said genomic target, (ii) the nucleotide triplet at positions -5 to -3 is identical to the reverse complementary sequence of the nucleotide triplet which is present at positions +3 to +5 of said genomic target, (iii) the nucleotide triplet at positions +8 to +10 of the I-*Cre*I site has been replaced with the nucleotide triplet which is present at positions +8 to +10 of said genomic target and (iv) the nucleotide triplet at positions -10 to -8 is identical to the reverse complementary sequence of the nucleotide triplet at positions +8 to +10 of said genomic target,
(i) combining the variants obtained in steps (g) and (h) to form heterodimers, and
(j) selecting and/or screening the heterodimers from step (i) which are able to cleave said genomic DNA target from the human beta globin gene.

One of the step(s) (c), (d), (e) or (f) may be omitted. For example, if step (c) is omitted, step (d) is performed with a mutant I-*Cre*I site wherein both nucleotide triplets at positions -10 to -8 and -5 to -3 have been replaced with the nucleotide triplets which are present at positions -10 to -8 and -5 to -3, respectively of said genomic target, and the nucleotide triplets at positions +3 to +5 and +8 to +10 have been replaced with the reverse complementary sequence of the nucleotide triplets which are present at positions -5 to -3 and -10 to -8, respectively of said genomic target.

Steps (a), (b), (g), (h) and (i) may further comprise the introduction of additional mutations at other positions contacting the DNA target sequence or interacting directly or indirectly with said DNA target, at positions which improve the binding and/or cleavage properties of the mutants, or at positions which either prevent or impair the formation of functional homodimers or favor the formation of the heterodimer, as defined above.

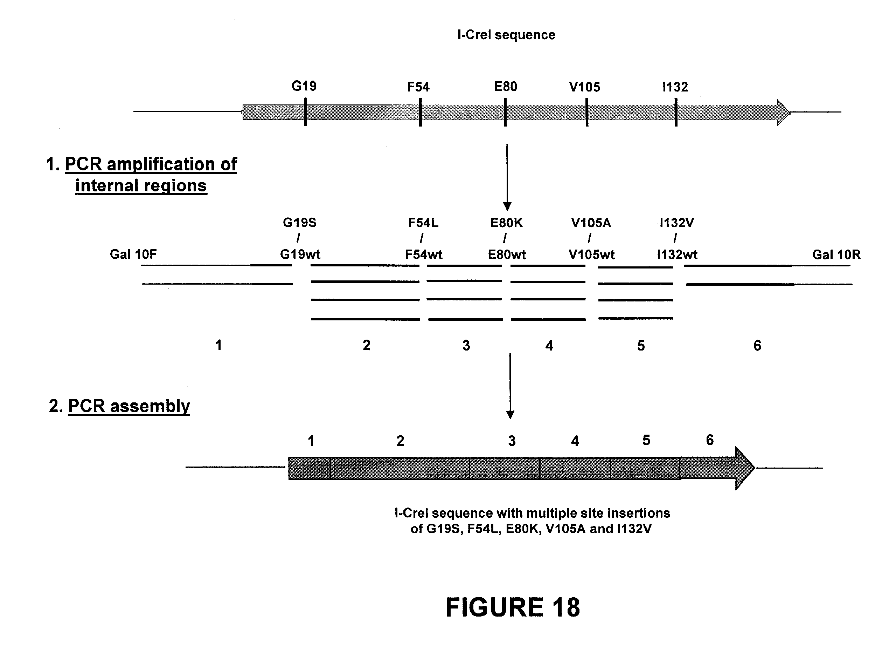

The additional mutations may be introduced by site-directed mutagenesis and/or random mutagenesis on a variant or on a pool of variants, according to standard mutagenesis methods which are well-known in the art, for example by using PCR. Site-directed mutagenesis may be advantageously performed by amplifying overlapping fragments comprising the mutated position(s), as defined above, according to well-known overlapping PCR techniques. In addition, multiple site-directed mutagenesis, as described in figure 18, may advantageously be performed on a variant or on a pool of variants.

In particular, random mutations may be introduced on the whole variant or in a part of the variant, in particular the C-terminal half of the variant (positions 80 to 163) to improve the binding and/or cleavage properties of the mutants towards the DNA target from the gene of interest. Site-directed mutagenesis at positions which improve the binding and/or cleavage properties of the mutants, for example at positions 19, 54, 80, 87, 105 and /or 132, may also be combined with random-mutagenesis. The mutagenesis may be performed by generating random/site-directedmutagenesis libraries on a pool of variants, according to standard mutagenesis methods which are well-known in the art and commercially available.

Preferably, the mutagenesis is performed on one monomer of the heterodimer formed in step (i) or obtained in step (j), advantageously on a pool of monomers, preferably on both monomers of the heterodimer of step (i) or (j).

Preferably, at least two rounds of selection/screening are performed according to the process illustrated by figure 4 of Arnould et al., J. Mol. Biol., Epub 10 May 2007. In the first round, one of the monomers of the heterodimer is mutagenised (monomer Y in figure 4), co-expressed with the other monomer (monomer X in figure 4) to form heterodimers, and the improved monomers Y⁺ are selected against the target from the gene of interest. In the second round, the other monomer (monomer X) is mutagenised, co-expressed with the improved monomers Y⁺ to form heterodimers, and selected against the the target from the gene of interest to obtain meganucleases (X⁺ Y⁺) with improved activity.The mutagenesis may be random-mutagenesis or site-directed mutagenesis on a monomer or on a pool of monomers, as indicated above. Both types of mutagenesis are advantageously combined. Additional rounds of selection/screening on one or both monomers may be performed to improve the cleavage activity of the variant.

The cleavage activity of the improved meganuclease obtainable by the method according to the present invention may be measured by a direct repeat recombination assay, in yeast or mammalian cells, using a reporter vector, by comparison with that of the initial meganuclease. The reporter vector comprises two truncated, non-functional copies of a reporter gene (direct repeats) and the genomic DNA target sequence which is cleaved by the initial meganuclease, within the intervening sequence, cloned in a yeast or in a mammalian expression vector. Expression of the meganuclease results in cleavage of the genomic DNA target sequence. This cleavage induces homologous recombination between the direct repeats, resulting in a functional reporter gene (LacZ, for example), whose expression can be monitored by appropriate assay. A stronger signal is observed with the improved meganuclease, as compared to the initial meganuclease. Alternatively, the activity of the improved meganuclease towards its genomic DNA target can be compared to that of I-*Cre*I towards the I*-Cre*I site, at the same genomic locus, using a chromosomal assay in mammalian cells (Arnould et al., J. Mol. Biol., 2007, 371, 49-65).

The (intramolecular) combination of mutations in steps (g) and (h) may be performed by amplifying overlapping fragments comprising each of the two subdomains, according to well-known overlapping PCR techniques.

The (intermolecular) combination of the variants in step (i) is performed by co-expressing one variant from step (g) with one variant from step (h), so as to allow the formation of heterodimers. For example, host cells may be modified by one or two recombinant expression vector(s) encoding said variant(s). The cells are then cultured under conditions allowing the expression of the variant(s), so that heterodimers are formed in the host cells, as described previously in the International PCT Application WO 2006/097854 and Arnould et al., J. Mol. Biol., 2006, 355, 443-458.

The selection and/or screening in steps (c), (d), (e), (f) and/or (j) may be performed by using a cleavage assay *in vitro* or *in vivo,* as described in the International PCT Application WO 2004/067736, Arnould et al., J. Mol. Biol., 2006, 355, 443-458, Epinat et al., Nucleic Acids Res., 2003, 31, 2952-2962 and Chames et al., Nucleic Acids Res., 2005, 33, e178.

According to another advantageous embodiment of said method, steps (c), (d), (e), (f) and/or (j) are performed *in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break.

The subject matter of the present invention is also an I-*Cre*I variant having mutations at positions 26 to 40 and/or 44 to 77 of I-*Cre*I that is useful for engineering the variants able to cleave a DNA target from the human beta globin gene, according to the present invention. In particular, the invention encompasses the I-*Cre*I variants as defined in step (c) to (f) of the method for engineering I-*Cre*I variants, as defined above, including the variants of Tables II, III, VI, VIII, X, XIII, XIV, XVII and XIX. The invention encompasses also the I-*Cre*I variants as defined in step (g) and (h) of the method for engineering I-*Cre*I variants, as defined above, including the variants of the sequence SEQ ID NO: 38 to 76, 80 to 122, 163, 223 to 230 (combined variants of Tables II, III, VI, XIII, XIV and XVII).

Single-chain chimeric meganucleases able to cleave a DNA target from the gene of interest are derived from the variants according to the invention by methods well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing single-chain chimeric meganucleases derived from the variants as defined in the present invention.

The polynucleotide sequence(s) encoding the variant as defined in the present invention may be prepared by any method known by the man skilled in the art. For example, they are amplified from a cDNA template, by polymerase chain reaction with specific primers. Preferably the codons of said cDNA are chosen to favour the expression of said protein in the desired expression system.

The recombinant vector comprising said polynucleotides may be obtained and introduced in a host cell by the well-known recombinant DNA and genetic engineering techniques.

The I-*Cre*I variant or single-chain derivative as defined in the present the invention are produced by expressing the polypeptide(s) as defined above; preferably said polypeptide(s) are expressed or co-expressed (in the case of the variant only) in a host cell or a transgenic animal/plant modified by one expression vector or two expression vectors (in the case of the variant only), under conditions suitable for the expression or co-expression of the polypeptide(s), and the variant or single-chain derivative is recovered from the host cell culture or from the transgenic animal/plant.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

In addition to the preceding features, the invention further comprises other features which will emerge from the description which follows, which refers to examples illustrating the I-*Cre*I meganuclease variants and their uses according to the invention to Table XXII which summarizes the different sequences, described in the present invention, as well as to the appended drawings in which:
- figure 1 illustrates two different strategies for restoring a functional gene by meganuclease-induced recombination. **A**. Gene correction. A mutation occurs within the HBB gene. Upon cleavage by a meganuclease and recombination with a repair matrix the deleterious mutation is corrected. **B**. Exonic sequences knock-in. A mutation occurs within the HBB gene. The mutated mRNA transcript is featured below the gene. In the repair matrix, exons located upstream of the cleavage site are fused in frame (as in a cDNA), with a polyadenylation site to stop transcription in 3'. Introns and exons sequences can be used as homologous regions. Exonic sequences knock-in results into an engineered gene, transcribed into a mRNA able to code for a functional HBB protein.
- figure 2 illustrates the modular structure of homing endonucleases and the combinatorial approach for making redesigned Homing endonucleases. **A**. Tridimensional structure of the *I-CreI* homing endonuclease bound to its DNA target. The catalytic core is surrounded by two αββαββα folds forming a saddle-shaped interaction interface above the DNA major groove. **B**. Different binding sequences derived from the *I-CreI* target sequence (top right and bottom left) to obtain heterodimers or single chain fusion molecules cleaving non palindromic chimeric targets (bottom right). **C**. The identification of smaller independent subunit, i.e., subunit within a single monomer or αββαββα fold (top right and bottom left) allows for the design of novel chimeric molecules (bottom right), by combination of mutations within a same monomer. Such molecules cleave palindromic chimeric targets (bottom right). **D**. The combination of the two former steps allows a larger combinatorial approach, involving four different subdomains. A large collection of I-*Cre*I derivatives with locally altered specificity is generated. In a first step, couples of novel meganucleases are combined in new homodimeric proteins (by combinations of mutations within a same monomer; "half-meganucleases") cleaving palindromic targets derived from the target one wants to cleave. Then, the combination of such "half-meganuclease" can result in an heterodimeric species cleaving the target of interest (custom meganuclease). Thus, the identification of a small number of new cleavers for each subdomain allows for the design of a very large number of novel endonucleases with fully redesigned specificity.
- figure 3 represents the genomic locus of the human HBB gene. The human HBB gene (Accession number NT_009237.17; 1606 bp; SEQ ID NO: 3) is included in a 4080 bp fragment (SEQ ID NO: 4). Exons sequences are indicated as grey boxes with their junctions. The HBB5, HBB6 and HBB8 targets are indicated with their sequences and positions. HBB8 target is the mutated version of the natural HBB6 sequence leading to the sickle cell anemia. The mutation (an A to T transversion) that changes the glutamic acid residue to a valine is indicated.
- figure 4 represents the HBB5 target sequence and its derivatives. All targets are aligned with the C1221 target, a palindromic sequence cleaved by I-*Cre*I. 10GGT_P, 10AAG_P, 5CCT_P and 5AGG_P are close derivatives found to be cleaved by I-*Cre*I mutants. They differ from C1221 by the boxed motives. C1221, 10GGT_P, 10AAG_P, 5CCT_P and 5AGG_P were first described as 24 bp sequences, but structural data suggest that only the 22 bp are relevant for protein/DNA interaction. However, positions ±12 are indicated in parenthesis. HBB5 is the DNA sequence located in the human HBB gene at position 1237-1258 (Figure 3). HBB 5.3 is the palindromic sequence derived from the right part of HBB5, and HBB5.4 is the palindromic sequence derived from the left part of HBB 5. As shown in the figure, the boxed motives from 10GGT_P, 10AAG_P, 5CCT_P and 5AGG_P are found in the HBB5 series of targets.
- figure 5 represents the HBB6/HBB8 target sequences and their derivatives. All targets are aligned with the C1221 target, a palindromic sequence cleaved by I-*Cre*I. 10AGA_P, 10TGA_P, 5TCT_P and 5CCT_P are close derivatives found to be cleaved by I-*Cre*I mutants. They differ from C1221 by the boxed motives. C1221, 10AGA_P, 10TGA_P, 5TCT_P and 5CCT_P were first described as 24 bp sequences, but structural data suggest that only the 22 bp are relevant for protein/DNA interaction. However, positions ±12 are indicated in parenthesis. HBB6 is the natural target located in the human HBB gene at position 1138-1159 (Figure 3). HBB8, which is also located in the human HBB gene at position 1138-1159, includes the A to T transversion at position 1148 that is responsible for Sickle cell anemia (Figure 3). In the HBB 8.2 target, the sequence in the middle of the target is replaced with the bases found in C1221. HBB8.3 is the palindromic sequence derived from the right part of HBB8, and HBB8.4 is the palindromic sequence derived from the left part of HBB8. HBB 8.5 and HBB8.6 are palindromic sequences derived respectively from the left part and the right part of HBB 8 with the CCAC sequence present in the middle. HBB 6.5 and HBB6.6 are palindromic sequences derived respectively from the left part and the right part of HBB 6 with the CCTC sequence present in the middle. As shown in the Figure, the boxed motives from 10AGA_P, 10TGA_P, 5TCT_P and 5CCT_P are found in the HBB6/HBB8 series of targets.

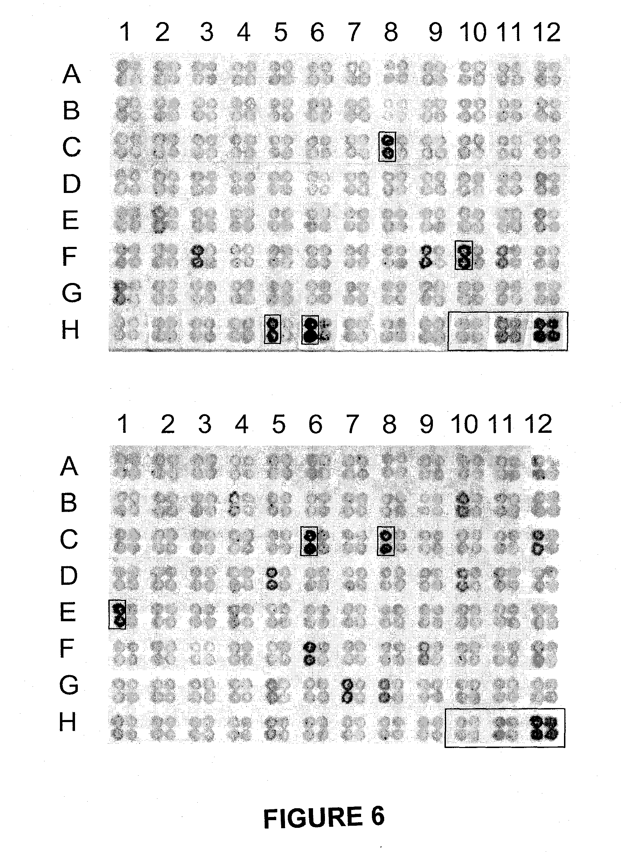

- figure 6 illustrates cleavage of HBB5.3 by combinatorial mutants. The figure displays an example of primary screening of I-*Cre*I combinatorial mutants with the HBB5.3 target. In the upper filter, the sequences of positive mutants at position C8, F10, H5 and H6 (boxed) are KDSRQS/DASKR, KNSHQS/KYSDT, KKSAQS/KYSDT and KKSAQS/RYSNQ, respectively (same nomenclature as for Table II). In the lower filter, the sequences of positive mutants at position C6, C8 and E1 (boxed) are respectively KNSSKS/KYSDT, KNSRRS/KSSNV and KKSTQS/RYSNQ. H10, H11, H12 are positive controls of different strength.

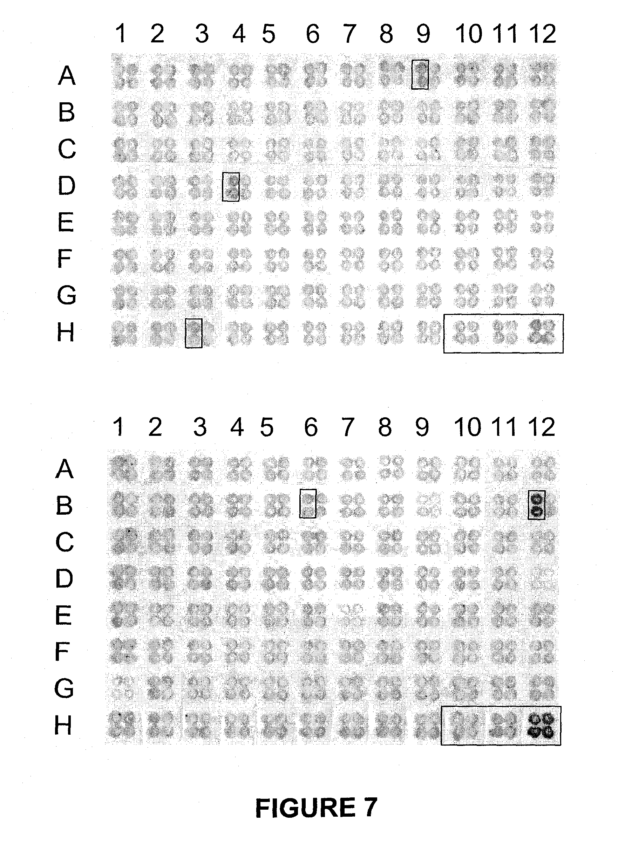

- figure 7 illustrates cleavage of HBB 5.4 by combinatorial mutants. The figure displays an example of primary screening of I-*Cre*I combinatorial mutants with the HBB5.4 target. In the upper filter, the sequences of positive mutants at positions A9, D4 and H3 (boxed) are KGSYQS/RYSET, KNSYQS/ARSER and KNQYQS/ARSER respectively (same nomenclature as for Table III). In the lower filter, the sequences of positive mutants at positions B6 and B12 (boxed) are KQSRQS/ARSER and KGSYQS/ARSER respectively. H10, H11, H12 are positive controls of different strength.

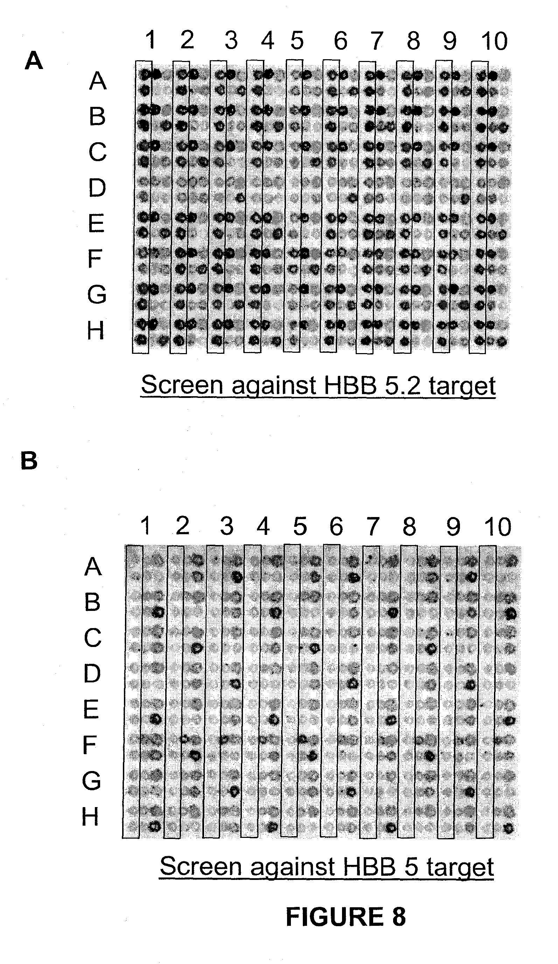

- figure 8 illustrates cleavage of HBB5.2 and HBB5 targets by heterodimeric combinatorial mutants. **A**. Secondary screening of 80 combinations of *I-CreI* mutants with the HBB5.2 target. **B**. Secondary screening of the same combinations of I-*Cre*I mutants with the HBB 5 target. Clones screened against the HBB 5.2 target (panel A) or the HBB 5 target (panel B) are indicated by vertical boxes.

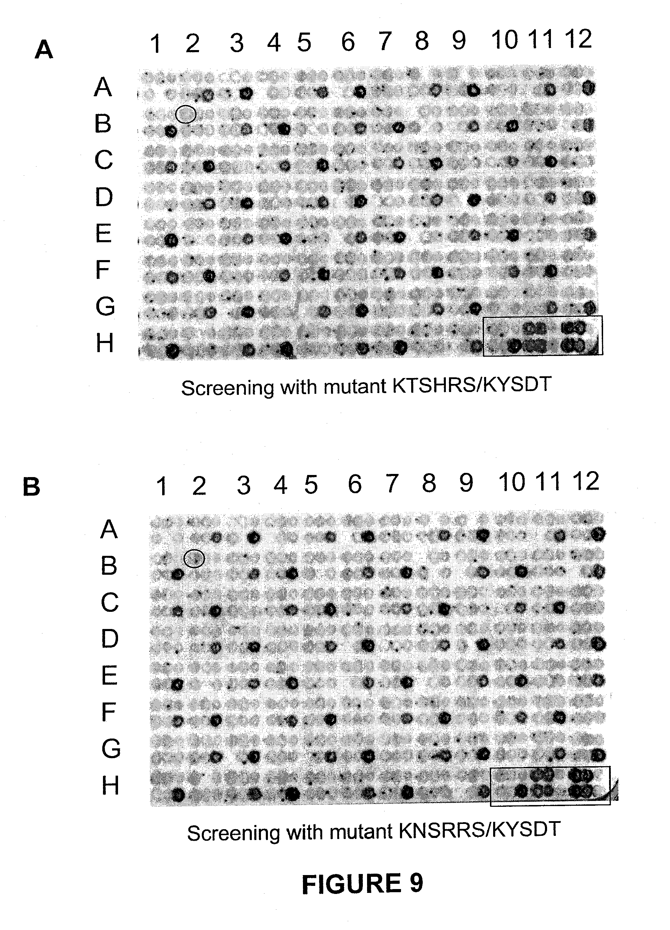

- figure 9 illustrates cleavage of the HBB 5 target. A series *of I-CreI* N75 mutants cutting HBB5.4 were optimized and co-expressed with two mutants cutting HBB5.3 Cleavage is tested with the HBB5 target. Mutants displaying improved cleavage of HBB5 are circled. In the two filters shown, B2 corresponds either to the heterodimer:
   - 28K30N32T33Y38Q40S44A68R70S75E77R80K96R (KNTYQS/ARSER/80K96R) + KTSHRS/KYSDT (panel A) or
   - 28K30N32T33Y38Q40S44A68R70S75E77R80K96R (KNTYQS/ARSER/80K96R) + KNSRRS/KYSDT (panel B). H10, H11, H12 are positive controls of different strength.

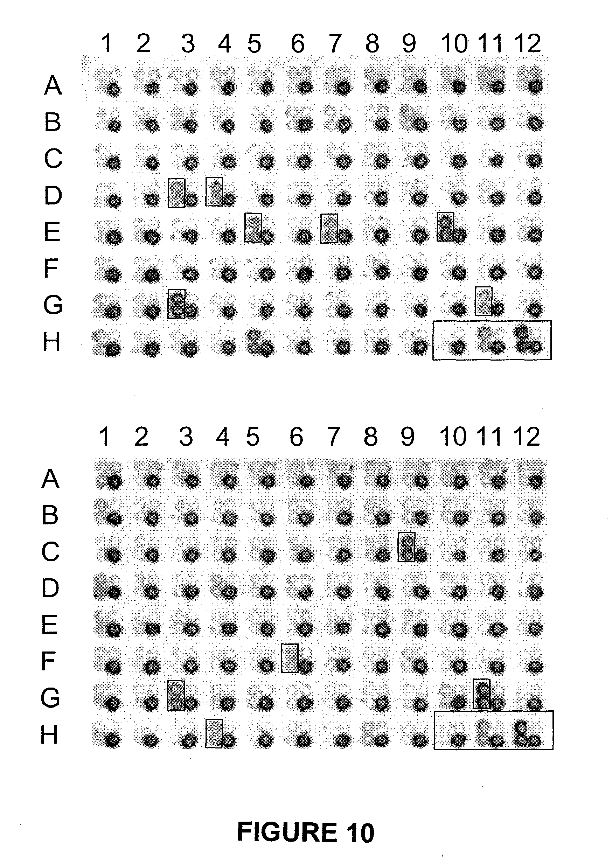

- figure 10 illustrates cleavage of HBB8.3 by combinatorial mutants. The figure displays an example of primary screening of *I-CreI* combinatorial mutants with the HBB 8.3 target. In the upper filter, the sequences of positive mutants at positions D3, D4, E5, E7, E10, G3 and G11 (boxed) are respectively KTSHRS/KYSNI, KNSSKS/KYSNY, KNSRRS/KYSNY, KNSTRS/KYSNV, KNSGKS/QASNR, KTSHRS/KYSNY, KDSRQS/KYSNY (same nomenclature as for Table VI). In the lower filter, the sequences of positive mutants at positions C9, F6, G3 and H4 (boxed) are respectively KNSGKS/KYSNI, KDSRQS/QSSNR, KSSGQS/KYSNY, KNSGRS/KYSNV (same nomenclature as for Table VI). H10, H11, H12 are positive controls of different strength.

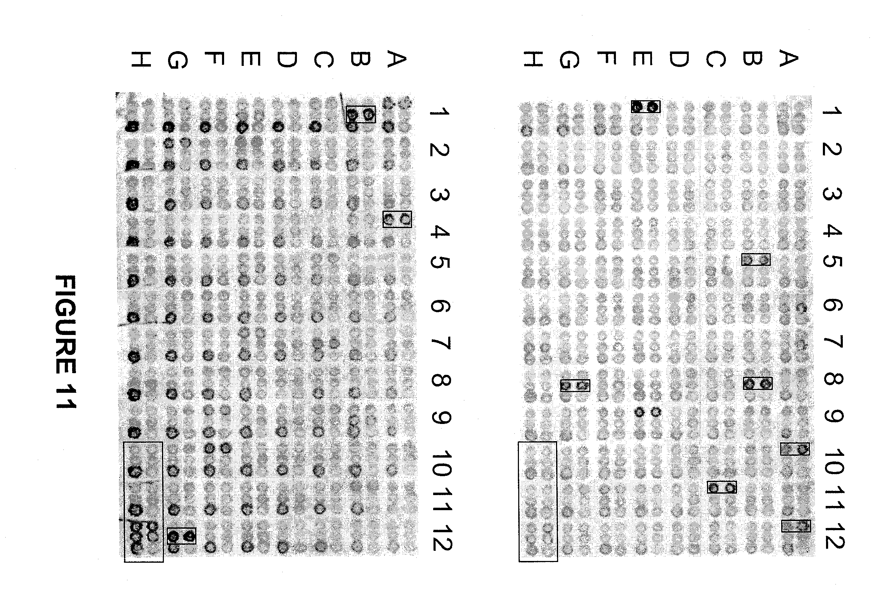

- figure 11 illustrates cleavage of HBB8.4 by combinatorial mutants. The figure displays an example of primary screening of I-*Cre*I combinatorial mutants with the HBB 8.4 target. In the upper filter, the sequences of positive mutants at positions A10, A12, B5, B8, C11, E1 and G8 (boxed) are respectively KNSSRH/RRSND, KTSGQS/DASKR, KNTCQS/KYSDT, KNETQS/RYSNQ, KNSCTS/KTSDR, KNETQS/DASKR, and KNETQS/DASKR (same nomenclature as for Table VII). In the second filter, the sequences of positive mutants at positions A4, B2 and G12 (boxed) are respectively KNTCQS/KTSDR, KNTTQS/KTSDR and KNSTQT/RYSNQ (same nomenclature as for Table VII). H10, H11, H12 are positive controls of different strength.

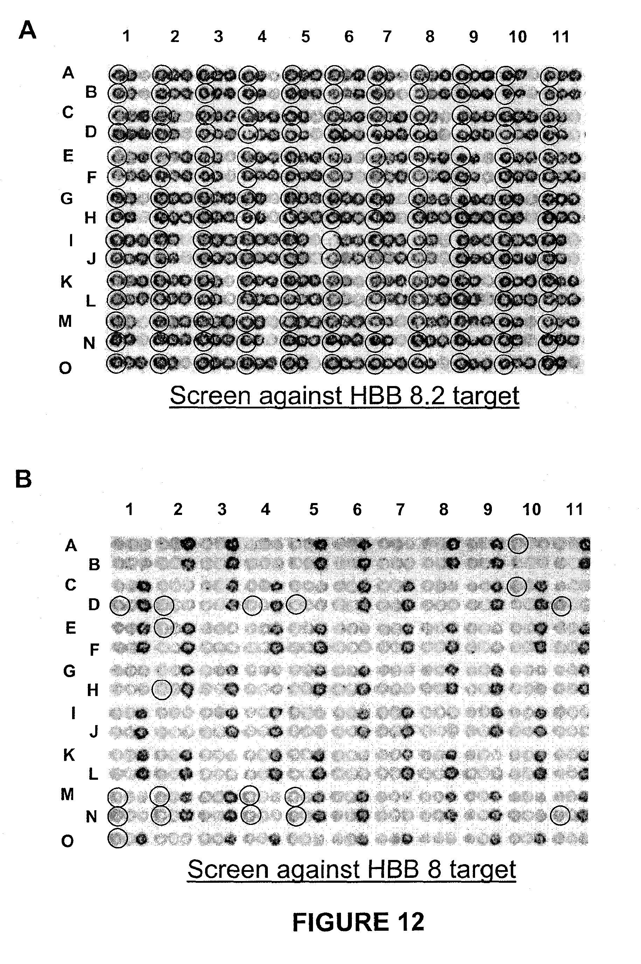

- figure 12 illustrates cleavage of HBB8 by heterodimeric combinatorial mutants. **A**. Secondary screening of combinations of I-*Cre*I mutants with the HBB8.2. target. **B**. Secondary screening of the same combinations of I-*Cre*I mutants with the HBB8 target. Positives clones obtained with the screening against the HBB8.2 target (panel A) or the HBB8 target (panel B) are indicated by circles.

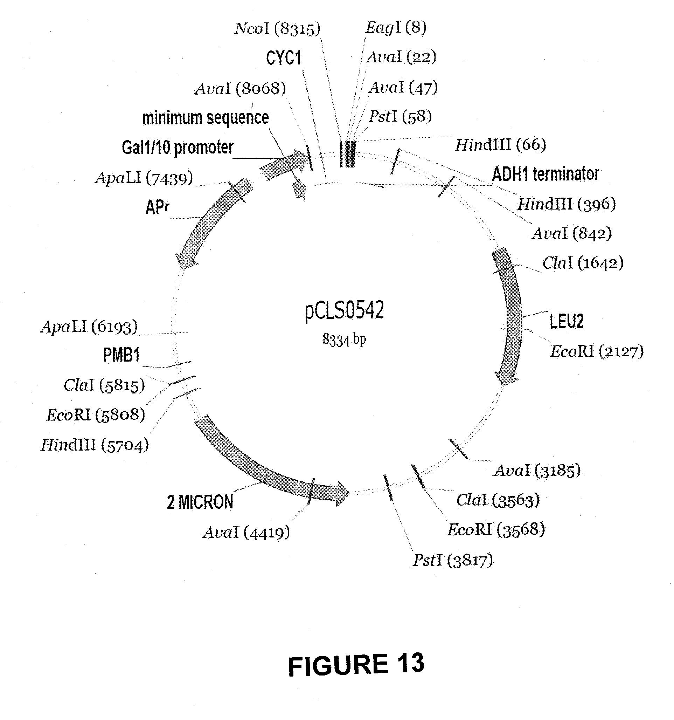

- figure 13 represents the pCLS0542 vector map

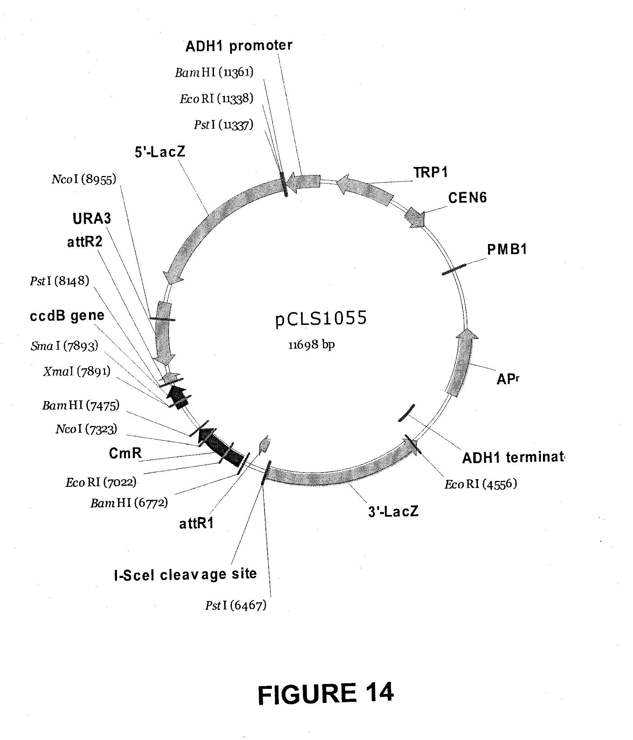

- figure 14 represents the pCLS1055 vector map

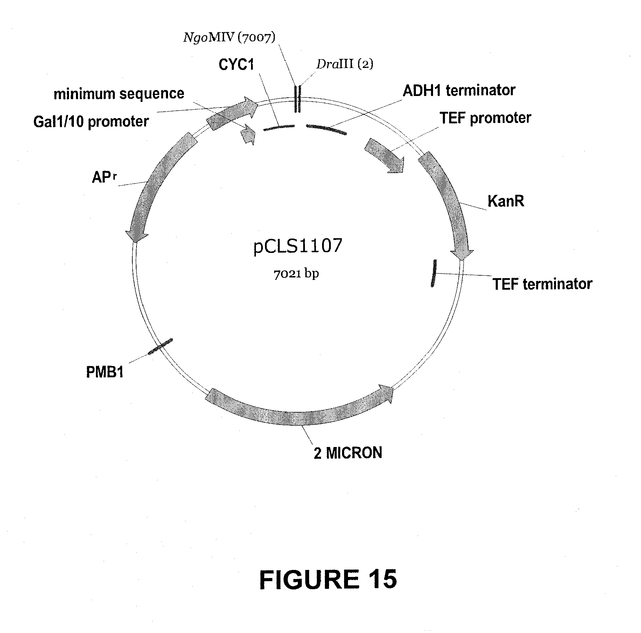

- figure 15 represents the pCLS1107 vector map.
- figure 16 represents meganuclease target sequences found in the human beta globin gene and the corresponding I-*Cre*I variant which is able to cleave said DNA target. The exons closest to the target sequences, and the exons junctions are indicated (columns 1 and 2), the sequence of the DNA target is presented (column 3), with the position of its first nucleotide by reference to SEQ ID NO: 4 (column 4). The minimum repair matrix for repairing the cleavage at the target site is indicated by its first nucleotide (start, column 7) and last nucleotide (end, column 8). The sequence of each I-*Cre*I variant is defined by the mutated residues at the indicated positions. For example, the first heterodimeric variant of figure 16 consists of a first monomer having S, G, D, N, S and N at positions 30, 33, 44, 68, 70 and 75, respectively and a second monomer having T, A, S, Y, R at positions 33, 38, 70, 75 and 77, respectively. The positions are indicated by reference to I-*Cre*I sequence SWISSPROT P05725 (SEQ ID NO: 1) ; I-*Cre*I has N, Y, Q, Q, R, R, D and I, at positions 30, 33, 38, 44, 68, 70, 75 and 77 respectively.

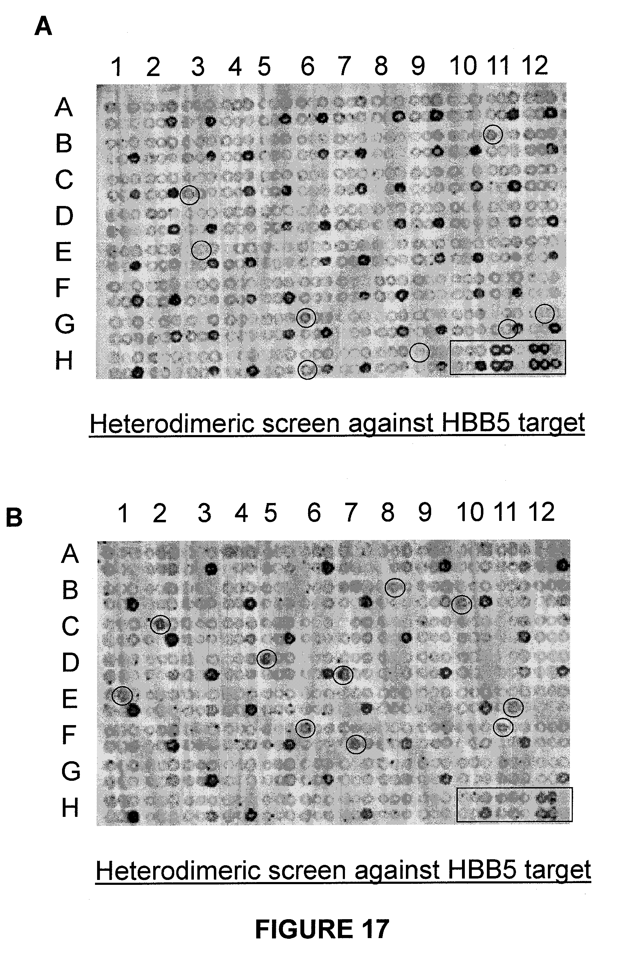

- figure 17 illustrates cleavage of HBB5 by optimized I-*Cre*I mutants cleaving HBB5.3 or HBB5.4 targets. **A**. Example of heterodimeric primary screening against the HBB5 target of I-*Cre*I refined mutants cleaving HBB5.3 sequence and having the substitution G19S obtained by site directed mutagenesis. In the filter, the sequences of positive mutants at position B11, C3, E3, G6, G11, G12, H6 and H9, are KTSHRS / KYSDT + 19S, KNSRRS / KYSDT + 19S, KNSRRS /KYSNQ + 19S + 82E, KNSRRS / RYSNQ + 19S, KNSSKS / KYSNQ + 19S, KTSHRS / KYSDT + 19S, KNSSKS / KYSNQ + 19S + 92R, KNSSKS / KYSDR + G19S, respectively (same nomenclature as for Table V). H10, H11, H12 are positive controls of different strength. **B**. Example of heterodimeric primary screening against the HBB 5 target of I*_Cre*I refined mutants cleaving HBB5.4 sequence and having the substitution G19S obtained by site directed mutagenesis. In the filter, the sequences of positive mutants are KNDYQS / ARSER +19S +80K +96R for the position B8, KNPYQS / ARSER + 19S for the position E1, KNTYQS / ARSER +19S + 80K for the positions E11, F6, F11 and KNTYQS / ARSER +19S + 80K + 96R for the positions B10, C2, D5, D7, F7 (same nomenclature as for Table V). H10, H11, H12 are positive controls of different strength.
- figure 18 illustrates the principle of multiple site directed mutagenesis of I-Crel mutants. Six groups of separate overlapping PCR reactions were first carried out that amplify internal fragments of the *I-CreI* N75 coding sequence containing the sequences between the positions concerned by the insertion of the mutations G19S, F54L, E80K, V105A, I132V. The six pools of fragments were then purified and assembled by PCR to obtain I-CreI coding sequences with the association of different mutations.
- figure 19 illustrates cleavage of HBB5 by optimized I-*Cre*I mutants cleaving HBB5.3 or HBB5.4 targets obtained by random mutagenesis. **A**. Example of heterodimeric screening against the HBB5 target of I_*Cre*I refined mutants cleaving HBB5.3 sequence. In the filter, positive mutants with an increased efficiency of cleavage of HBB5 target are indicated by circles and squares. The sequences of positive mutants at positions B5, E6 and E10 (squares) are KNSRRS /KYSDR+19S+87L+103S +147A, KNSRRS / KYSDR+16L+ 19S +87L +159R, KNSRRS / KYSNQ+ 19S+82E +105A + 162P (same nomenclature as for Table V) respectively. H10, H11, H12 are positive controls of different strength. **B**. Example of heterodimeric screening against the HBB 5 target of I_*Cre*I refined mutants cleaving HBB5.4 sequence. In the filter, positive mutants with an increased efficiency of cleavage of HBB 5 target are indicated by circles and squares. The sequences of positive mutants at positions E1, F3, G7 and H1 (squares) are KNTYQS / ARSER + 19S+ 80K, KNTYQS / ARSER+19S+34R+80K+87L+96R, KNTYQS /ARSER+24V+ 43L+80K+82R +87L+155P, KNTYQS /ARSER+19S+34R+80K+87L+96R (same nomenclature as for Table V) respectively. H10, H11, H12 are positive controls of different strength.
- figure 20 illustrates cleavage of HBB5 by optimized I-*Cre*I mutants cleaving HBB5.3 or HBB5.4 targets obtained by multiple site directed mutagenesis. A. Example of heterodimeric screening against the HBB 5 target of I_*Cre*I refined mutants cleaving HBB5.3 sequence. In the filter, positive mutants with an increased efficiency of cleavage of HBB 5 target are indicated by circles and squares. The sequences of positive mutants at positions A1, A8,D3 and H7 (squares) are KNSRRS / KYSNQ +19S+80K +132V, KNSRRS / KYSDR+ 19S +105A+132V+159E+160E, KSSHRS / KYSDQ +105A+132V, KSSHRS / KYSNQ +19S+80K (same nomenclature as for Table V) respectively. H10, H11, H12 are positive controls of different strength. **B**. Example of heterodimeric screening against the HBB 5 target of I_*Cre*I refined mutants cleaving HBB5.4 sequence. In the filter, positive mutants with an increased efficiency of cleavage of HBB5 target are indicated by circles and squares. The sequences of positive mutants at positions C2, C11, D3 and E2 (squares) are KNSYQS / ARSER +24V+43L+80K+96R, KNTYQS / ARSER + 19S+80K+96R+132V, KNTYQS / ARSER +80K + 96R +132V, KNDYQS/ARSER +19S+80K+96R +132V (same nomenclature as for Table V) respectively. H10, H11, H12 are positive controls of different strength.

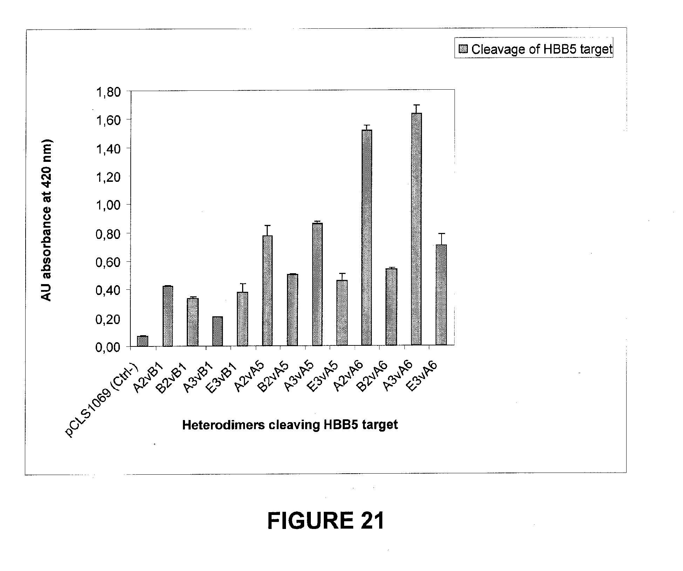

- figure 21 illustrates extrachromosomal cleavage of the HBB5 DNA target in CHO mammalian cells. Twelve heterodimeric combinations were tested for their efficacy of cleavage. The sequences of the optimized mutants tested are described in Table XII. The negative control pCLS 1069 is an empty expression vector.

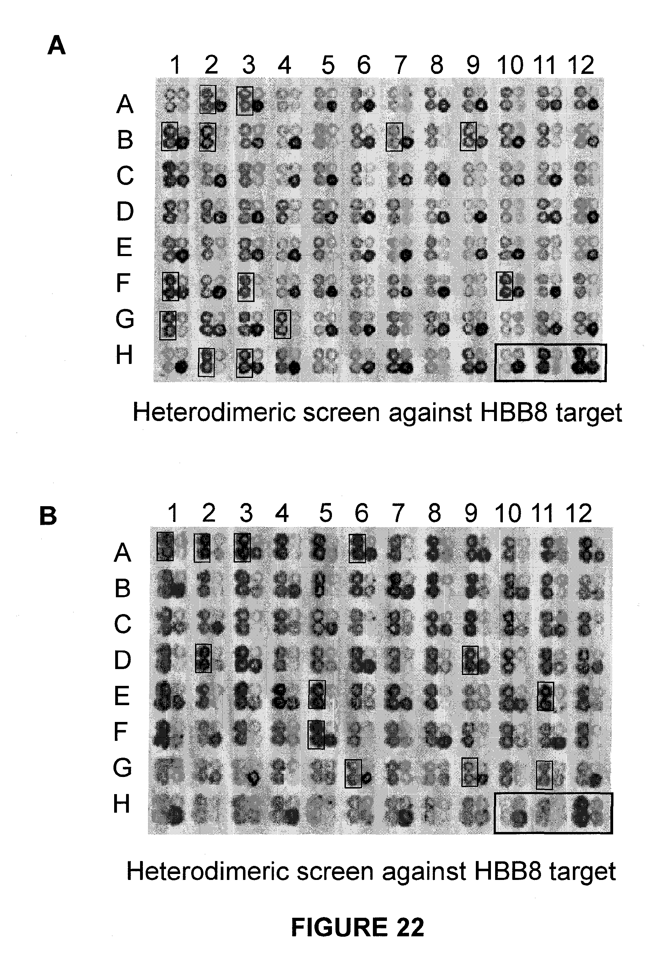

- figure 22 illustrates cleavage of the HBB8 target. A series of I-CreI N75 optimized site directed mutants with F54L, F87L, V105A, I132V mutations (panel A) or G19S and E80K mutations (panel B) and cutting HBB8.3 are coexpressed with one mutant cutting HBB8.4. Cleavage is tested with the HBB8 target. H10, H11, H12 are positive controls of different strength.

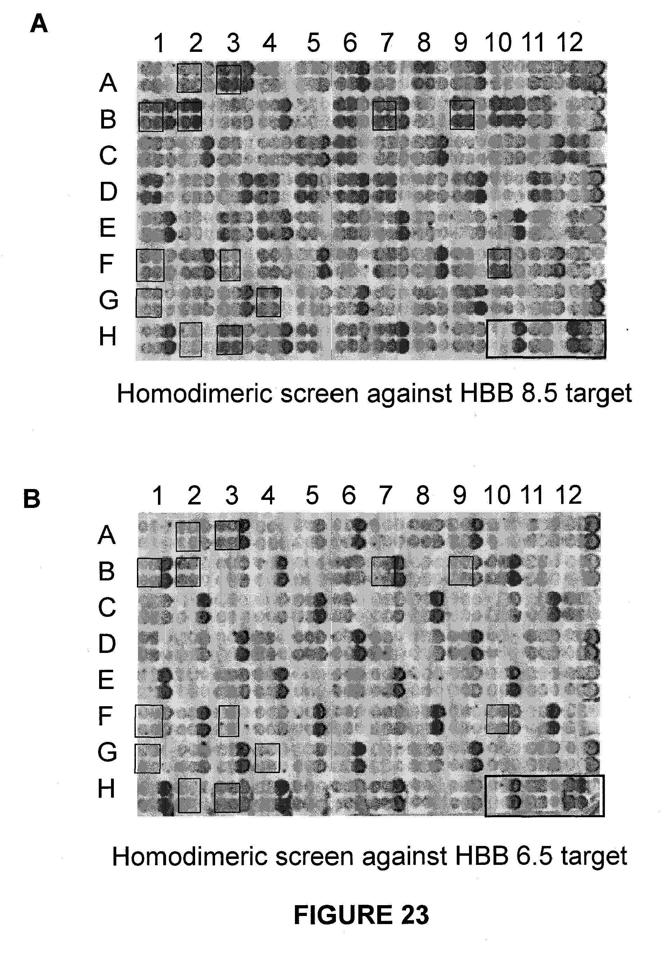

- figure 23 illustrates specificity of cleavage of HBB8.5 and HBB6.5 targets. A series of I-CreI N75 optimized site directed mutants with F54L, F87L, V105A, I132V mutations and cutting HBB8.3 were tested in an homodimeric screen for their efficacy of cleavage of the HBB8.5 target (panel A) or the HBB6.5 target (panel B). H10, H11, H12 are positive controls of different strength.

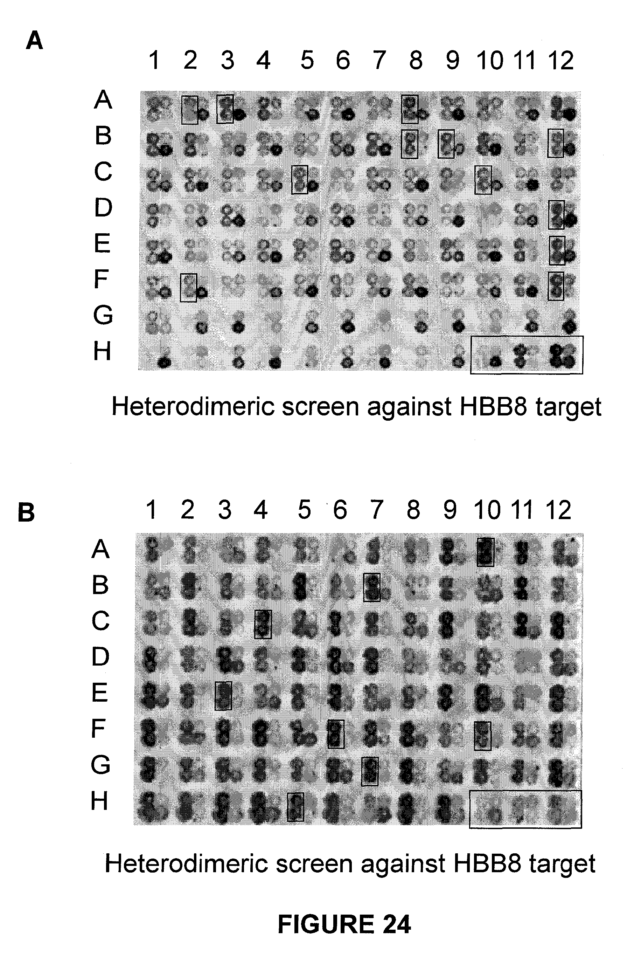

- figure 24 illustrates cleavage of the HBB8 target. A series of I-CreI N75 optimized site directed mutants with F54L, E80K, F87L, V105A, I132V mutations (panel A) or G19S mutations (panel B) and cutting HBB8.4 are coexpressed with one mutant cutting HBB8.3. Cleavage is tested with the HBB8 target. H10, H11, H12 are positive controls of different strength.

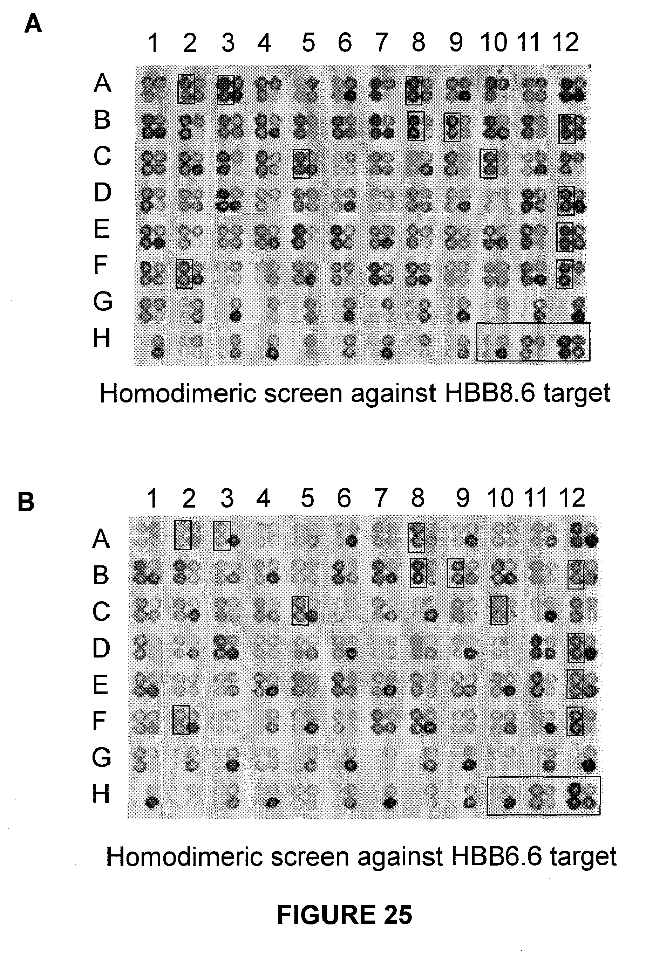

- figure 25 illustrates specificity of cleavage of HBB8.6 and HBB6.6 derived targets. A series of I-CreI N75 optimized site directed mutants with F54L, E80K, F87L, V105A, I132V mutations and cutting HBB8.4 were tested in an homodimeric screen for their efficacy of cleavage of the HBB8.6 target (panel A) or the HBB6.6 target (panel B). H10, H11, H12 are positive controls of different strength.

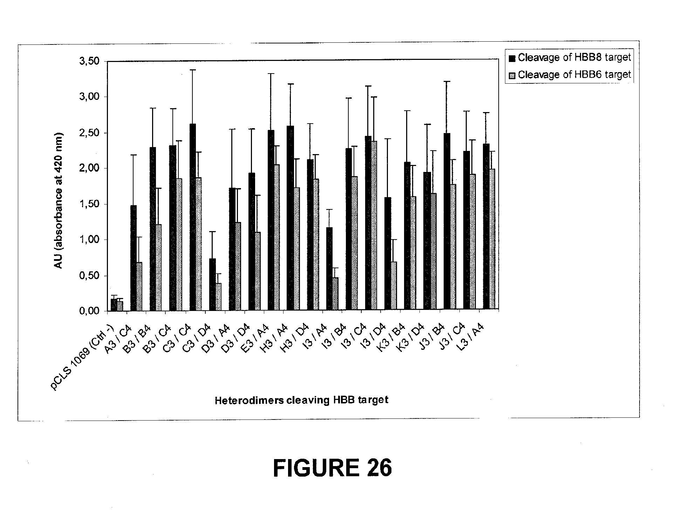

- figure 26 illustrates extrachromosomal cleavage efficiency of meganucleases targeting the HBB derived sequences in CHO mammalian cells. Nineteen heterodimeric combinations were compared for their efficacy of cleavage of the targets HBB8 and HBB6. The sequences of the optimized mutants tested are described in Table XXI. The negative control pCLS 1069 is an empty expression vector.

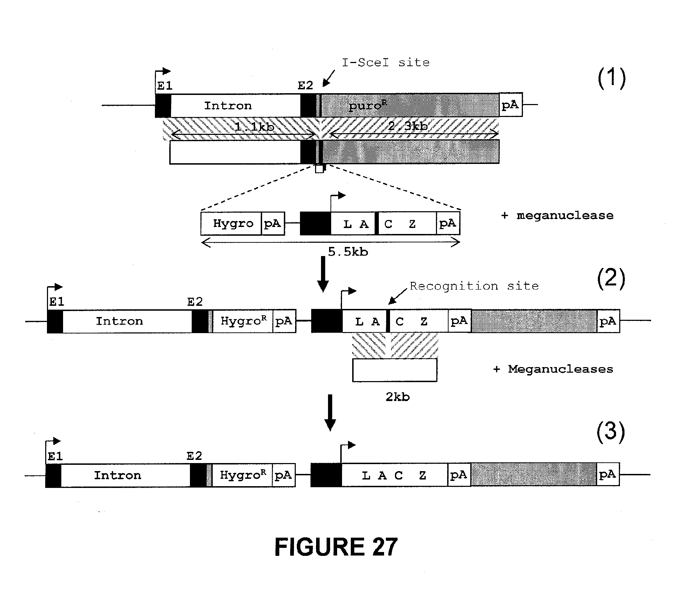

- figure 27 represents a reporter system in mammalian cells. The puromycin resistance gene, interrupted by an I-*Sce*I cleavage site 132bp downstream of the start codon, is under the control of the EFIα promoter (1). The transgene has been stably expressed in CHO-K1 cells in single copy. In order to introduce meganuclease target sites in the same chromosomal context, the repair matrix is composed of i) a promoterless hygromycin resistance gene, ii) a complete lacZ expression cassette and iii) two arms of homologous sequences (1.1 kb and 2.3 kb). Several repair matrixes have been constructed differing only by the recognition site that interrupts the lacZ gene (2). Thus, very similar cell lines have been produced as A1 cell line, I-*Sce*I cell line and I-*Cre*I cell line. A functional lacZ gene is restored when a lacZ repair matrix (2kb in length) is co-transfected with vectors expressing a meganuclease cleaving the recognition site (3). The level of meganuclease-induced recombination can be inferred from the number of blue colonies or foci after transfection.

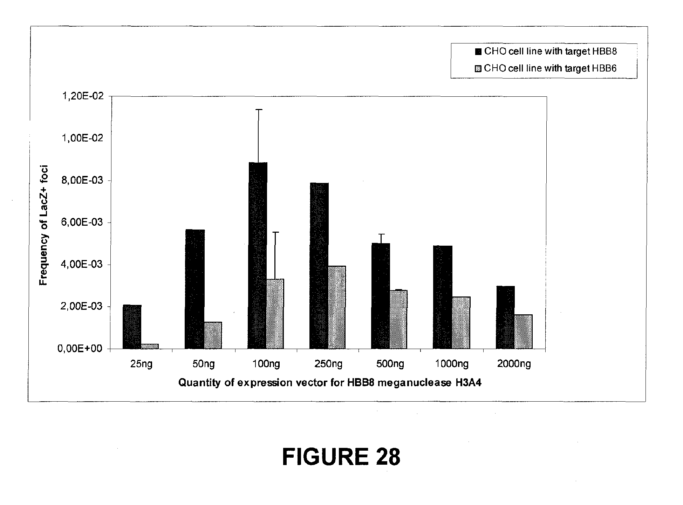

- figure 28 illustrates chromosomal cleavage efficiency of meganucleases cleaving the HBB8 DNA target sequence. The frequency of repair of the LacZ gene is detected after transfection of CHO cells containing an HBB8 or HBB6 chromosomal reporter system, with a repair matrix and various quantities of meganuclease expression vectors, coding for the optimized HBB8 heterodimer H3/A4 (see Table XXI for the sequences of the mutants).

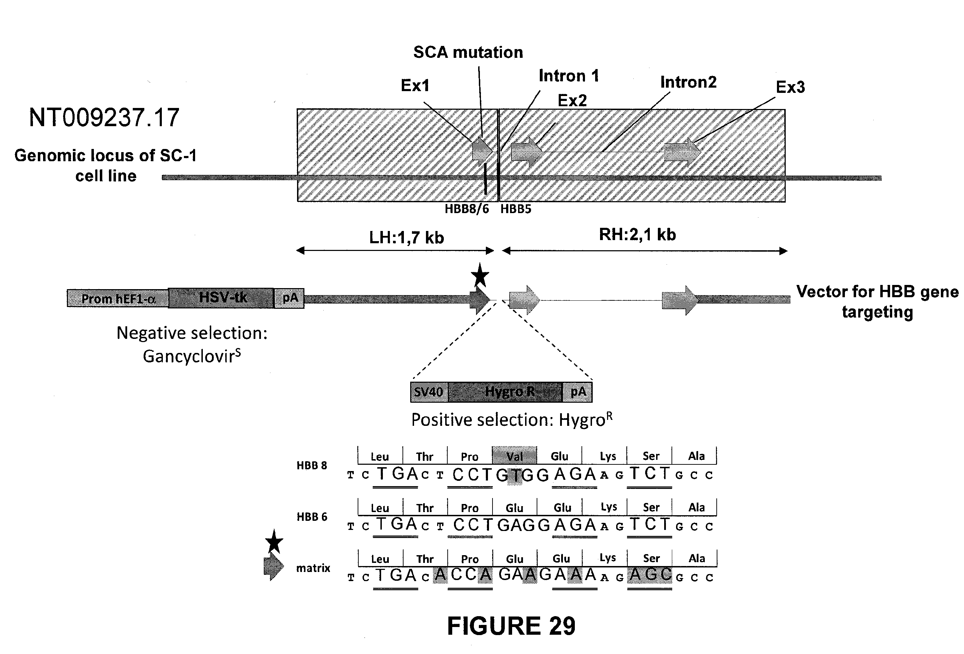

- figure 29 represents a knock-in vector for gene targeting of the human HBB locus. The structure of the human genomic HBB locus is depicted. The vector usefull for the gene targeting is described. LH and RH correspond to the left and right arms of homology. The markers for selection of clones to Hygromycin and Ganciclovir are shown. The modification of the sequence of the HBB6 site of the matrix is indicated.

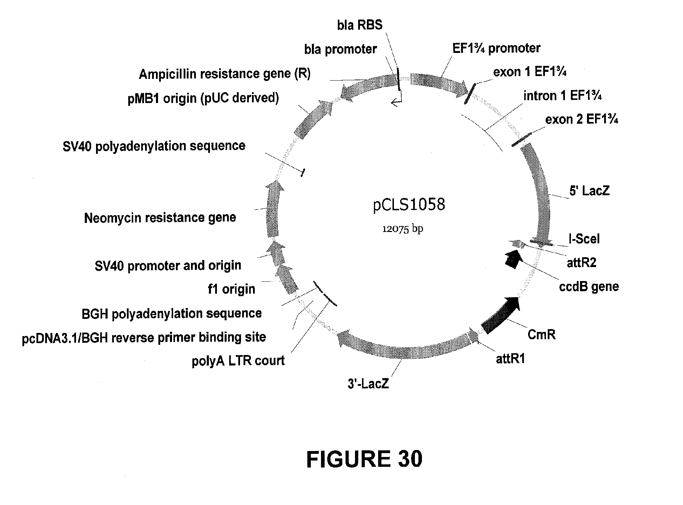

- figure 30 represents the pCLS1058 vector map.

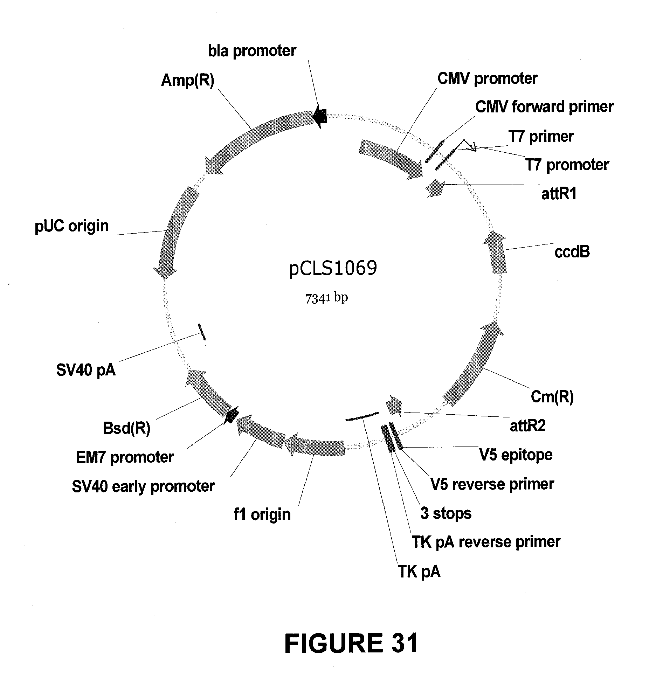

- figure 31 represents the pCLS1069 vector map.

It should be clearly understood, however, that these examples are given only by way of illustration of the subject of the invention, of which they in no way constitute a limitation.

### Example 1: Making of meganucleases cleaving HBB5.3

This example shows that I-*Cre*I mutants can cut the HBB5.3 DNA target sequence derived from the right part of the HBB5 target in a palindromic form (Figure 4). Target sequences described in this example are 22 bp palindromic sequences. Therefore, they will be described only by the first 11 nucleotides, followed by the suffix _P. For example, target HBB 5.3 will be noted also t**ggt**ct**cct**gt_P (SEQ ID NO: 25). HBB5.3 is similar to 5CCT_P at positions ±1, ±2, ±3, ±4, ±5 and to 10GGT_P at positions ±1, ±2, ±8, ±9 and ±10. It was hypothesized that positions ±6, ±7 and ±11 would have little effect on the binding and cleavage activity. Mutants able to cleave 5CCT_P (caaaac**cct**gt_P; SEQ ID NO: 22) were previously obtained by mutagenesis on I-*Cre*I N75 at positions 44, 68, 70, 75 and 77, as described in Arnould et al., J. Mol. Biol., 2006, 355, 443-458 and International PCT Applications WO 2006/097784, WO 2006/097853. Mutants able to cleave the 10GGT_P target (c**ggt**acgtcgt_P; SEQ ID NO: 20) were obtained by mutagenesis on I-*Cre*I N75 and D75 at positions 28, 30, 32, 33, 38, 40 and 70 (Smith et al, Nucleic Acids Res., 2006, 34, e149 and International PCT Applications WO 2007/049095 and WO 2007/057781). Thus combining such pairs of mutants would allow for the cleavage of the HBB 5.3 target. Both sets of proteins are mutated at position 70. However, two separable functional subdomains exist (Smith et al, Nucleic Acids Res., 2006, 34, e149 and International PCT Applications WO 2007/060495 and WO 2007/049156). That implies that this position has little impact on the specificity in base 10 to 8 of the target. Therefore, to check whether combined mutants could cleave the HBB 5.3 target, mutations at positions 44, 68, 70, 75 and 77 from proteins cleaving 5CCT_P were combined with the 28, 30, 32, 33, 38 and 40 mutations from proteins cleaving 10GGT_P.

### 1) Material and Methods

The method for producing meganuclease variants and the assays based on cleavage-induced recombination in mammal or yeast cells, which are used for screening variants with altered specificity are described in the International PCT Application WO 2004/067736; Epinat et al., Nucleic Acids Res., 2003, 31, 2952-2962; Chames et al., Nucleic Acids Res., 2005, 33, e178, and Arnould et al., J. Mol. Biol., 2006, 355, 443-458. These assays result in a functional LacZ reporter gene which can be monitored by standard methods.

### a) Construction of target vector

The target was cloned as follow: oligonucleotide corresponding to the target sequence flanked by gateway cloning sequence was ordered from Proligo: 5' tggcatacaagttttggtctcctgtacaggagaccaacaatcgtctgtca 3' (SEQ ID NO: 33). Double-stranded target DNA, generated by PCR amplification of the single stranded oligonucleotide, was cloned using the Gateway protocol (INVITROGEN) into yeast reporter vector (pCLS1055, Figure 14). Yeast reporter vector was transformed into *S. cerevisiae* strain FYBL2-7B (*MAT α, urα3Δ851, trp1Δ63, leu2Δ1, lys2Δ202*).

### b) Construction of combinatorial mutants

I-*Cre*I mutants cleaving 10GGT_P or 5CCT_P were identified as described previously in Smith et al, Nucleic Acids Res., 2006, 34, e149; International PCT Applications WO 2007/049095, WO 2007/057781, and Arnould et al., J. Mol. Biol., 2006, 355, 443-458; International PCT Applications WO 2006/097784, WO 2006/097853, respectively for the 10GGT_P or 5CCT_P targets. In order to generate I-*Cre*I derived coding sequence containing mutations from both series, separate overlapping PCR reactions were carried out that amplify the 5' end (aa positions 1-43) or the 3' end (positions 39-167) of the *I-CreI* coding sequence. For both the 5' and 3' end, PCR amplification is carried out using Ga110F (5'-gcaactttagtgctgacacatacagg-3'; SEQ ID NO: 34) or Gal10R (5'-acaaccttgattggagacttgacc-3'; SEQ ID NO: 35) primers specific to the vector (pCLS0542, Figure 13) and primers specific to the I-*Cre*I coding sequence for amino acids 39-43 (assF 5'-ctannnttgaccttt-3' (SEQ ID NO: 36) or assR 5'-aaaggtcaannntag-3' (SEQ ID NO: 37) where nnn code for residue 40. The PCR fragments resulting from the amplification reaction realized with the same primers and with the same coding sequence for residue 40 were pooled. Then, each pool of PCR fragments resulting from the reaction with primers Gal10F and assR or assF and Gal10R was mixed in an equimolar ratio. Finally, approximately 25 ng of each final pool of the two overlapping PCR fragments and 75 ng of vector DNA (pCLS0542) linearized by digestion with *Nco*I and *Eag*I were used to transform the yeast *Saccharomyces cerevisiae* strain strain FYC2-6A (MATα, trp1Δ63, leuΔ1, his3Δ200) using a high efficiency LiAc transformation protocol (Gietz and Woods, Methods Enzymol., 2002, 350, 87-96). An intact coding sequence containing both groups of mutations is generated by *in vivo* homologous recombination in yeast.

### c) Mating of meganuclease expressing clones and screening in yeast

Screening was performed as described previously (Arnould et al., J. Mol. Biol., 2006, 355, 443-458). Mating was performed using a colony gridder (QpixII, Genetix). Mutants were gridded on nylon filters covering YPD plates, using a low gridding density (about 4 spots/cm²). A second gridding process was performed on the same filters to spot a second layer consisting of different reporter-harboring yeast strains for each target. Membranes were placed on solid agar YPD rich medium, and incubated at 30°C for one night, to allow mating. Next, filters were transferred to synthetic medium, lacking leucine and tryptophan, with galactose (1%) as a carbon source, and incubated for five days at 37°C, to select for diploids carrying the expression and target vectors. After 5 days, filters were placed on solid agarose medium with 0.02% X-Gal in 0.5 M sodium phosphate buffer, pH 7.0, 0.1% SDS, 6% dimethyl formamide (DMF), 7mM β-mercaptoethanol, 1% agarose, and incubated at 37°C, to monitor β-galactosidase activity. Results were analyzed by scanning and quantification was performed using appropriate software.

### d) Sequencing of mutants

To recover the mutant expressing plasmids, yeast DNA was extracted using standard protocols and used to transform *E. coli.* Sequence of mutant ORF were then performed on the plasmids by MILLEGEN SA. Alternatively, ORFs were amplified from yeast DNA by PCR (Akada et al., Biotechniques, 2000, 28, 668-670), and sequence was performed directly on PCR product by MILLEGEN SA.

### 2) Results

I-*Cre*I combinatorial mutants were constructed by associating mutations at positions 44, 68, 70, 75 and 77 with the 28, 30, 32, 33, 38 and 40 mutations on the I-*Cre*I N75 or D75 scaffold, resulting in a library of complexity 1935. Examples of combinations are displayed on Table II. This library was transformed into yeast and 3456 clones (1.8 times the diversity) were screened for cleavage against HBB 5.3 DNA target (t**ggt**ct**cct**gt_P; SEQ ID NO: 25). 366 positives clones were found, which after sequencing and validation by secondary screening turned out to correspond to 176 different novel endonucleases (see Table II). The 20 novel endonucleases presented in Table II correspond to the sequences SEQ ID NO: 38 to 57 in the sequence listing. Examples of positives mutants cutting the HBB 5.3 target are shown in Figure 6.

Throughout the text and figures of the examples, combinatorial mutants sequences are named with an eleven letter code, after residues at positions 28, 30, 32, 33, 38, 40, 44, 68 and 70, 75 and 77. For example, KCSRQS/ KASDI stands for I-*Cre*I K28, C30, S32, R33, Q38, S40, K44, A68, S70, D75 and I77 (I-*Cre*I 28K30C32S33R38Q40S44K68A70S75D77I). Parental mutants are named with a six letter code, after residues at positions 28, 30, 32, 33, 38 and 40 or a five letter code, after residues at positions 44, 68, 70, 75 and 77. For example, KCSRQS stands for I-*Cre*I K28, C30, S32, R33, Q38 and S40K, and KASDI stands for I-*Cre*I K44, A68, S70, D75 and I77.

**Table II: Cleavage of the HBB5.3 target by the combinatorial variants**

| **Amino acids at positions 28, 30, 32, 33, 38 and 40** **(KCSRQS stands for K28, C30, S32, R33, Q38 and S40)** | **Amino acids at positions 44, 68, 70, 75, and 77** **(KASDI stands for K44, A68, S70, D75 and 177)** | | | | | | | | | |
|---|---|---|---|---|---|---|---|---|---|---|
| | **KASDI** | **KASNT** | **KSSNI** | **KSSNL** | **KYSDR** | **KYSDT** | **KYSNN** | **KYSNR** | **KYSYN** | **RYSNQ** |
| **KCSRQS** | | | | | + (SEQ ID NO 38) | | | | | |
| **KKSAQS** | | + (SEQ ID NO 39) | | | | | | | | + (SEQ ID NO 40) |
| **KNGHQS** | | | | | | | | | | + (SEQ ID NO 41) |
| **KNSHQS** | | | | | | + (SEQ ID NO 42) | | | | |
| **KNSRRS** | + (SEQ ID NO 43) | + (SEQ ID NO 44) | | | | + (SEQ ID NO 45) | | | | + (SEQ ID NO 46) |
| **KNSSKS** | + (SEQ ID NO 47) | | | | | + (SEQ ID NO 48) | + (SEQ ID NO 49)* | | + (SEQ ID NO 50) | |
| **KNSSTS** | | | | + (SEQ ID NO 51) | | | | | | |
| **KRDYQS** | | + (SEQ ID NO 52) | | | | | | + (SEQ ID NO 53) | | |
| **KSSHQS** | | | | | + (SEQ ID NO 54) | | | | | |
| **KTSHRS** | | + (SEQ ID NO 55) | | | | + (SEQ ID NO 56) | | | | |
| **KTSRHS** | | | + (SEQ ID NO 57) | | | | | | | |

| | | | | | | | | | | |
|---|---|---|---|---|---|---|---|---|---|---|
| Only 110 out of the 1935 combinations are displayed + indicates cleavage of the HBB5.3 target by the combinatorial variant * SEQ ID: 49 is the same as SEQ ID: 163; the mutation in position 165 was detected after a second round of sequencing | | | | | | | | | | |

### Example 2: Making of meganucleases cleaving HBB 5.4

This example, shows that I-CreI variants can cleave the HBB5.4 DNA target sequence derived from the left part of the HBB5 target in a palindromic form (Figure 4). All target sequences described in this example are 22 bp palindromic sequences. Therefore, they will be described only by the first 11 nucleotides, followed by the suffix _P. For example, HBB5.4 will be called c**aag**ac**agg**gt_P (SEQ ID NO: 26). HBB5.4 is similar to 5AGG_P at positions ±1, ±2, ±3, ±4, ±5, ±6, ±7, ±9, ±10 and ±11 and to 10AAG_P at positions ±1, ±2, ±6, ±7, ±8, ±9, ±10 and ±11. It was hypothesized that positions ±6 and ±11 would have little effect on the binding and cleavage activity. Mutants able to cleave 5AGG_P were obtained by mutagenesis on *I-CreI* N75 at positions 44, 68, 70, 75 and 77, as described in Arnould et al., J. Mol. Biol., 2006, 355, 443-458 and International PCT Applications WO 2006/097784, WO 2006/097853. Mutants able to cleave the 10AAG_P target were obtained by mutagenesis on I-*Cre*I N75 and D75 at positions 28, 30, 32, 33, 38, 40, and 70, as described in Smith et al, Nucleic Acids Res., 2006, 34, e149 and International PCT Applications WO 2007/049095 and WO 2007/057781). Thus combining such pairs of mutants should allow for the cleavage of the HBB5.4 target.

Both sets of proteins are mutated at position 70. However, two separable functional subdomains exist (Smith et al, Nucleic Acids Res., 2006, 34, e149 and International PCT Applications WO 2007/060495 and WO 2007/049156). That implies that this position has little impact on the specificity in base 10 to 8 of the target. Therefore, to check whether combined mutants could cleave the HBB5.4 target, mutations at positions 44, 68, 70, 75 and 77 from proteins cleaving 5AGG_P (caaaac**agg**gt_P; SEQ ID NO: 23) were combined with the 28, 30, 32, 33, 38, 40 mutations from proteins cleaving 10AAG_P (c**aag**acgtcgt_P; SEQ ID NO: 21).

### 1) Material and Methods

See example 1 except that 25 ng of the PCR product and 75ng of vector DNA (pCLS1107, Figure 15) linearized by digestion with *DraIII* and *NgoMIV* were used to transform the yeast *Saccharomyces cerevisiae* strain FYC2-6A (MATα, trp1Δ63, leu2Δ1, his3Δ200) using a high efficiency LiAc transformation protocol (Gietz and Woods 2002). Expression plasmids containing an intact coding sequence for the I-*Cre*I mutant is generated *by in vivo* homologous recombination in yeast.

### 2) Results

I-*Cre*I combinatorial mutants were constructed by associating mutations at positions 44, 68, 70, 75 and 77 with the 28, 30, 32, 33, 38 and 40 mutations on the I-*Cre*I N75 or D75 scaffold, resulting in a library of complexity 920. Examples of combinatorial mutants are displayed on Table III. This library was transformed into yeast and 1728 clones (1.9 times the diversity) were screened for cleavage against HBB5.4 DNA target (c**aag**ac**agg**gt_P; SEQ ID NO: 26). 25 positives clones were found, which after sequencing and validation by secondary screening turned out to be correspond to 19 different novel endonucleases (see Table III). The 19 novel endonucleases presented in Table III correspond to the sequences SEQ ID NO: 58 to 76 in the sequence listing. Examples of positives mutants cutting the HBB5.4 target are shown in Figure 7.

**Table III: Cleavage of the HBB5.4 target by the combinatorial variants**

| **Amino acids at positions 28, 30, 32, 33, 38 and 40** **(ex : KGSYQS stands for K28, and S40)** | **Amino acids at positions 44, 68, 70, 75, and 77** **(ex: ARSER stands for A44, R68, S70, E75 and R77)** | | | | | | | | | |
|---|---|---|---|---|---|---|---|---|---|---|
| | **ARSER** | **RYSET** | **RYSEV** | **TPSER** | **TRSER** | **TRSYI** | **TYSER** | **YRSQV** | **ARSYQ** | **NRSYI** |
| **KGSYQS** | + (SEQ ID NO 58) | + (SEQ ID NO 59) | + (SEQ ID NO 60) | | + (SEQ ID NO 61) | | | | | |
| **KNDYQS** | + (SEQ ID NO 62) | | | | | | | | | |
| **KNQYQS** | + (SEQ ID NO 63) | | | | | | | | | |
| **KNSYQS** | + (SEQ ID NO 64) | | | + (SEQ ID NO 65) | + (SEQ ID NO 66) | | | | | |
| **KNTYQS** | + (SEQ ID NO 67) | + (SEQ ID NO 68) | + (SEQ ID NO 69) | | + (SEQ ID NO 70) | | + (SEQ ID NO 71) | | | |
| **KQSRQS** | + (SEQ ID NO 72) | | | | | | | | | |
| **KSSHQS** | | | | | | + (SEQ ID NO 73) | | | | |
| **KSSRQS** | | | | | + (SEQ ID NO 74) | | | | | |
| **SNSYQK** | | | | | + (SEQ ID NO 75) | | | + (SEQ ID NO 76) | | |
| **KTSHQS** | | | | | | | | | | |
| **KNSRQA** | | | | | | | | | | |

| | | | | | | | | | | |
|---|---|---|---|---|---|---|---|---|---|---|
| *Only 110 out of the 920 combinations are displayed + indicates cleavage of the HBB5.4 target by the combinatorial variant | | | | | | | | | | |

### Example 3: Making of meganucleases cleaving HBB5

I-*Cre*I mutants able to cleave each of the palindromic HBB5 derived targets (HBB5.3 and HBB5.4) were identified in examples 1 and 2. Pairs of such mutants (one cutting HBB5.3 and one cutting HBB5.4) were co-expressed in yeast. Upon co-expression, there should be three active molecular species, two homodimers, and one heterodimer. It was assayed whether the heterodimers that should be formed cut the HBB5.2 and HBB5 targets.

### 1) Material and Methods

### a) Cloning of mutants in yeast expression vector marked with Kan^{R}

To co-express two I-*Cre*I mutants in yeast, mutants cutting the HBB5.4 sequence were subcloned in a a yeast expression vector marked with a kanamycin resistance gene (pCLS1107, Figure 15). Mutants were amplified by PCR reaction using primers common for pCLS0542 and pCLS1107: Gal10F 5'-gcaactttagtgctgacacatacagg-3' (SEQ ID NO: 34) and Gal10R 5'-acaaccttgattggagacttgacc-3'(SEQ ID NO: 35). Approximately 25 ng of PCR fragment and 75 ng of vector DNA (pCLS1107) linearized by digestion with *DraIII* and *NgoMIV* are used to transform the yeast *Saccharomyces cerevisiae* strain FYC2-6A (*MATα, trp1Δ63, leu2Δ1, his3Δ200*) using a high efficiency LiAc transformation protocol. An intact coding sequence for the I-*Cre*I mutant is generated by *in vivo* homologous recombination in yeast. Each yeast strain containing a mutant cutting the HBB5.4 target subcloned in pCLS1107 vector was then mated with yeast carrying the HBB5.4 target to validate it. To recover the mutant expressing plasmids, yeast DNA was extracted using standard protocols and used to transform *E. coli.* and prepare *E. coli* DNA.

### b) Mutants coexpression

Yeast strain expressing a mutant cutting the HBB5.3 target in pCLS0542 expression vector was transformed with DNA coding for a mutant cutting the HBB5.4 target in pCLS 1107 expression vector. Transformants were selected on - L Glu + G418 medium.

### c) Mating of meganucleases coexpressing clones and screening in yeast

Mating was performed using a colony gridder (QpixII, Genetix). Mutants were gridded on nylon filters covering YPD plates, using a low gridding density (about 4 spots/cm²). A second gridding process was performed on the same filters to spot a second layer consisting of different reporter-harbouring yeast strains for each target. Membranes were placed on solid agar YPD rich medium, and incubated at 30°C for one night, to allow mating. Next, filters were transferred to synthetic medium, lacking leucine and tryptophan, adding G418, with galactose (1%) as a carbon source, and incubated for five days at 37°C, to select for diploids carrying the expression and target vectors. After 5 days, filters were placed on solid agarose medium with 0.02% X-Gal in 0.5 M sodium phosphate buffer, pH 7.0, 0.1% SDS, 6% dimethyl formamide (DMF), 7mM β-mercaptoethanol, 1% agarose, and incubated at 37°C, to monitor β-galactosidase activity. Results were analyzed by scanning and quantification was performed using appropriate software.

### 2) Results

Co-expression of mutants cleaving the HBB5.3 (8 mutants) and HBB5.4 (10 mutants) sequences resulted in efficient cleavage of the HBB5.2 target in almost all cases (Figure 8 panel A). However, none of these combinations was able to cut the HBB5 natural target (Figure 8 panel B) that differs from the HBB5.2 sequence just by 3 bp at positions -2, +1 and +2. (Figure 4). Functional combinations cleaving HBB5.2 target are summarized in Table IV.

**Table IV: Combinations that resulted in cleavage of HBB5.2 target**

| **Mutant HBB5.4, amino acids at positions 28, 30, 32, 33, 38, 40144 68707577** **(ex KGSYQSIRYSET: stands for K28, G30, S32 , Y33 , Q38, S40/ R44, Y68, S70, E75 and T77)** | **Mutant HBB5.3,** **amino acids at positions 28,30,32,33,38,40 / 44,68,70,75 and 77** **(ex: KNGHQS/RYSNQ stands for K28, N30, G32 , H33 , Q38, S40/ R44, Y68, S70, N75 and Q77)** | | | | | | | |
|---|---|---|---|---|---|---|---|---|
| | **KNGHQS/ RYSNQ** | **KKSAQS/ RYSNQ** | **KNSSKS/ KYSNN*** | **KRDYQS/ KYSNR** | **KNSRRS / RYSNQ** | **KTSHRS/ KYSDT** | **KNSGKS/ KYSYN** | **KCSRQS/ KYSDR** |
| **KGSYQS/ RYSET** | + | + | + | + | + | + | + | + |
| **KNSYQS/ ARSER** | + | + | + | + | + | + | + | + |
| **KNQYQS/ ARSER** | + | + | + | + | + | + | + | + |
| **KNDYQS/ ARSER** | + | + | + | + | + | + | + | + |
| **KSSRQS/ TRSER** | + | + | + | + | + | + | + | + |
| **KNTYQS/ TRSER** | + | + | + | + | + | + | + | + |
| **KGSYQS/ ARSER** | + | + | + | + | + | + | + | + |
| **KGSYQS/ TRSER** | + | + | + | + | + | + | + | + |
| **KNTYQS/ ARSER** | + | + | + | + | + | + | + | + |
| **KQSRQS/ ARSER** | + | + | + | + | + | + | + | + |

| | | | | | | | | |
|---|---|---|---|---|---|---|---|---|
| + indicates that the heterodimeric mutant was cleaving the HBB5.2 target. * this mutant is referenced as SEQ ID NO: 49 which is the same as SEQ ID: 163, the mutation in position 165 was detected after a second round of sequencing | | | | | | | | |

### Example 4: Making of meganucleases cleaving HBB5 by random mutagenesis of proteins cleaving HBB 5.4 and assembly with proteins cleaving HBB5.3

I-*Cre*I mutants able to cleave the non palindromic HBB5.2 target were identified by assembly of mutants cleaving the palindromic HBB5.3 and HBB 5.4 target. However, none of these combinations was able to cleave HBB5, which differs from HBB5.2 only by 3 bp at positions -2, +1 and +2 (Figure 4).

Therefore, protein combinations cleaving HBB5.2 were mutagenized, and mutants cleaving HBB5 efficiently were screened. According to the structure of the *I-CreI* protein bound to its target, there is no contact between the 4 central base pairs (positions -2 to 2) and the I-*Cre*I protein (Chevalier et al., Nat. Struct. Biol., 2001, 8, 312-316; Chevalier B.S. and Stoddard B.L., Nucleic Acids Res., 2001, 29, 3757-3754; Chevalier et al., J. Mol. Biol., 2003, 329, 253-269). Thus, it is difficult to rationally choose a set of positions to mutagenize, and mutagenesis was done on the whole protein. Random mutagenesis results in high complexity libraries, and the complexity of the variants libraries to be tested was limited by mutagenizing only one of the two components of the heterodimers cleaving HBB 5.2.

Thus, proteins cleaving HBB 5.4 were mutagenized, and it was tested whether they could efficiently cleave HBB 5 when coexpressed with proteins cleaving HBB 5.3.

### 1) Material and Methods

### a) Construction of libraries by random mutagenesis

Random mutagenesis libraries were created on a pool of chosen mutants by PCR using Mn²⁺ or by a two-step PCR process using dNTP derivatives 8-oxo-dGTP and dPTP as described in the protocol from JENA BIOSCIENCE GmbH for the JBS dNTP-Mutagenesis kit. Primers used are preATGCreFor (5'-gcataaattactatacttctatagacacgcaaacacaaatacacagcggccttgccacc-3'; SEQ ID NO: 77) and ICrelpostRev (5'-ggctcgaggagctcgtctagaggatcgctcgagttatcagtcggccgc-3'; SEQ ID NO: 78). Approximately 25 ng of the PCR product and 75 ng of vector DNA (pCLS1107, Figure 15) linearized by digestion with *DrαIII* and *NgoMIV* were used to transform the yeast *Saccharomyces cerevisiae* strain FYC2-6A (MATα, trp1Δ63, leu2Δ1, his3Δ200) using a high efficiency LiAc transformation protocol (Gietz and Woods, Methods Enzymol., 2002, 350, 87-96). Expression plasmids containing an intact coding sequence for the I-*Cre*I mutant were generated by *in vivo* homologous recombination in yeast.

### b) Mutant-target yeast strains, screening and sequencing

The yeast strain FYBL2-7B (MAT a, ura3Δ851, trp1Δ63, leu2Δ1, lys2Δ202) containing the HBB5 target in the yeast reporter vector (pCLS1055, Figure 14) is transformed with mutants cutting the HBB 5.3 target, in the pCLS0542 vector marked with *LEU2* gene, using a high efficiency LiAc transformation protocol. Mutant-target yeasts are used as target strains for mating assays as described in example 1. Positives resulting clones were verified by sequencing (MILLEGEN) as described in example 1.

### 2) Results

Four mutants cleaving HBB5.4 (KGSYQS/RYSET, KGSYQS/ARSER, KGSYQS/TRSER, KNTYQS/ARSER according to the nomenclature of Table IV) were pooled, randomly mutagenized and transformed into yeast. 2280 transformed clones were then mated with a yeast strain that contains (i) the HBB5 target in a reporter plasmid (ii) an expression plasmid containing a mutant that cleaves the HBB5.3 target (KTSHRS/KYSDT or KNSRRS/KYSDT according to nomenclature of Table IV). After mating with this two yeast strains, one positive clone was found to cleave the HBB5 target. In a control experiment, this positive clone was not found to trigger cleavage of HBB5 without co-expression of the KTSHRS/KYSDT or KNSRRS/KYSDT I-*Cre*I mutants. In conclusion, this positive clone contained proteins able to form heterodimers with KTSHRS/KYSDT or KNSRRS/KYSDT leading to cleavage activity for the HBB5 target. The yeast screening assay leading to the identification of the positive clone is shown in Figure 9. Sequence of the positive clone according to the nomenclature of Table IV is listed in Table V.

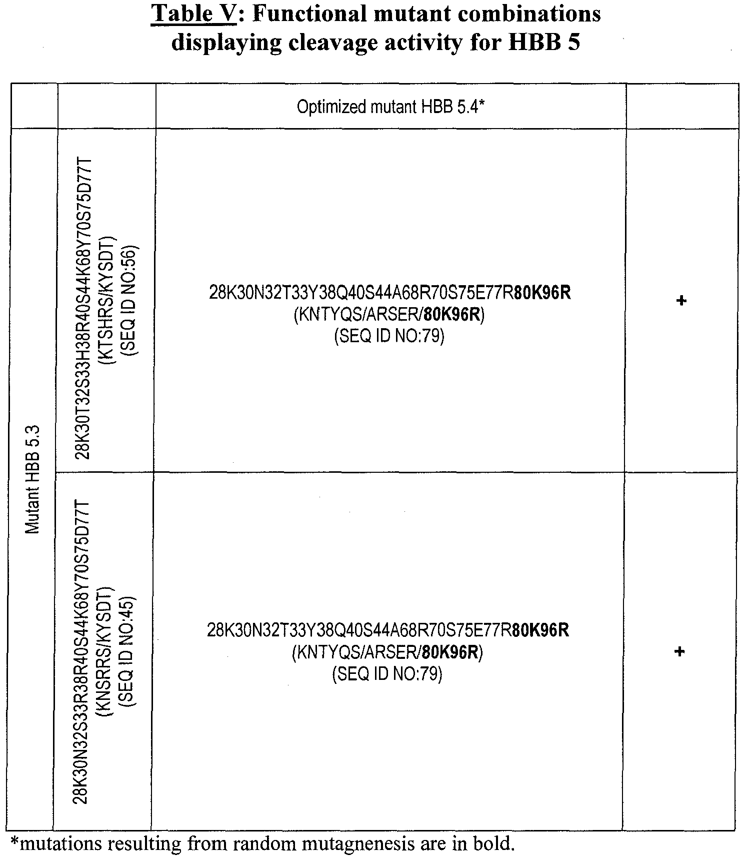

### Example 5: Refinement of meganucleases targeting HBB5 by random mutagenesis and site-directed mutagenesis of protein cleaving HBB5.3 and assembly with proteins cleaving HBB5.4

I-*Cre*I mutants cleaving the palindromic HBB5.3 target were identified and tested by co-expression with mutants cleaving the palindromic HBB5.4 target for their ability to cleave the non palindromic target HBB5 (Example 3). Nevertheless none of the heterodimeric combinations tested was able to cut the HBB5 sequence. In order to obtain a cleavage of this target, I-*Cre*I mutants cleaving the HBB5.3 sequence were randomly mutagenized or site directed mutagenized and new variants cleaving HBB5 with high efficacy, when co-expressed with mutant cleaving the HBB5.4 target, were screened.

Six amino-acid substitutions which enhance the activity of I-*Cre*I derivatives were individually introduced into the coding sequence of proteins cleaving HBB5.3. These mutations correspond to the replacement of Glycine 19 with Serine (G19S), Phenylalanine 54 with Leucine (F54L), Glutamic Acid 80 with Lysine (E80K), Phenylalanine 87 with Leucine (F87L), Valine 105 with Alanine (V105A) and Isoleucine 132 with Valine (I132V). The resulting mutagenized proteins were then tested for their ability to induce efficient cleavage of the HBB5 target, upon co-expression with a mutant cleaving HBB5.4.

### 1) Material and Methods

### a) Construction of libraries by random mutagenesis

Same protocol as in example 4 except that 25 ng of the PCR product and 75 ng of vector DNA (pCLS0542, Figure 13) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz et al, Methods Enzymol, 2002, 350, 87-96). Expression plasmids containing an intact coding sequence for the I-CreI mutant were generated by *in vivo* homologous recombination in yeast.

### b) Construction of libraries by site directed mutagenesis

Libraries were created by PCR on a pool of initial mutants cleaving HBB5.3. As an example, to introduce the G19S substitution into the coding sequences of the mutants, two separate overlapping PCR reactions were carried out that amplify the 5' end (residues 1-24) or the 3' end (residues 14-167) of the I-*Cre*I N75 coding sequence. For both the 5' and 3' end, PCR amplification is carried out using a primer with homology to the vector (Gal110F 5'-gcaactttagtgctgacacatacagg-3' (SEQ ID NO: 34) or GaI10R 5'-acaaccttgattggagacttgacc-3' (SEQ ID NO: 35)) and a primer specific to the I-*Cre*I coding sequence for amino acids 14-24 that contains the substitution mutation G19S (G19SF 5'-gccggctttgtggactctgacggtagcatcatc-3' (SEQ ID NO: 151) or G19SR 5'-gatgatgctaccgtcagagtccacaaagccggc-3' (SEQ ID NO: 152)). The resulting PCR products contain 33 bp of homology with each other. The PCR fragments were purified. Finally, approximately 25 ng of each of the two overlapping PCR fragments and 75 ng of vector DNA (pCLS0542, Figure 13) linearized by digestion with *Nco*I and *Eag*I were used to transform the yeast *Saccharomyces cerevisiae* strain FYC2-6A (MATα, trp1Δ63, leu2Δ1, his3Δ200) using a high efficiency LiAc transformation protocol (Gietz et al, Methods Enzymol, 2002, 350, 87-96). Intact coding sequences containing the G19S substitution were generated *by in vivo* homologous recombination in yeast.

The same strategy was used with the following pair of oligonucleotides to create the other libraries containing the F54L, E80K, F87L, V105A and I132V substitutions, respectively:
* F54LF: 5'-acccagcgccgttggctgctggacaaactagtg-3' (SEQ ID NO: 153) and F54LR: 5'-cactagtttgtccagcagccaacggcgctgggt-3' (SEQ ID NO: 154);
* E80KF: 5'-ttaagcaaaatcaagccgctgcacaacttcctg-3' (SEQ ID NO: 155) and E80KR: 5'-caggaagttgtgcagcggcttgattttgcttaa -3' (SEQ ID NO: 156);
* F87LF: 5'-aagccgctgcacaacctgctgactcaactgcag-3' (SEQ ID NO: 157) and F87LR: 5'-ctgcagttgagtcagcaggttgtgcagcggctt-3' (SEQ ID NO: 158);
* V105AF: 5'-aaacaggcaaacctggctctgaaaattatcgaa-3' (SEQ ID NO: 159) and V105AR: 5'-ttcgataattttcagagccaggtttgcctgttt-3' (SEQ ID NO: 160);
* I132VF: 5'-acctgggtggatcaggttgcagctctgaacgat-3' (SEQ ID NO: 161) and I132VR: 5'-atcgttcagagctgcaacctgatccacccaggt-3' (SEQ ID NO: 162).

### c) Mutant-target yeast strains, screening and sequencing:

The yeast strain FYBL2-7B (MATα, ura3Δ851, trp1Δ63, leu2Δ1, lys2Δ202) containing the HBB 5 target in the yeast reporter vector (pCLS 1 055, Figure 14) was transformed with mutants, in the leucine vector (pCLS0542, Figure 13), cutting the HBB 5.3 target, using a high efficiency LiAc transformation protocol. Mutant-target yeasts were used as target strains for mating assays as described in example 1. Positives resulting clones were verified by sequencing (Millegen) as described in example 1.

### 2) Results

Libraries obtained by random mutagenesis and by site directed mutagenesis were constructed on a pool of fourteen initial I-*Cre*I mutants cleaving HBB5.3 target (Table VI). 2232 and 372 transformed clones for each library were then mated with a yeast strain that (i) contains the HBB5 target in a reporter plasmid (ii) expresses the mutant 28K30N32T33Y38Q40S44A68R70S75E77R+80K+96R also called KNTYQS / ARSER + 80K + 96R, an optimized variant cleaving the HBB5.4 target already described in example 4.

The identification of some positives mutants harbouring the G19S mutation after site directed mutagenesis and cutting efficiently the HBB 5 target are shown in Figure 17, panel A. After mating with this yeast strain, 80 new I-CreI clones cleaving the HBB5 target more efficiently than the initial mutants when forming heterodimers with the mutant cleaving the HBB5.4 target were identified. Sequencing of such 80 positive clones (51 clones obtained by random mutagenesis, 15 clones with G19S mutation, 2 clones with F87L mutation, 3 clones with V105A mutation, 9 clones with I132V mutation) allowed the identification of 49 distinct novel mutants giving higher levels of cleavage of HBB5 target.

As an example, the sequences of 14 I-CreI optimized mutants cleaving the HBB5.3 target when forming heterodimer with the mutant KNTYQS /ARSER + 80K + 96R cleaving HBB5.4 are compiled in Table VII. These optimized variants correspond to the sequences SEQ ID NO: 164 to 177.

**Table VI: Group of fourteen mutants cleaving the HBB5.3 target used for construction of libraries by random mutagenesis and site directed mutagenesis. ND corresponds to a non determined sequence**

| | **Sequence of mutants cleaving HBB5.3 target amino acids at positions 28, 30, 32, 33, 38, 40144, 68, 70, 75 and 77 and additional mutations at different positions ex: 28K30N32S33S38K40S / 44K68Y70S75N77N + 165T stands for K28, N30, S32 , S33 , K38, S401 K44, Y68, S70, N75, N77 and T165** | **SEQ ID NO** |
|---|---|---|
| **1** | **28K30N32G33H38Q40S144R68Y70S75N77Q KNGHQSIRYSNQ** | **41** |
| **2** | **28K30K32S33A38Q40S/44R68Y70S75N77Q KKSAQS/RYSNQ** | **40** |
| **3** | **28K30N32S33S38K40S /44K68Y70S75N77N + 165T KNSSKS I KYSNN + 165T** | **163** |
| **4** | **28K30R32D33Y38Q40S/44K68Y70S75N77R KRDYQS/KYSNR** | **53** |
| **5** | **ND** | |
| **6** | **28K30N32S33R38R40S/44R68Y70S75N77Q KNSRRS/RYSNQ** | **46** |
| **7** | **28K30T32S33R38H40S/44K68S70S75N771 KTSRHS/KSSNI** | **57** |
| **8** | **28K30N32S33S38K40S/44K68Y70S75Y77N KNSSKSIKYSYN** | **49** |
| **9** | **28K30C32S33R38Q40S144K68Y70S75D77R KCSRQSIKYSDR** | **38** |
| **10** | **28K30N32S33S38T40S/44K68S70S75N77L KNSSTS/KSSNL** | **51** |
| **11** | **28K30N32S33H38Q40S/44K68Y70S75D77T KNSHQS/KYSDT** | **42** |
| **12** | **28K30N32S33S38K40S/44K68Y70S75D77T KNSSKS/KYSDT** | **48** |
| **13** | **28K30N32S33R38R40S/44K68Y70S75D77T KNSRRS/KYSDT** | **45** |
| **14** | **28K30S32S33H38Q40S/44K68Y70S75D77R KSSHQS/KYSDR** | **54** |

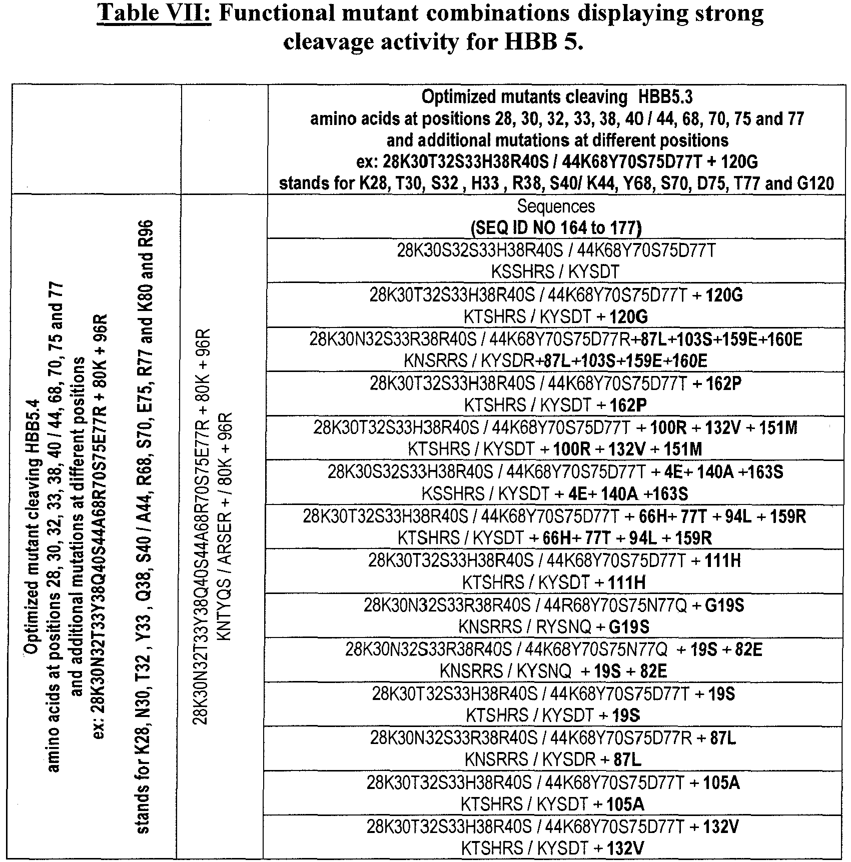

### Example 6: Improvement of cleavage activities of meganucleases targeting HBB5 by site-directed mutagenesis of proteins cleaving HBB5.4 and assembly with proteins cleaving HBB5.3

As described in example 4, just one I-*Cre*I optimized mutant called KNTYQS / ARSER + 80K + 96R cleaving the palindromic HBB5.4 target was already identified from the heterodimeric yeast screening of a library obtained by random mutagenesis and tested by co-expression with mutants cleaving the palindromic HBB5.3 target. In order to increase the number of the I-*Cre*I cutters for the HBB5 sequence, another set of libraries corresponding to site- directed mutagenesis of a pool of mutants cleaving the HBB5.4 target by the insertion of the six amino-acid substitutions G19S, F54L, E80K, F87L, V105A and I132V were constructed.

The resulting proteins were tested for their ability to induce efficient cleavage of the HBB5 target.

### 1) Material and Methods

### a) Construction of libraries by site directed mutagenesis

Same protocol as in example 5 except that 25 ng of each of the two overlapping PCR fragments and 75 ng of vector DNA (pCLS1107, Figure 15) linearized by digestion with *DraIII* and *NgoMIV* were used to transform the yeast *Saccharomyces cerevisiae* strain FYC2-6A (MATα, trp1Δ63, leu2Δ1, his3Δ200) using a high efficiency LiAc transformation protocol (Gietz et al, Methods Enzymol, 2002, 350, 87-96).. Intact coding sequences containing the different mutations were generated *by in vivo* homologous recombination in yeast.

### b) Mutant-target yeast strains, screening and sequencing

The yeast strain FYBL2-7B (MAT a, ura3Δ851, trp1Δ63, leu2Δ1, lys2Δ202) containing the HBB 5 target in the yeast reporter vector (pCLS1055, Figure 14) was transformed with mutants cutting the HBB 5.4 target, cloned in the kanamicyn vector (pCLS1107, Figure 15), using a high efficiency LiAc transformation protocol. Mutant-target yeasts were used as target strains for mating assays as described in example 1. Positives resulting clones were verified by sequencing (Millegen) as described in example 1.

### 2) Results

Libraries constructed by site directed mutagenesis were derived from a pool of twelve I-CreI mutants corresponding to eleven initial mutants and one optimized mutant called KNTYQS / ARSER + 80K + 96R cleaving HBB5.4 target (Table VIII). 372 transformed clones for each library were then mated with a yeast strain that (i) contains the HBB5 target in a reporter plasmid (ii) expresses the mutant 28K30T32S33R38H40S / 44K68S70S75N77I also called KTSRHS / KSSNI, an initial variant cleaving the HBB5.3 target already described in example 3.

Some positives optimized mutants harbouring the G19S mutation after site directed mutagenesis and cutting efficiently the HBB 5 target are shown in Figure 17 panel B. After mating with this yeast strain, 20 new I-Crel clones cleaving the HBB5 target more efficiently than the initial mutants when forming heterodimers with the mutant cleaving the HBB5.3 target were identified. Sequencing of such 20 positive clones (10 clones with G19S mutation, 6 clones with F87L mutation and 4 clones with I132V mutation) allowed the identification of 8 distinct novel mutants. The sequences of such *I-CreI* optimized mutants cleaving the HBB5 target (SEQ ID NO: 179 to 186) are compiled in Table IX.

**Table VIII: Group of twelve mutants cleaving the HBB5.4 target used for construction of libraries by site directed mutagenesis**

| | **Sequence of mutants cleaving HBB5.4 target amino acids at positions 28, 30, 32, 33, 38, 40 / 44, 68, 70, 75 and 77 and additional mutations at different positions ex: 28K30N32T33Y38Q40S /44A68R70S75E77R + 80K + 96R stands for K28, N30, S32 , Y33 , Q38, S40/ A44, R68, S70, E75, R77 and K80 and R96** | **SEQ ID NO** |
|---|---|---|
| **1** | **28K30G32833Y38Q408/44A68R70S75E77R** **KGSYQS/ARSER** | **58** |
| **2** | **28K30G32S33Y38Q40S/44R68Y70S75E77T** **KGSYQS/RYSET** | **59** |
| **3** | **28k30G32S33Y38Q40S/44T68R70S75E77R** **kGSYQS/TRSER** | **61** |
| **4** | **28K30N32D33Y38Q40S/44A68R70S75E77R** **KNDYQS/ARSER** | **62** |
| **5** | **28K30N32Q33Y38Q40S/44A68R70S75E77R** **KNQYQS/ARSER** | **63** |
| **6** | **28K30N32S33Y38Q40S/44A68R70S75E77R** **KNSYQS/ARSER** | **64** |
| **7** | **28K30N32T33Y38Q40S/44A68R70S75E77R** **KNTYQS/ ARSER** | **67** |
| **8** | **28K30N32T33Y38Q40S/44T68R70S75E77R** **KNTYQS/TRSER** | **70** |
| **9** | **28K30Q32S33R38Q40S/44A68R70S75E77R** **KQSRQS/ARSER** | **72** |
| **10** | **28K30S32S33H38Q40S/44T68R70S75Y77I** **KSSHQS/TRSYI** | **73** |
| **11** | **28K30S32S33R38Q40S/44T68R70S75E77R** **KSSRQS/TRSER** | **74** |
| **12** | **28K30N32T33Y38Q40S /44A68R70S75E77R + 80K + 96R** **KNTYQS/ ARSER +80K+96R** | **79** |

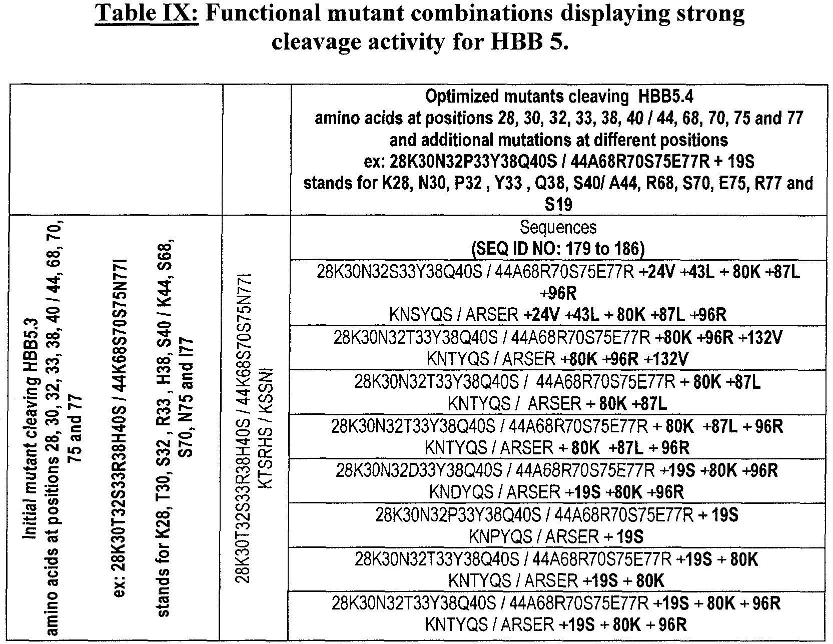

### Example 7: Refinement of the efficiency of the cleavage of the meganucleases targeting HBB5 by random mutagenesis and multiple site-directed mutagenesis of proteins cleaving HBB5.3 and HBB5.4, and assembly with proteins cleaving HBB5.4 and HBB5.3.

The results of the experiments of mutagenesis of mutants targeting the palindromic targets HBB5.3 and HBB5.4 lead to the identification of optimized mutants cleaving more efficiently the sequence HBB5 when tested in heterodimeric co-expression yeast screening assay (see examples 5 and 6). Moreover, as shown in example 6 (Table IX), the sequences of some optimized mutants (KNTYQS / ARSER + 80K +87L, KNTYQS / ARSER +19S + 80K), indicate that the association of the substitutions E80K with F87L and G19S with E80K within the same mutant can lead to an increase of it's efficiency of cleavage of the HBB5 target. In order to obtain a large panel of I-CreI mutants with a maximal efficacy of cleavage of the HBB5 target, new libraries of random and multiple site-directed mutagenesis (Figure 18) concerning the substitutions G19S, F54L, E80K, V105A and I132V were constructed from a pool of optimized mutants targeting the sequences HBB5.3 and HBB5.4 (Table X).

### 1) Material and Methods

### a) Construction of libraries by random mutagenesis

Same protocol as in example 4. For the libraries derived from the pool of the mutants targeting HBB5.3 or HBB5.4 sequences, 25 ng of the PCR product and 75 ng of vector DNA pCLS0542 linearized by digestion with *Nco*I and *Eag*I (Figure 13) or pCLS1107 linearized by digestion with *DraIII* and *NgoMIV* (Figure 15) were used respectively to transform the yeast *Saccharomyces cerevisiae* strain FYC2-6A (MATα, trp1Δ63, leu2Δ1, his3Δ200) using a high efficiency LiAc transformation protocol (Gietz et al, Methods Enzymol, 2002, 350, 87-96). Expression plasmids containing an intact coding sequence for the I-*Cre*I mutant were generated by *in vivo* homologous recombination in yeast.

### b) Construction of libraries by multiple site directed mutagenesis

Same protocol as in example 5 except that to obtain a multiple insertion of the G19S, F54L, E80K, V105A and I132V substitutions into the coding sequences of the mutants cleaving HBB5.3 and HBB5.4 targets, six groups of separate overlapping PCR reactions were carried out that amplify internal fragments of the I-*Cre*I N75 coding sequence containing the sequences between the different mutations. As an example, for the multiple site directed mutagenesis for the insertion of the mutations G19S and F54L, PCR amplification is carried out using primers specific to the I-*Cre*I coding sequence for amino acids 14-24 that contains or not the substitution G19S (G19SF 5'-gccggctttgtggactctgacggtagcatcatc-3' (SEQ ID NO: 151) and G19wtF 5'-gccggctttgtggacggtgacggtagcatcatc -3' (SEQ ID NO: 187)) with primers specific to the I*-Cre*I coding sequence for amino acids 49-59 that contains or not the substitution F54L (F54LR 5'-cactagtttgtccagcagccaacggcgctgggt-3' (SEQ ID NO: 154) and F54wtR 5'- cactagtttgtccagaaaccaacggcgctgggt -3' (SEQ ID NO: 188), leading to the generation of four fragments of PCR with different ends.

The same strategy was used with the following groups of oligonucleotides to create the other internal fragments:
* Gal10F: 5'-gcaactttagtgctgacacatacagg-3' (SEQ ID NO: 34) with G19SR: 5'-gatgatgctaccgtcagagtccacaaagccggc-3' (SEQ ID NO: 152) and G19wtR: 5'-gatgatgctaccgtcaccgtccacaaagccggc-3' (SEQ ID NO: 189)
* F54LF: 5'-acccagcgccgttggctgctggacaaactagtg-3' (SEQ ID NO: 153) and F54wtF: 5'-acccagcgccgttggtttctggacaaactagtg-3' (SEQ ID NO: 190) with E80KR 5'-caggaagttgtgcagcggcttgattttgcttaa-3' (SEQ ID NO: 156) and E80wtR 5'-caggaagttgtgcagcggcttgatttcgcttaa-3' (SEQ ID NO: 191)
* E80KF: 5'-ttaagcaaaatcaagccgctgcacaacttcctg-3' (SEQ ID NO: 155) and E80wtF: 5'-ttaagcgaaatcaagccgctgcacaacttcctg-3' (SEQ ID NO: 192) with V105AR 5'-ttcgataattttcagagccaggtttgcctgttt-3' (SEQ ID NO: 160) and V105wtR 5'-ttcgataattttcagaaccaggtttgcctgttt-3' (SEQ ID NO: 193)
* V105AF: 5'-aaacaggcaaacctggctctgaaaattatcgaa-3' (SEQ ID NO: 159) and V105wtF: 5'-aaacaggcaaacctggttctgaaaattatcgaa-3' (SEQ ID NO: 194) with I132VR 5'-atcgttcagagctgcaacctgatccacccaggt-3' (SEQ ID NO: 162) and I132wtR 5'-atcgttcagagctgcaatctgatccacccaggt-3' (SEQ ID NO: 195)
* I132VF: 5'-acctgggtggatcaggttgcagctctgaacgat-3' (SEQ ID NO: 161) and I132wtF: 5'-acctgggtggatcagattgcagctctgaacgat-3' (SEQ ID NO: 196) with Gal10R 5'-acaaccttgattggagacttgacc-3' (SEQ ID NO: 35)

The resulting overlapping PCR products contain 15 bp of homology with each other. The PCR fragments corresponding to each internal region were then purified pooled and I-CreI coding sequences containing or not the mutations G19S, F54L, E80K, V105A and I132V were generated by PCR assembly. Finally, approximately 25 ng of each assembled PCR fragment obtained with the mutants cleaving HBB5.3 and HBB5.4 targets and 75 ng of vector DNA pCLS0542 (Figure 13) linearized by digestion with *Nco*I and *Eag*I or vector DNA pCLS1107 linearized by digestion with *DraIII* and *NgoMIV* were respectively used to transform the yeast *Saccharomyces cerevisiae* strain FYC2-6A (MATα, trp1Δ63, leu2Δ1, his3Δ200) using a high efficiency LiAc transformation protocol (Gietz *et al,* Methods Enzymol, 2002, 350, 87-96). Intact coding sequences containing the substitutions were generated by *in vivo* homologous recombination in yeast.

### c) Mutant-target yeast strains, screening and sequencing

The yeast strain FYBL2-7B (MAT a, ura3Δ851, trp1Δ63, leu2Δ1, lys2Δ202) containing the HBB 5 target in the yeast reporter vector (pCLS1055, Figure 14) was transformed with mutants cutting the HBB 5.3 target cloned in the leucine vector (pCLS0542, Figure 13) or the mutants cutting the HBB 5.4 target cloned in the kanamycin vector (pCS 1107, Figure 15), using a high efficiency LiAc transformation protocol (Gietz et al, Methods Enzymol, 2002, 350, 87-96). Mutant-target yeasts were used as target strains for mating assays as described in example 1. Positives resulting clones were verified by sequencing (Millegen) as described in example 1.

### 2) Results

Libraries of random mutagenesis or multiple site directed mutagenesis were derived from a pool of eight and six I-*Cre*I mutants corresponding to optimized mutants cleaving the targets HBB5.3 and HBB5.4 (Table X). 2232 or 2790 transformed clones for each kind of library were obtained. The clones corresponding to the libraries derived from the HBB5.3 mutants were mated with a yeast strain that (i) contains the HBB5 target in a reporter plasmid (ii) expresses the mutant 28K30N32S33Y38Q40S / 44A68R70S75E77R +24V +43L + 80K +87L +96R also called KNSYQS / ARSER +24V +43L + 80K +87L +96R, a refined variant cleaving the HBB5.4 target already described in example 6. The clones corresponding to the libraries derived from the HBB5.4 mutants were mated with a yeast strain that (i) contains the HBB5 target in a reporter plasmid (ii) expresses the mutant 28K30S32S33H38R40S / 44K68Y70S75D77T also called KSSHRS / KYSDT, an optimized variant cleaving the HBB5.3 target already described in example 5.

Examples of positives mutants cutting efficiently the HBB5 target identified with the two kinds of mutagenesis are shown in the Figures 19 and 20. The screening of the libraries of random mutagenesis lead to the identification of 66 and 45 new I-CreI clones cleaving the HBB5 target more efficiently than the initial mutants when forming heterodimers with the mutants targeting the HBB5.4 (Figure 19, panelA) or the HBB5.3 (Figure 19, panelB) sequences respectively. For the libraries of multiple site directed mutagenesis, 62 and 36 refined I-CreI mutants cleaving the HBB5 target with a maximal efficacy were also obtained from the pool of mutants cutting the sequence HBB5.3 (Figure 20, panelA) or the sequence HBB5.4 (Figure 20, panelB).

Sequencing of such positive clones allowed the identification of 98 and 45 novel optimized mutants leading to efficient cleavage of HBB5 target in yeast heterodimeric screen assay. The sequences of some I-*Cre*I optimized mutants cleaving the HBB5.3 and HBB5.4 targets are compiled in Table XI.

**Table X: Groups of eight and six optimized mutants cleaving the HBB5.3 and HBB5.4 targets used for construction of libraries by random mutagenesis and multiple site directed mutagenesis**

| **Mutant** **(SEQ ID NO:)** | **Sequence of mutants cleaving HBB5.3 target amino acids at positions 28, 30, 32, 33, 38, 40 / 44, 68, 70, 75 and 77and additional mutations at different positions ex: 28K30N32S33R38R40S / 44K68Y70S75N77Q + 19S + 82E KNSRRS/KYSNQ + 19S + 82E stands for K28, N30, S32, R33 , R38, S40/ K44, Y68, S70, N75, Q77 and S19 and E82** | **Mutant** **(SEQ ID NO:)** | **Sequence of mutants cleaving HBB5.4 target amino acids at positions 28, 30, 32, 33, 38, 40 / 44, 68, 70, 75 and 77 and additional mutations at different positions ex: 28K30N32T33Y38Q40S / 44A68R70S75E77R +19S + 80K KNTYQS / ARSER +19S + 80K stands for K28, N30, T32 , Y33 , Q38, S40/ A44, R68, S70, E75, R77 and S19 and K80** |
|---|---|---|---|
| **1 (164)** | 28K30S32S33H38R40S/ 44K68Y70S75D77T KSSHRS/KYSDT | **1 (186)** | 28K30N32T33Y38Q40S/ 44A68R70S75E77R **+19S + 80K + 96R** KNTYQS/ARSER **+19S + 80K + 96R** |
| **2 (166)** | 28K30N32S33R38R40S/ 44K68Y70S75D77R **+87L+103S+159E+160E** KNSRRS/KYSDR **+87L+103S+159E+160E** | **2 (185)** | 28K30N32T33Y38Q40S/ 44A68R70S75E77R **+19S + 80K** KNTYQS / ARSER **+19S + 80K** |
| **3 (170)** | 28K30T32S33H38R40S/ 44K68Y70S75D77T **+ 66H + 94L + 159R** KTSHRS / KYSDT **+ 66H+ 77T + 94L + 159R** | **3 (183)** | 28K30N32D33Y38Q40S/ 44A68R70S75E77R **+19S +80K +96R** KNDYQS/ARSER **+19S +80K +96R** |
| **4 (173)** | 28K30N32S33R38R40S/ 44K68Y70S75N77Q **+ 19S + 82E** KNSRRS / KYSNQ **+ 19S + 82E** | **4 (184)** | 28K30N32P33Y38Q40S/ 44A68R70S75E77R **+ 19S** KNPYQS / ARSER **+ 19S** |
| **5 (174)** | 28K30T32S33H38R40S/ 44K68Y70S75D77T **+ 19S** KTSHRS / KYSDT **+ 19S** | **5 (181)** | 28K30N32T33Y38Q40S/ 44A68R70S75E77R **+ 80K +87L** KNTYQS / ARSER **+ 80K +87L** |
| **6 (175)** | 28K30N32S33R38R40S/ 44K68Y70S75D77R **+ 87L** KNSRRS / KYSDR + **87L** | **6 (179)** | 28K30N32S33Y38Q40S/ 44A68R70S75E77R **+24V +43L + 80K +87L +96R** KNSYQS/ARSER **+24V +43L + 80K +87L +96R** |
| **7 (176)** | 28K30T32S33H38R40S/ 44K68Y70S75D77T **+ 105A** KTSHRS / KYSDT **+ 105A** | | |
| **8 (177)** | 28K30T32S33H38R40S/ 44K68Y70S75D77T **+ 132V** KTSHRS / KYSDT **+ 132V** | | |

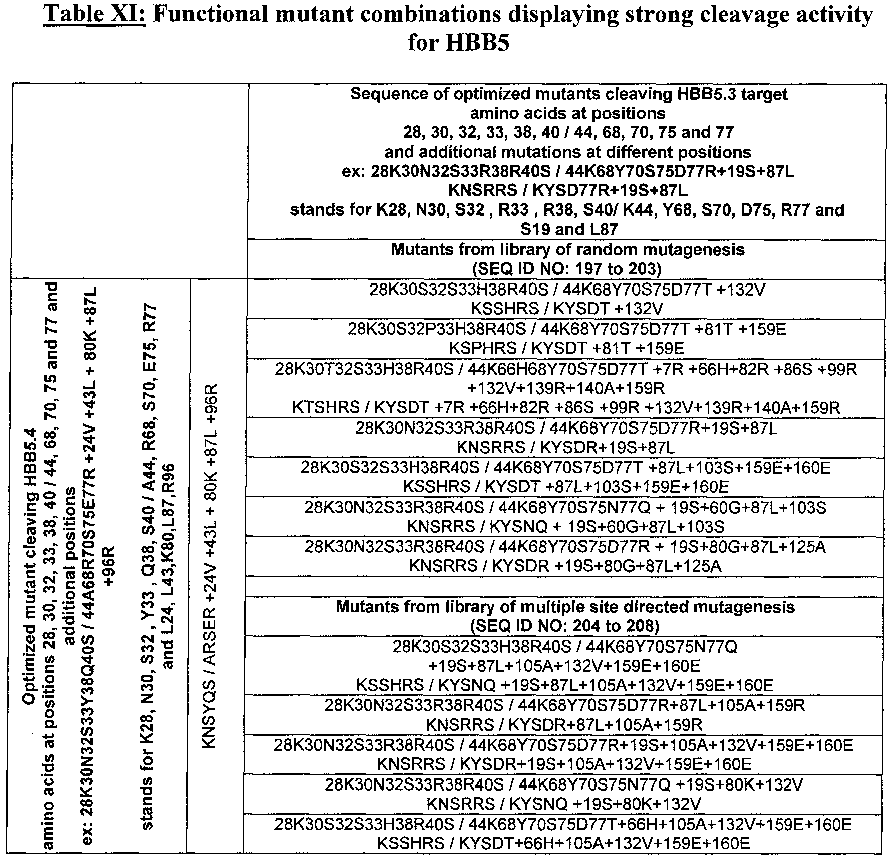

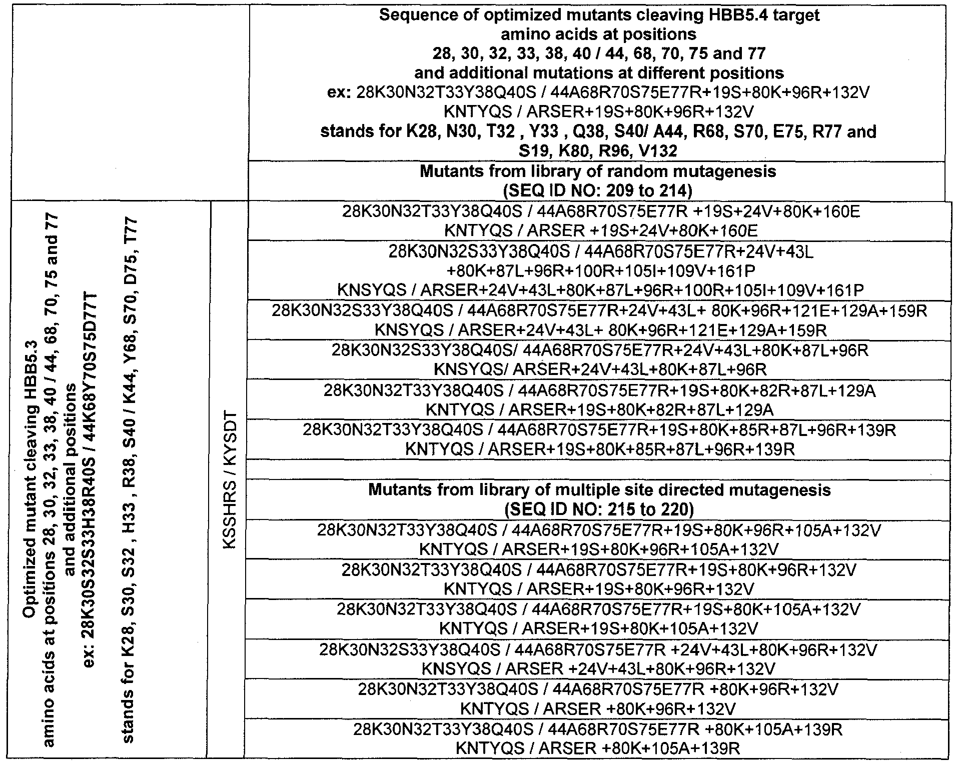

### Example 8: Validation of HBB 5 target cleavage in an extrachromosomic model in CHO cells

Several I-*Cre*I refined mutants able to efficiently cleave the HBB 5 target in yeast when forming heterodimers were identified in example 7. In order to validate and characterize the heterodimer displaying the maximal efficacy to cleave the HBB 5 sequence in CHO cells, the efficiency of combinations of mutants able to cut the HBB 5 target were tested using an extrachromosomal assay in mammalian cells.

### 1) Materials and methods

### a) Cloning of HBB 5 target in a vector for CHO screen

The target of interest was cloned as follows: oligonucleotide corresponding to the target sequence flanked by gateway cloning sequence was ordered from PROLIGO. Double-stranded target DNA, generated by PCR amplification of the single stranded oligonucleotide, was cloned using the Gateway protocol (INVITROGEN) into CHO reporter vector (pCLS1058, Figure 30). Cloned target was verified by sequencing (MILLEGEN).

### b) Re-cloning of meganucleases

The ORF of I-CreI optimized mutants cleaving the HBB5.3 and HBB5.4 targets identified in example 7 were re-cloned in pCLS1069 (Figure 31). ORFs were amplified by PCR on yeast DNA using the primers attB1-ICreIFor: (5'-ggggacaagtttgtacaaaaaagcaggcttcgaaggagatagaaccatggccaataccaaatataacaaagagttcc-3' (SEQ ID NO: 221); and attB2-ICreIRev (5'-ggggaccactttgtacaagaaagctgggtttagtcggccgccggggaggatttcttcttctcgc-3' (SEQ ID NO: 222). PCR products were cloned in CHO expression vector pCDNA6.2 from INVITROGEN (pCLS1069, Figure 31) using the Gateway protocol (INVITROGEN). Resulting clones were verified by sequencing (MILLEGEN).

### c) Extrachromosomal assay in mammalian cells

CHO cells were transfected with Polyfect® transfection reagent according to the supplier's protocol (QIAGEN). 72 hours after transfection, culture medium was removed and 150 µl of lysis/revelation buffer for β-galactosidase liquid assay was added (typically 1 liter of buffer contained: 100 ml of lysis buffer (Tris-HCl 10 mM pH7.5, NaCl 150 mM, Triton X100 0.1 %, BSA 0.1 mg/ml, protease inhibitors), 10 ml of Mg 100X buffer (MgCl₂ 100 mM, β-mercaptoethanol 35 %), 110 ml ONPG 8 mg/ml and 780 ml of sodium phosphate 0.1M pH7.5). After incubation at 37°C, OD was measured at 420 nm. The entire process was performed on an automated Velocity 11 BioCel platform. Per assay, 150 ng of target vector was cotransfected with 12.5 ng of each HBB5 mutant expression vector (12.5 ng of vector encoding the mutant cleaving the palindromic HBB5.3 target and 12.5 ng of vector encoding the mutant cleaving palindromic HBB5.4 target).

### 2) Results

Mutants described in Table XI were first re-cloned in the mammalian expression vector pCLS 1069 (Figure 31).

The Figure 21 shows the results obtained for twelve combinations of heterodimers and the values of the different combinations are compiled in the Table XII. Analysis of the efficiencies of cleavage of the HBB5 sequence demonstrates that mainly two combinations (MtA2_MtA6, MtA3_MtA6) of I-CreI mutants are able to cut efficiently the HBB5 target in CHO cells. Such observation is in agreement with the results of the functional heterodimeric yeast screen assays.

**Table XII: Functional heterodimeric combinations cutting efficiently HBB5 target in CHO cells.**

| **Optimized mutants cleaving HBB5.4 amino acids at positions 28, 30, 32, 33, 38, 40/44, 68, 70, 75 and 77 and additional mutations at different positions ex: 28K30N32T33Y38Q40S/ 44A68R70S75E77R +19S+24V+80K stands for K28, N30, T32 , Y33 , Q38, S40/ A44, R68, S70, E75, R77 and S19, V24, K80** | **Optimized mutants cleaving HBB5.3 amino acids at positions 28,30,32,33,38,40 / 44,68,70,75 and 77 and additional mutations at different positions ex: 28K30832P33H38R408 / 44K68Y70875D77T +81T stands for K28, S30, P32 , H33, R38, S40/ K44, Y68, S70, D75, T77 and T81** | | | |
|---|---|---|---|---|
| | **Mt A2 (SEQ ID NO:277)** | **Mt B2 (SEQ ID NO: 278)** | **Mt A3 (SEQ ID NO:279)** | **Mt E3 (SEQ ID NO:280)** |
| | 28K30S32P 33H38R40S/ 44K68Y70S 75D77T +81T KSPHRS/ KYSDT +81T | 28K30S32P 33H38R40S/ 44K68Y70S 75D77T +34R+81T KSSHRS / KYSDT+34R+8 1T | 28K30T32S 33H38R40S/ 44K68Y70S 75D77T +66H +82R +86S+99R +132V+139R +140A KTSHRS/ KYSDT +66H +82R +86S+99R +132V+139R +140A | 28K30T32S 33H38R40S / 44K68Y70S 75D77T +49A+66H +82R+86S+ 99R+132V +139R+140A KTSHRS / KYSDT +49A+66H +82R+86S +99R+132V +139R+140A |
| **MtB1 (SEQ ID NO: 281)** 28K30N32T33Y 38Q40S / 44A68R70S75E77R +19S+24V+80K KNTYQS/ARSER +19S+24V+80K | 0,42 | 0,33 | 0,20 | 0,37 |
| **MtA5 (SEQ ID NO: 213)** 28K30N32T33Y 38Q40S / 44A68R70S75E77R +19S +80K +82R+87L+129A KNTYQS / ARSER +19S +80K +82R+87L+129A | 0,78 | 0,50 | 0,86 | 0,46 |
| **Mt A6 (SEQ ID NO:214)** 28K30N32T33Y 38Q40S /44A68R70S75E77R +19S +80K+85R+87L +96R+139R KNTYQS/ARSER +19S +80K+85R+87L +96R+139R | 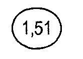 | 0,54 | 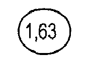 | 0,71 |

### Example 9: Making of meganucleases cleaving HBB8.3

This example shows that I-CreI mutants can cut the HBB8.3 DNA target sequence derived from the right part of the HBB8 target in a palindromic form (Figure 5). Target sequences described in this example are 22 bp palindromic sequences. Therefore, they will be described only by the first 11 nucleotides, followed by the suffix _P. For example, target HBB 8.3 will be noted also c**agacttct**gt_P (SEQ ID NO: 31). HBB8.3 is similar to 5TCT_P at positions ±1, ±2, ±3, ±4, ±5, ±8, ±10 and ±11 and to 10AGA_P at positions ±1, ±2, ±8, ±9, and ±10. Mutants able to cleave 5TCT_P were obtained by mutagenesis on I-*Cre*I N75 at positions 44, 68, 70, 75 and 77, as described in Arnould et al., J. Mol. Biol., 2006, 355, 443-458 and International PCT Applications WO 2006/097784, WO 2006/097853. Mutants able to cleave the 10AGA_P target were obtained by mutagenesis on I-*Cre*I N75 at positions 28, 30, 32, 33, 38, 40 and 70, as described in Smith et al, Nucleic Acids Res., 2006, 34, e149 and International PCT Applications WO 2007/060495 and WO 2007/049156. Thus, combining such pairs of mutants would allow for the cleavage of the HBB 8.3 target.

Both sets of proteins are mutated at position 70. However, two separable functional subdomains exist (Smith et al, Nucleic Acids Res., 2006, 34, e149 and International PCT Applications WO 2007/057781 and WO 2007/049095). That implies that this position has little impact on the specificity in base 10 to 8 of the target. Therefore, to check whether combined mutants could cleave the HBB8.3 target, mutations at positions 44, 68, 70, 75 and 77 from proteins cleaving 5TCT_P (caaaac**tct**gt_P) were combined with the 28, 30, 32, 33, 38 and 40 mutations from proteins cleaving 10AGA_P (c**aga**acgtcgt_P).

### 1) Material and Methods

The experimental procedure is as described in example 1.

### 2) Results

I-*Cre*I combinatorial mutants were constructed by associating mutations at positions 44, 68, 70, 75 and 77 with the 28, 30, 33, 38 and 40 mutations on the I-*Cre*I scaffold, resulting in a library of complexity 1600. This library was transformed into yeast and 2880 clones (1, 8 times the diversity) were screened for cleavage against the HBB8.3 DNA target (c**agacttct**gt_P; SEQ ID NO: 31). 264 positives clones were found, which after sequencing and validation by secondary screening turned out to be correspond to 126 different novel endonucleases (see Table XIII). The 24 novel endonucleases presented in Table XIII correspond to the sequences SEQ ID NO: 80 to 103. Examples of positives mutants cutting the HBB8.3 target are shown in Figure 10.

**Table XIII: Cleavage of the HBB8.3 target by the combinatorial variants**

| **Amino acids at positions 28, 30, 32, 33, 38 and 40** **(ex : KDSRQS stands for K28, D30, S32, R33, Q38 and S40)** | **Amino acids at positions 44, 68, 70, 75, and 77** **(ex: KASNI stands for K44, A68, S70, N75 and 177)** | | | | | | | |
|---|---|---|---|---|---|---|---|---|
| | **KASNI** | **KYSNI** | **KYSNL** | **KYSNQ** | **KYSNV** | **KYSNY** | **QASNR** | **QNSNR** |
| **KDSRQS** | | | | + (SEQ ID NO: 80) | | + (SEQ ID NO: 81) | | |
| **KNSCRS** | | | | + (SEQ ID NO: 82) | | | | |
| **KNSGKS** | | + (SEQ ID NO: 83) | | | | + (SEQ ID NO: 84) | + (SEQ ID NO: 85) | + (SEQ ID NO: 86) |
| **KNSGRS** | | | | | + (SEQ ID NO: 87) | + (SEQ ID NO: 88) | | |
| **KNSKRS** | | | | | | + (SEQ ID NO: 89) | | |
| **KNSRNS** | | + (SEQ ID NO: 90) | | | | + (SEQ ID NO: 91) | | |
| **KNSRTS** | | | | | | | + (SEQ ID NO: 92) | |
| **KNSTRS** | | | | + (SEQ ID NO: 93) | | + (SEQ ID NO: 94) | | |
| **KQTYQS** | | | | | | + (SEQ ID NO: 95) | | |
| **KQTYRS** | + (SEQ 10 NO 96) | | | | | | | |
| **KRDYQS** | | | | | | + (SEQ ID NO: 97) | | |
| **KRDYSS** | | | | | | | + (SEQ ID NO: 98) | |
| **KSSGQS** | | | + (SEQ ID NO: 99) | | | + (SEQ ID NO: 100) | | |
| **KTSGQS** | | | | | | | | + (SEQ ID NO: 101) |
| **KTSHRS** | | | | + (SEQ ID NO: 102) | | + (SEQ ID NO: 103) | | |

| | | | | | | | | |
|---|---|---|---|---|---|---|---|---|
| * Only 120 out of the 1600 combinations are displayed + indicates that the combinatorial mutant was cleaving the HBB8.3 target. | | | | | | | | |

### Example 10: Making of meganucleases cleaving HBB8.4

This example shows that I-CreI variants can cleave the HBB8.4 DNA target sequence derived from the leftpart of the HBB8 target in a palindromic form (Figure 5). All target sequences described in this example are 22 bp palindromic sequences. Therefore, they will be described only by the first 11 nucleotides, followed by the suffix _P. For example, HBB 8.4 will be called c**tgactcct**gt_P; SEQ ID NO: 32). HBB 8.4 is similar to 5CCT_P at positions ±1, ±2, ±3, ±4, ±5, ±8 and ±11 and to 10TGA_P at positions ±1, ±2, ±8, ±9, ±10 and ±11. It was hypothesized that positions ±6 and ±11 would have little effect on the binding and cleavage activity. Mutants able to cleave 5CCT_P (caaaac**cct**gt_P; SEQ ID NO: 22) were obtained by mutagenesis on I-*Cre*I N75 at positions 44, 68, 70, 75 and 77, as described in Arnould et al., J. Mol. Biol., 2006, 355, 443-458 and International PCT Applications WO 2006/097784, WO 2006/097853. Mutants able to cleave the 10TGA_P target (c**tga**acgtcgt_P; SEQ ID NO: 28) were obtained by mutagenesis on I-*Cre*I N75 at positions 28, 33, 38, 40 and 70, as described in Smith et al, Nucleic Acids Res., 2006, 34, e149 and International PCT Applications WO 2007/060495 and WO 2007/049156. Thus combining such pairs of mutants would allow for the cleavage of the HBB 8.4 target.

Both sets of proteins are mutated at position 70. However, two separable functional subdomains exist (Smith et al, Nucleic Acids Res., 2006, 34, e149 and International PCT Applications WO 2007/049095 and WO 2007/057781). That implies that this position has little impact on the specificity in base 10 to 8 of the target. Therefore, to check whether combined mutants could cleave the HBB 8.4 target, mutations at positions 44, 68, 70, 75 and 77 from proteins cleaving 5CCT_P (caaaaccctgt_P) were combined with the 28, 33, 38 and 40 mutations from proteins cleaving 10TGA_P (c**tga**acgtcgt_P).

### 1) Material and Methods

The experimental procedure is as described in example 2.

### 2) Results

I-*Cre*I combined mutants were constructed by associating mutations at positions 44, 68, 70, 75 and 77 with the 28, 30, 32, 33, 38 and 40 mutations on the I-*Cre*I scaffold (Table XIV), resulting in a library of complexity 1720. This library was transformed into yeast and 3168 clones (1, 8 times the diversity) were screened for cleavage against the HBB8.4. 141 positives clones were found, which after sequencing and validation by secondary screening turned out to be correspond to 93 different novel endonucleases (see Table XIV). The 19 novel endonucleases presented in Table XIV correspond to the sequences SEQ ID NO: 104 to 122. Examples of positives mutants cutting the HBB8.4 target are shown in Figure 11.

**Table XIV: Cleavage of the HBB8.4 target by the combinatorial variants**

| **Amino acids at positions 28, 30, 32, 33,38 and 40** **(ex :KNETQS stands for K28, N30, E32, T33, Q38 and S40)** | **Amino acids at positions 44, 68, 70, 75, and 77** **(ex: DASKR stands for D44, A68, S70, K75 and R77)** | | | | | | | | | |
|---|---|---|---|---|---|---|---|---|---|---|
| | **DASKR** | **KASDR** | **KESNR** | **KSSNV** | **KTSDR** | **KYSDT** | **KYSYN** | **NESNR** | **RRSND** | **RYSNQ** |
| **KNETQS** | + (SEQ ID NO 104) | | | | | | | | | |
| **KNSCHS** | | | | | | | | | | + SEQ ID NO 105) |
| **KNSCTS** | | | | + SEQ ID NO 106) | | + SEQ ID NO 107) | | | | + SEQ ID NO 108) |
| **KNSGRS** | | | | | + SEQ ID NO 109) | | | | | |
| **KNSGTS** | | | | | + SEQ ID NO 110) | | | | | |
| **KNSSSS** | | + SEQ ID NO 111) | | | | | | | | |
| **KNSTQS** | + SEQ ID NO 112) | | | | | | | | | |
| **KNSTRQ** | | | | | | | | + SEQ ID NO 113) | | |
| **KNSTSS** | | | | | | | | | | + SEQ ID NO: 114) |
| **KNSVHS** | | | | + SEQ ID NO 115) | | | + SEQ ID NO: 116) | | | + SEQ ID NO 117) |
| **KNTCQS** | + SEQ ID NO. 118) | | + SEQ ID NO 119) | | | | | | + SEQ ID NO 120) | + SEQ ID NO: 121) |
| **KNTTQS** | | | | | + SEQ ID NO. 122) | | | | | |

| | | | | | | | | | | |
|---|---|---|---|---|---|---|---|---|---|---|
| * Only 120 out of the 1720 combinations are displayed + indicates that the combinatorial mutant was cleaving he HBB8.4 target | | | | | | | | | | |

### Example 11: Making of meganucleases cleaving HBB8

We have previously identified I-*Cre*I mutants able to cleave each of the palindromic HBB 8 derived targets (HBB8.3 and HBB8.4, see examples 9 and 10). Pairs of such mutants in yeast (one cutting HBB 8.3 and one cutting HBB 8.4) were co-expressed in yeast. Upon coexpression, there should be three active molecular species, two homodimers, and one heterodimer. It was tested whether the heterodimers cut the HBB8.2 and HBB8 targets.

### 1) Material and Methods

The experimental procedure is as described in example 3.

### 2) Results

Co-expression of mutants cleaving the HBB 8.3 (11 mutants) and HBB 8.4 (15 mutants) sequences resulted in efficient cleavage of the HBB 8.2 target in almost all cases (Figure 12, panel A). In addition, some of these combinations were able to cut the HBB 8 natural target (Figure 12, panel B) that differs from the HBB 8.2 sequence by 3 bp at positions -1, -2 and 2 (Figure 5). Functional combinations are summarized in Table XV and Table XVI.

**Table XV: Combinations that resulted in cleavage of HBB8.2 target**

| **Mutant +HBB 8.4, amino acids at positions 28, 30, 32, 33, 38, 40 /44 68 70 7577 (ex:KNSTSS/ RYSNQ stands for K28, N30, S32 , T33 , S38, S401 R44, Y68, S70, N75 and Q77)** | **Mutant + HBB 8.3,** **amino acids at positions 28, 30, 32, 33, 38, 40 / 44, 68, 70, 75 and 77** **(ex: KNSGKS/QASNR stands for K28, N30, S32, G33, K38, S40/ Q44, A68, S70, N75 and R77)** | | | | | | | | | | |
|---|---|---|---|---|---|---|---|---|---|---|---|
| | KNSGKS/ GASNR | KTSHRS / KYSNY | KTSHRS/ KYSNQ | KNSTRS/ KYSNY | KNSSRS/ KYSNY | KNSGKS/ KYSNI | KNSGKS/ QNSNR | KQTYRS/ KASNI | KNSTRS/ KYSNQ | KNSCRS/ KYSNQ | KNSGKS/ KYSNY |
| KNSTSS/ RYSNQ | + | + | + | + | + | + | + | + | + | + | + |
| KNTTQS/ KTSDR | + | + | + | + | + | + | + | + | + | + | + |
| KNETQS/ DASKR | + | + | + | + | + | + | + | + | + | + | + |
| KNSCHS/ RYSNQ | + | + | + | + | + | + | + | + | + | + | + |
| KNSTRQ/ NESNR | + | + | + | + | + | + | + | + | + | + | + |
| KNTTQS/ KTSDR | + | + | + | + | + | + | + | + | + | + | + |
| KNSCTS/ RYSNQ | + | + | + | + | + | + | + | + | + | + | + |
| KNSSSS/ KASDR | + | + | + | + | + | + | + | + | + | + | + |
| KNSVHS/ RYSNQ | + | + | + | + | + | + | + | + | + | + | + |
| KNSGRS/ KTSDR | + | + | + | + | + | + | + | + | + | + | + |
| KNSCTS/ KYSDT | + | + | + | + | + | + | + | + | + | + | + |
| KNSVHS/ KYSYN | + | + | + | + | + | + | + | + | + | + | + |
| ND | + | + | + | + | + | + | + | + | + | + | + |
| KNTCQS/ DASKR | + | + | + | + | + | + | + | + | + | + | + |
| KNTCQS/ RYSNQ | + | + | + | + | + | + | + | + | + | + | + |

| | | | | | | | | | | | |
|---|---|---|---|---|---|---|---|---|---|---|---|
| + indicates that the heterodimeric mutant was cleaving the HBB8.2 target. ND corresponds to an unidentified sequence. | | | | | | | | | | | |

**Table XVI: Combinations that resulted in cleavage of HBB8 target**

| **Mutant HBB8.4, amino acids at positions 28, 30, 32, 33, 38, 40 / 44 68 70 75 77 (ex: KNSTSS/ RYSNQ stands for K28, N30, S32 , T33 , S38, S40/ R44, Y68, S70, N75 and Q77)** | **Mutant HBB8.3,** **amino acids at positions 28, 30, 32, 33, 38, 40/44, 68, 70, 75 and 77** **(ex: KNSGKS/QASNR stands for K28, N30, S32, G33, K38, S40/ Q44, A68, S70, N75 and R77)** | | | | | | | | | | |
|---|---|---|---|---|---|---|---|---|---|---|---|
| | KNSGKS/ QASNR (SEQ ID NO 85) | KTSHRS/ KYSNY (SEO ID NO 103) | KTSHRS/ KYSNQ | KNSTRS/ KYSNY (SEQ ID NO 94) | KNSGRS/ KYSNY (SEQ ID NO 88) | KNSGKS/ KYSNI | KNSGKS/ QNSNR | KQTYRS/ KASNI | KNSTRS/ KYSNQ | KNSCRS/ KYSNQ (SEQ ID NO 82) | KNSGKS/ KYSNY (SEQ ID NO 84) |
| **KNSTSS/ RYSNQ** (SEQ ID NO 114) | | | | | | | | | | + | |
| **KNTTQSI KTSDR** | | | | | | | | | | | |
| **KNETQS/ DASKR** (SEQ ID NO 104) | | | | | | | | | | + | |
| **KNSCHS/ RYSNQ** (SEQ ID NO 105) | + | + | | + | + | | | | | | + |
| **KNSTRQ/ NESNR** (SEQ ID NO 113) | | + | | | | | | | | | |
| **KNTTQS/ KTSDR** | | | | | | | | | | | |
| **KNSCTSI RYSNQ** | | | | | | | | | | | |
| **KNSSSS/ KASDR** (SEQ ID NO 111) | | + | | | | | | | | | |
| **KNSVHS/ RYSNQ** | | | | | | | | | | | |
| **KNSGRS/ KTSDR** | | | | | | | | | | | |
| **KNSCTS/ KYSDT** | | | | | | | | | | | |
| **KNSVHS/ KYSYN** | | | | | | | | | | | |
| **ND** | + | + | | + | + | | | | | | |
| **KNTCQS/ DASKR** (SEQ ID NO 118) | + | + | | + | + | | | | | | + |
| **KNTCQS/ RYSNQ** (SEQ ID NO 121) | + | | | | | | | | | | |

| | | | | | | | | | | | |
|---|---|---|---|---|---|---|---|---|---|---|---|
| + indicates that the heterodimeric mutant was cleaving the HBB8 target. ND corresponds to an unidentified sequence. | | | | | | | | | | | |

As is evident from the above, the invention is not at all limited to those of its embodiments, implementations and applications which have just been described more explicitly; it encompasses, on the contrary, all the variants which may occur to the specialist in this field, without departing from the framework or the scope of the present invention.

### Example 12: Making of meganucleases cleaving HBB8 with higher efficacy by site-directed mutagenesis of protein cleaving HBB8.3 and assembly with proteins cleaving HBB8.4

I-*Cre*I mutants able to cleave the non palindromic HBB8 target were identified by co-expression of mutants cleaving the palindromic HBB8.3 and mutants cleaving the palindromic HBB8.4 target (Example 11). To increase the number and efficacy of I-*Cre*I mutants able to cleave the non palindromic HBB8 target, mutants cleaving the palindromic HBB8.3 target were site directed mutagenized and new variants cleaving HBB8 with high efficacy, when co-expressed with mutants cleaving the HBB 8.4 target, were screened.

Six amino-acid substitutions which enhance the activity of I-*Cre*I derivatives were individually introduced into the coding sequence of proteins cleaving HBB8.3. These mutations correspond to the replacement of Glycine 19 with Serine (G19S), Phenylalanine 54 with Leucine (F54L), Glutamic Acid 80 with Lysine (E80K), Phenylalanine 87 with Leucine (F87L), Valine 105 with Alanine (V105A) and Isoleucine 132 with Valine (I132V). The resulting mutagenized proteins were then tested for their ability to induce efficient cleavage of the HBB8 target, upon co-expression with a mutant cleaving HBB8.4.

### 1) Material and Methods

The experimental procedure is as described in example 5.

### 2) Results

Libraries containing the six amino-acids substitutions (Glycine 19 with Serine, Phenylalanine 54 with Leucine, Glutamic Acid 80 with Lysine, Phenylalanine 87 with Leucine, Valine 105 with Alanine and Isoleucine 132 with Valine) were constructed on a pool of seventeen initial I-*Cre*I mutants cleaving HBB8.3 target (Table XVII). 372 transformed clones for each library were then mated with a yeast strain that (i) contains the HBB8 target in a reporter plasmid (ii) expresses the mutant 28K30N32T33C38Q40S44D68A70S75K77R also called KNTCQS /DASKR, a variant cleaving the HBB8.4 target described in example 11.

After mating with this yeast strain, 186 new I-*Cre*I clones were found to cleave the HBB8 target more efficiently than the initial mutants when forming heterodimers with a mutant cleaving the HBB8.4 target. These 186 mutants (72 clones with G19S mutation, 60 clones with F54L mutation, 21 clones with E80K mutation, 5 clones with F87L mutation, 9 clones with V105A mutation and 19 clones with I132V mutation) were then rearranged (wells A1 to H9 of the rearranged plate) and submitted to a validation screen conducted exactly in the same conditions as the first one (Figure 22). Sequencing of the 186 positive clones allowed the identification of 114 distinct novel mutants giving higher levels of cleavage of HBB8 target.

The sequence of 25 I-*Cre*I optimized mutants cleaving the HBB8.3 target when forming heterodimer with the mutant KNTCQS / DASKR cleaving HBB8.4 are listed in Table XVIII (SEQ ID NO: 231 to 255). Some of these I-*Cre*I mutants are expected mutants due to the site-directed mutagenesis, but also contain unexpected mutations probably due to the PCR reaction and micro-recombination between mutants of the pool used for the libraries construction.

In order to test the specificity of cleavage of the HBB8 target, the 93 optimized mutants with the simple mutations F54L, F87L, V105A and I132V cleaving the HBB8.3 target were mated with a yeast strain that contains the HBB8.5 or the HBB6.5 target (Figure 5) in a reporter plasmid. For almost all the I-*Cre*I optimized mutants tested the palindromic target HBB8.5 with the mutation responsible of the Sickle Cell Anemia (Figure 23, panel A) is more efficiently cleaved than the HBB6.5 palindromic target derived from the wild type sequence (Figure 23, panel B).

**Table XVII: Group of seventeen mutants cleaving the HBB8.3 target used for site directed mutagenesis**

| | **Sequence of mutants cleaving HBB8.3 target amino acids at positions 28, 30, 32, 33, 38, 40 / 44, 68, 70, 75 and 77 and additional mutations at different positions ex: KNSGKS / QNSNR / 87L stands for K28, N30, S32, G33 , K38, S40/ Q44, N68, S70, N75, R77 and L87** | **SEQ ID NO:** |
|---|---|---|
| **1** | **28K30N32S33C38R40S44K68Y70S75N77Q** **KNSCRS/KYSNQ** | **82** |
| **2** | **28K30N32S33C38R40S44Q68A70S75N77R** **KNSCRS/QASNR** | **223** |
| **3** | **28K30N32S33G38K40S44K68Y70S75N77I** **KNSGKS/KYSNI** | **83** |
| **4** | **28K30N32S33G38K40S44K68Y70S75N77Y** **KNSGKS/KYSNY** | **84** |
| **5** | **28K30N32S33G38K40S44Q68A70S75N77R** **KNSGKS/QASNR** | **85** |
| **6** | **28K30N32S33G38K40S44Q68N70S75N77R + 87L** **KNSGKS/QNSNR + 87L** | **224** |
| **7** | **28K30N32S33G38R40S44K68Y70S75N77Y** **KNSGRS/KYSNY** | **88** |
| **8** | **28K30N32S33S38K40S44K68Y70S75N77I+ 12H** **KNSSKS/KYSNI + 12H** | **225** |
| **9** | **28K30N32S33T38R40S44K68Y70S75N77Q + 4E** **KNSTRS/KYSNQ + 4E** | **226** |
| **10** | **28K30N32S33T38R40S44K68Y70S75N77Y** **KNSTRS/KYSNY** | **94** |
| **11** | **28K30N32S33T38R40S44Q68A70S75N77R** **KNSTRS/QASNR** | **227** |
| **12** | **28K30N32S33T38R40S44Q68N70S75N77R** **KNSTRS/QNSNR** | **228** |
| **13** | **28K30Q32T33Y38R40S44K68A70S75N77I** **KQTYRS/KASNI** | **96** |
| **14** | **28K30S32S33G38Q40S44K68Y70S75N77Y + 129A** **KSSGQS / KYSNY + 129A** | **229** |
| **15** | **28K30T32S33H38R40S44K68Y70S75N77Q** **KTSHRS/KYSNQ** | **102** |
| **16** | **28K30T32S33H38R40S44K68Y70S75N77Y** **KTSHRS/KYSNY** | **103** |
| **17** | **28K30T32S33H38R40S44Q68N70S75N77R** **KTSHRS/QNSNR** | **230** |

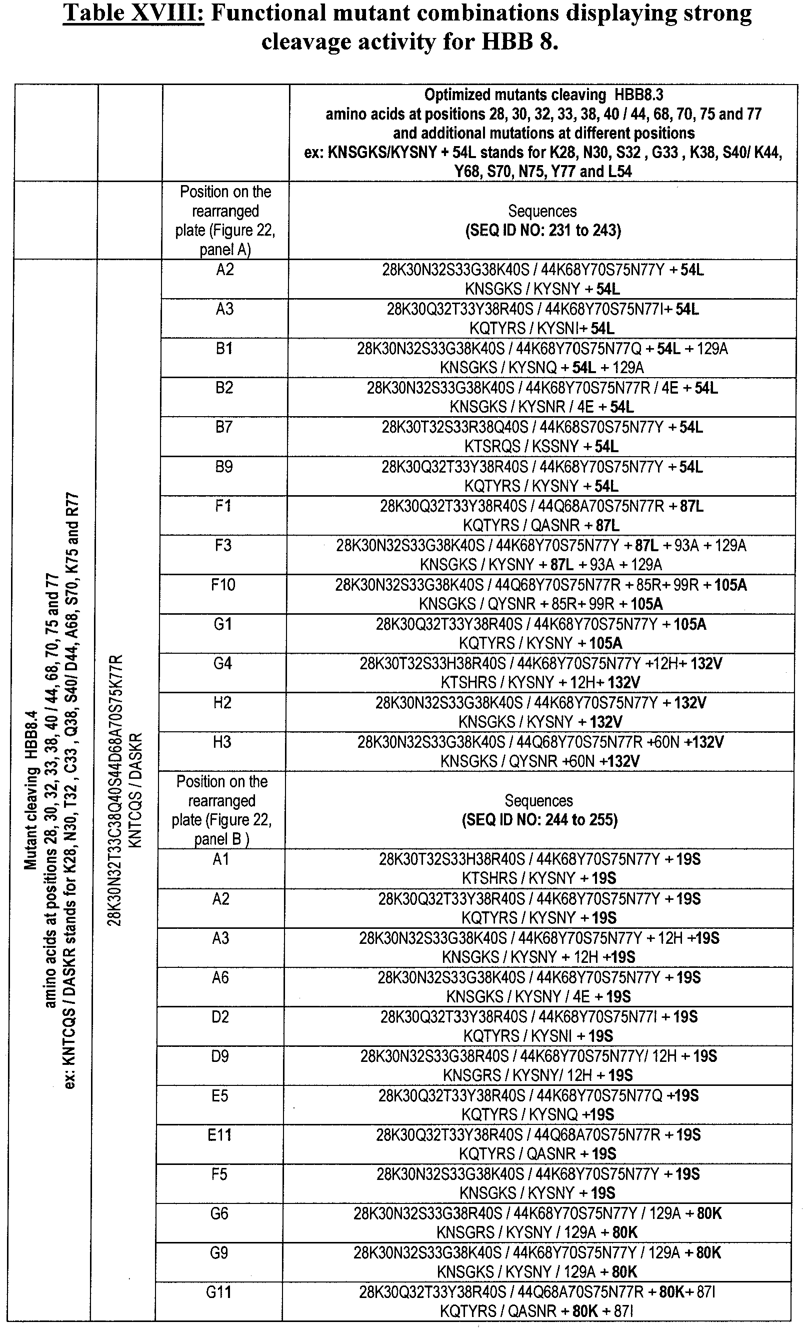

### Example 13: Making of meganucleases cleaving HBB8 with higher efficacy by site-directed mutagenesis of protein cleaving HBB8.4 and assembly with proteins cleaving HBB8.3

I-*Cre*I mutants able to cleave the non palindromic HBB8 target were identified by co-expression of mutants cleaving the palindromic HBB8.3 and mutants cleaving the palindromic HBB8.4 target (Example 11). To increase the number and efficacy of I-*Cre*I mutants able to cleave the non palindromic HBB8 target, mutants cleaving the palindromic HBB8.4 target were also site directed mutagenized and new variants cleaving HBB8 with high efficacy, when co-expressed with mutants cleaving the HBB 8.3 target, were screened.

The six mutations (G19S, F54L, E80K, F87L, V105A and I132V) described in example 12 were individually introduced into the coding sequence of proteins cleaving HBB8.4, and the resulting proteins were tested for their ability to induce efficient cleavage of the HBB8 target, upon co-expression with a mutant cleaving HBB8.3.

### 1) Material and Methods

Site-directed mutagenesis libraries were created by PCR on a pool of initial mutants cleaving HBB8.4 as described in example 5. The PCR fragments were purified. Approximately 25 ng of each of the two overlapping PCR fragments and 75 ng of vector DNA (pCLS1107, Figure 15) linearized by digestion with *DraIII* and *NgoMIV* are used to transform the yeast *Saccharomyces cerevisiae* strain FYC2-6A (MATα, trp1Δ63, leu2Δ1, his3Δ200) using a high efficiency LiAc transformation protocol. Intact coding sequences containing the single mutations are generated by *in vivo* homologous recombination in yeast.

### 2) Results

Libraries containing the six amino-acids substitutions were constructed on a pool of fourteen initial I-*Cre*I mutants cleaving HBB8.4 target (Table XIX). 372 transformed clones for each library were then mated with a yeast strain that (i) contains the HBB8 target in a reporter plasmid (ii) expresses the mutant 28K30T32S33H38R40S44K68Y70S75N77Y also called KTSHRS / KYSNY, a variant cleaving the HBB8.3 target described in example 11.

After mating with this yeast strain, 166 new I-CreI clones were found to cleave the HBB8 target more efficiently than the initial mutants when forming heterodimers with a mutant cleaving the HBB8.3 target. These 166 mutants (93 clones with G19S mutation, 6 clones with F54L mutation, 12 clones with E80K mutation, 17 clones with F87L mutation, 23 clones with V105A mutation and 15 clones with 1132V mutation) were then rearranged and submitted to a validation screen conducted exactly in the same conditions as the first one (Figure 24). Sequencing of the 166 positive clones allowed the identification of 78 distinct novel mutants giving higher levels of cleavage of HBB8 target.

Some examples of 20 I-*Cre*I optimized mutants cleaving the HBB8.4 target when forming heterodimer with the mutant KTSHRS / KYSNY cleaving HBB8.3 are listed in Table XX. Some of these I-*Cre*I mutants are expected mutants due to the site-directed mutagenesis, but also contain unexpected mutations probably due to the PCR reaction and micro-recombination between mutants of the pool used for the libraries construction.

In order to test the specificity of cleavage of the HBB8 target, the 93 optimized mutants with the simple mutations F54L, E80K, F87L, V105A and I132V cleaving the HBB8.4 target were mated with a yeast strain that contains the HBB8.6 or the HBB6.6 target (Figure 5) in a reporter plasmid. For almost all the I-*Cre*I optimized mutants tested the palindromic target HBB8.6 with the mutation responsible of the Sickle Cell Anemia is more efficiently cleaved than the HBB6.6 palindromic target derived from the wild type sequence (Figure 25, panel A and B).

**Table XIX: group of fourteen mutants cleaving the HBB8.4 target used for site directed mutagenesis ND corresponds to a sequence unidentified**

| | **Sequence of mutants cleaving HBB8.4 target amino acids at positions 28, 30, 32, 33, 38, 40 / 44, 68, 70, 75 and 77 and additional mutations at different positions ex: KNSCHS / RYSNQ + 121R stands for K28, N30, S32, C33, H38, S40/ R44, Y68, S70, N75, Q77 and R121** | **SEQ ID NO:** |
|---|---|---|
| **1** | **28K30N32S33T38S40S44R68Y70S75N77Q** **KNSTSS/RYSNQ** | **114** |
| **2** | **28K30N32E33T38Q40S44D68A70S75K77R** **KNETQS/DASKR** | **104** |
| **3** | **28K30N32S33T38R40Q44N68E70S75N77R** **KNSTRQ/NESNR** | **113** |
| **4** | **28K30N32S33C38T40S44R68Y70S75N77Q** **KNSCTS/RYSNQ** | **108** |
| **5** | **28K30N32S33V38H40S44R68Y70S75N77Q** **KNSVHS/RYSNQ** | **117** |
| **6** | **28K30N32S33C38T40S44K68Y70S75D77T** **KNSCTS/KYSDT** | **107** |
| **7** | **ND*** | |
| **8** | **28K30N32T33C38Q40S44R68Y70S75N77Q** **KNTCQS/RYSNQ** | **121** |
| **9** | **28K30N32T33T38Q40S44K68T70S75D77R** **KNTTQS/KTSDR** | **122** |
| **10** | **28K30N32S33C38H40S44R68Y70S75N77Q/121R** **KNSCHS/RYSNQ + 121R** | **256** |
| **11** | **28K30N32S33S38S40S44K68A70S75D77R** **KNSSSS/KASDR** | **111** |
| **12** | **28K30N32S33G38R40S44K68T70S75D77R** **KNSGRS /KTSDR** | **109** |
| **13** | **28K30N32S33V38H40S44K68Y70S75Y77N** **KNSVHS/KYSYN** | **116** |
| **14** | **28K30N32T33C38Q40S44D68A70S75K77R** **KNTCQS/DASKR** | **118** |

| | | |
|---|---|---|
| * unidentified sequence | | |

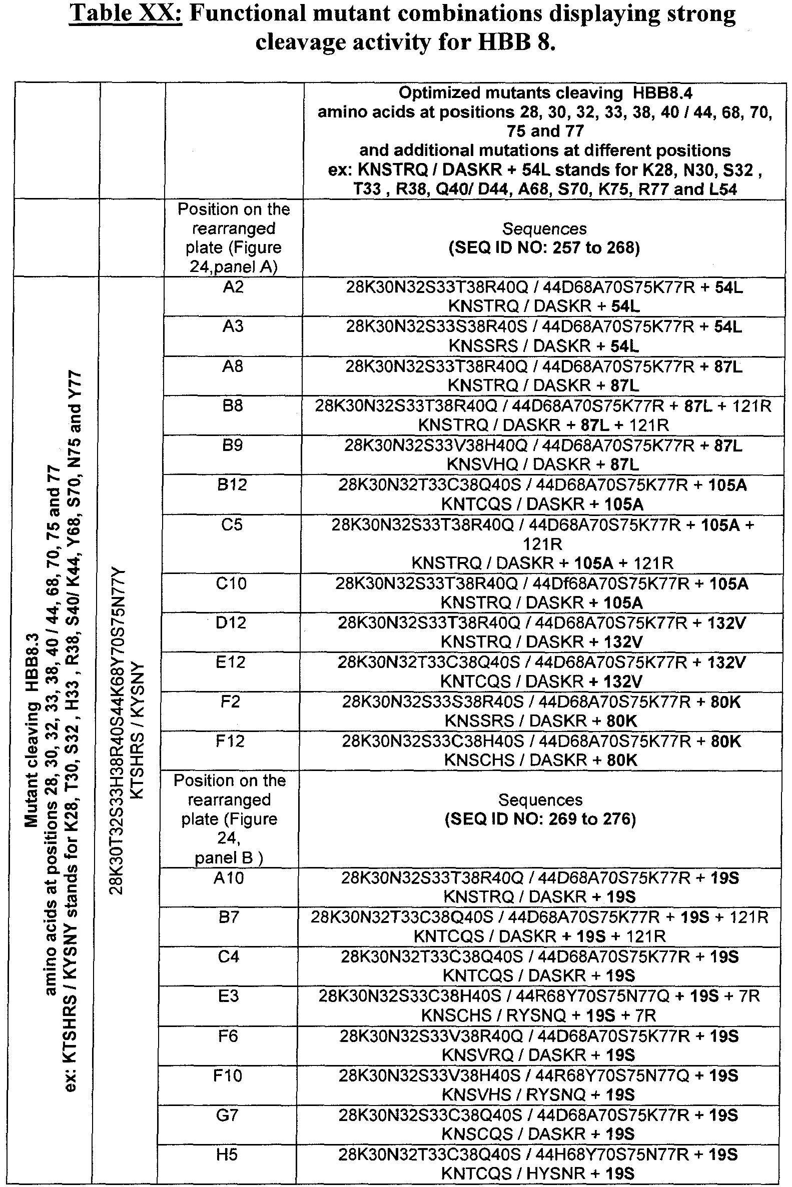

### Example 14: Functional efficiency of meganucleases cleaving HBB8 in mammalian cells

In examples 12 and 13, we have identified I-*Cre*I refined mutants able to efficiently cleave the HBB8 target in yeast. In this example, we tested the ability of combinations of the mutants cleaving the HBB8.3 and HBB8.4 sequences to cut the HBB8 target using an extrachromosomal and a chromosomal reporter assay in CHO mammalian cells.

### 1) Materials and Methods

Protocol of cloning of HBB8 derived targets in a vector for CHO screen, re-cloning of meganucleases and extrachromosomal assay in mammalian cells is as described in example 8.

### a) Chromosomal assay in CHO cells

CHO cell lines harbouring the reporter system (Figure 27) were seeded at a density of 2x10⁵ cells per 10cm dish in complete medium (Kaighn's modified F-12 medium (F12-K), supplemented with 2 mM L-glutamine, penicillin (100 UI/ml), streptomycin (100 µg/ml), amphotericin B (Fongizone) (0.25 µg/ml) (INVITROGEN-LIFE SCIENCE) and 10 % FBS (SIGMA-ALDRICH CHIMIE). The next day, cells were transfected with Polyfect transfection reagent (QIAGEN). Briefly, 2 µg of lacz repair matrix vector was co-transfected with various amounts of meganucleases expression vectors. After 72 hours of incubation at 37 °C, cells were fixed in 0.5 % glutaraldehyde at 4 °C for 10 min, washed twice in 100 mM phosphate buffer with 0.02 % NP40 and stained with the following staining buffer (10 mM Phosphate buffer, 1 mM MgCl₂, 33 mM K hexacyanoferrate (III), 33 mM K hexacyanoferrate (II), 0.1 % (v/v) X-Gal). After, an overnight incubation at 37 °C, plates were examined under a light microscope and the number of LacZ positive cell clones counted. The frequency of LacZ repair was expressed as the number of LacZ+ foci divided by the number of transfected cells (5x10⁵) and corrected by the transfection efficiency.

### 2) Results

Comparison of the cleavage activity of heterodimers of I-*Cre*I improved mutants against the two targets HBB8 and HBB6 was first monitored using the extrachromosomal assay in mammalian CHO cells. As it can be seen in Table XXI and Figure 26 nineteen heterodimeric combinations triggering efficient cleavage of the HBB derived targets were identified. Analysis of the efficiencies of cleavage of the HBB8 and HBB6 demonstrates that for most of the active heterodimeric combinations identified the target HBB8 with the mutation responsible of the Sickle Cell Anemia is more efficiently cleaved than the target HBB6 corresponding to the non mutated sequence. Such observation is in agreement with the results of the functional heterodimeric yeast screen assays.

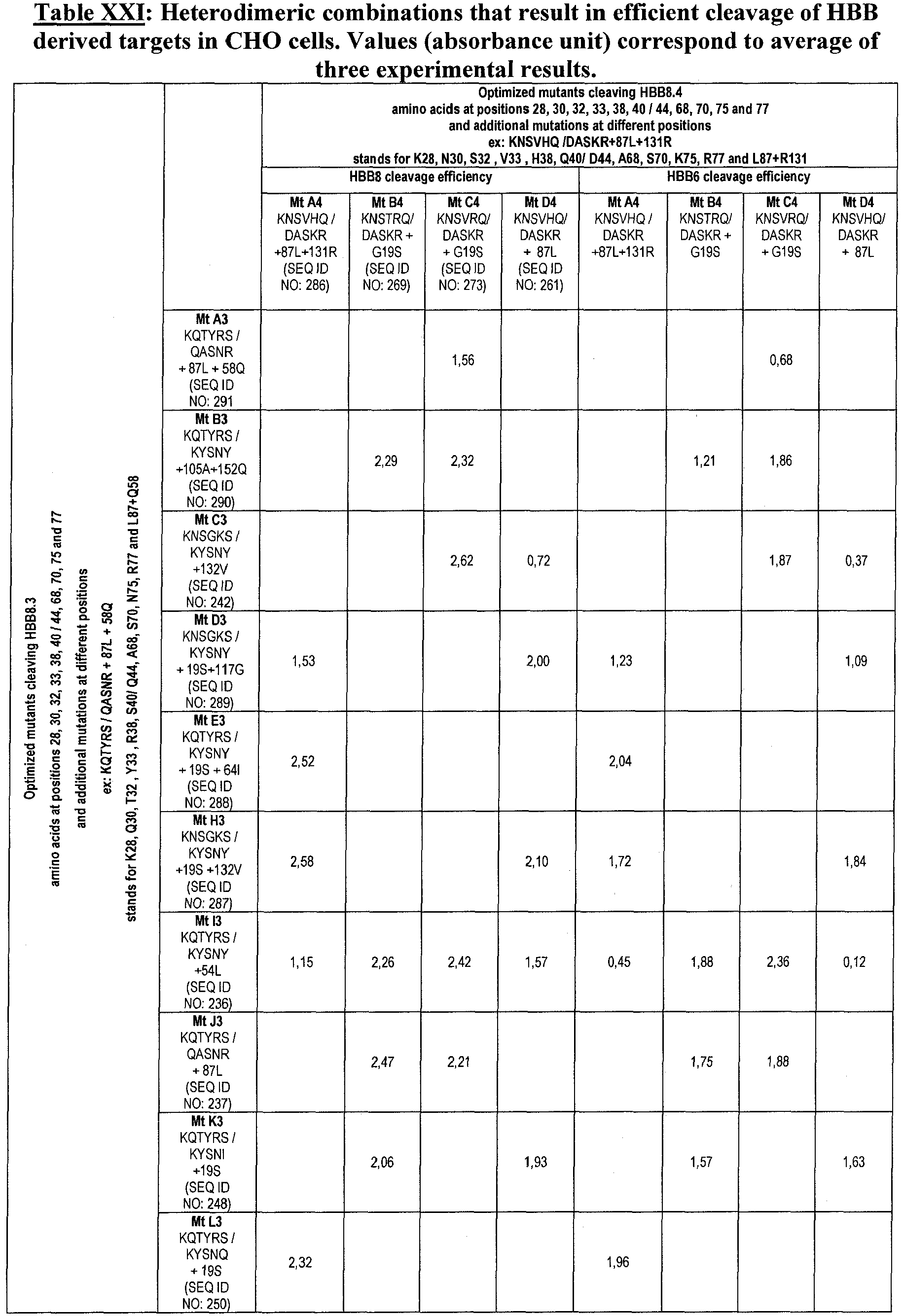

The activity of one of the most efficient HBB8 heterodimer containing the two I-*Cre*I optimized mutants HBB8.3_H3 and HBB8.4_A4 was also tested using a chromosomal assay in a CHO cell line containing either the HBB8 or the HBB6 targets. This chromosomal assay has been extensively described in a recent publication (Arnould et al., J Mol Biol, 2007, 371, 49-65). Briefly, a CHO cell line carrying a single copy transgene was first created. The transgene contains a human EF1α, promoter upstream an I-*Sce*I cleavage site (Figure 27, step 1). Second, the I-*SceI* meganuclease was used to trigger DSB-induced homologous recombination at this locus, and insert a 5.5 kb cassette with the novel meganuclease cleavage site (Figure 27, step2). This cassette contains a non functional LacZ open reading frame driven by a CMV promoter, and a promoter-less hygromycin marker gene. The LacZ gene itself is inactivated by a 50 bp insertion containing the meganuclease cleavage site to be tested (here, the HBB8 or HBB6 cleavage sites). These cell lines can in turn be used to evaluate DSB-induced gene targeting efficiencies (LacZ repair) with engineered I-*Cre*I derivatives cleaving the HBB8 target (Figure 27, step3).

The two cell lines were co-transfected with the repair matrix and various amounts of the vectors expressing the meganucleases. The frequency of repair of the LacZ gene increased from a maximum of 3.9 x10⁻³ with the cell line harboring the HBB6 target, to a maximum of 8.8 x 10⁻² with the cell line harboring the HBB8 target (Figure 28).

These finding confirm that in a chromosomal context the HBB8 target with the mutation responsible of the Sickle Cell Anemia is also more efficiently cleaved than HBB6 target derived from the wild type locus. Such result is in agreement with the results obtained with the extrachromosomal assay implying that I-*Cre*I optimized mutants may be used for genomic correction of a mutation responsible of a genetic disease.

### Example 15: Design of the strategy for the genome engineering at the HBB locus in human cell line

I-*Cre*I refined mutants able to efficiently cleave the HBB5 and HBB8/6 targets in yeast and in mammalian cells (CHO K1 cells) have been identified in examples 7, 8, 12, 13 and 14. It was therefore useful to construct the molecular tools to test the efficiency of the HBB meganucleases to correct the mutation responsible of the Sickle cell Anemia. For that purpose, a Knock-in matrix (KI) was designed to analyse the induction of homologous recombination by the heterodimeric combinations of I-*Cre*I refined mutants at the HBB locus of the lymphoblastoid human SC-1 (ATCC_CRL_8756) cell line.

### 1) Materials and methods

### a) Knock-in (KI) matrix

The Knock-in matrix consists on an hygromycin resistance coding sequence (CDS) cloned between two human HBB homology arms [LH HBB of 1730 bp from 4032375 to 4034105 and RH HBB of 2136 bp from 4034106 to 4036242 in the genomic contig NT009237.17. Position 4034105 is within the HBB5 target (position 11 of the HBB5 target) and corresponds to position 1247 on SEQ ID NO: 4 (figure 3). The resulting plasmid is described in Figure 29. The homology arms are amplified from genomic DNA purified from the human lymphoblastoid SC-1 cell line that is homozygous for the sickle cell allele. The Knock-in matrix includes an HBB6 wild type modified site (Figure 29) that is not cleaved by the HBB meganucleases and that does not change the open reading frame of the HBB protein. The coding sequence of the hygromycin resistance gene (*hygro* CDS) is operatively linked to an SV40 promoter region and to the SV40 polyA signal. An expression cassette for the thymidine kinase of HSV virus is included to set up a negative selection to Ganciclovir for the selection of clones corresponding to homologous recombination events at the HBB locus.

### b) Knock-in experiment in human SC-1 cell line

Human lymphoblastoid SC-1 cell line (ATCC_CRL_8756) is cultivated in complete RPMI 1640 medium supplemented with 20 % fetal calf serum, penicillin, streptomycin, fungizon, 10 mM HEPES, 1 mM sodium pyruvate, 4,5 g/L glucose, 1,5 g/L sodium bicarbonate. After transfection, selection is performed using Hygromycin in complete medium. After ten days of selection with Hygromycin, negative selection to Ganciclovir is applied to select clones that are resistant to Hygromycin and Ganciclovir. Such clones are amplified and genomic DNA extracted.

### c) PCR analysis of knock-in events

Knock-in events may be detected by PCR analysis on genomic DNA using the pair of primers located respectively in the human HBB sequence upstream of the LH HBB left homology arm and in the hygromycin CDS, to obtain a KI specific PCR amplification. The sequencing of the PCR amplified fragments would allow to determine the efficiency of the correction of the Sickle Cell Anemia mutation.

**Table XXII: Summary of the sequences presented in the sequence listing**

| SEQ ID NO: | Sequence |
|---|---|
| 1 | I-*Cre*I protein |
| 2 | C1221 target |
| 3 | Human HBB gene (1606 bp) |
| 4 | 4080 bp fragment |
| 5 | I-*Cre*I N75 scaffold |
| 6 to 19 | DNA targets (figure 16) |
| 20 | 10GGT_P (figure 4) |
| 21 | 10AAG_P (figure 4) |
| 22 | SCCT_ P (figure 4) |
| 23 | 5AGG_ P (figure 4) |
| 24 | HBB5.2 |
| 25 | HBB5.3 |
| 26 | HBB5.4 |
| 27 | 10AGA P (figure 5) |
| 28 | 10TGA_P (figure 5) |
| 29 | 5TCT_P (figure 5) |
| 30 | HBB8.2 |
| 31 | HBB8.3 |
| 32 | HBB8.4 |
| 33 | Oligo example 1 |
| 34 | Gal10F |
| 35 | Gal10p |
| 36 | AssF |
| 37 | AssR |
| 38 to 57 | 20 variants HBB5.3 Table II (including 12 variants HBB5.3 Table VI) |
| 58 to 76 | 19 variants HBB5.4 Table III (including 11 variants HBB5.4 Table VIII) |
| 77 | preATGCreFor primer |
| 78 | I-CreIpostRev primer |
| 79 | Optimized variant HBB5.4 table V |
| 80 to 103 | 24 variants HBB8.3 Table XIII (including 9 variants HBB83 Table XVII) |
| 104 to 122 | 19 variants HBB8.4 Table XIV (including 12 variants HBB8.4 Table XIX) |
| 123 to 135 | First monomer figure 16 |
| 136 to 148 | Second monomer figure 16 |
| 149 | Human HBB mRNA |
| 150 | Human beta globin protein |
| 151 | G19SF |
| 152 | G19SR |
| 153 | F54LF |
| 154 | F54LR |
| 155 | E80KF |
| 156 | E80KR |
| 157 | F87LF |
| 158 | F87LR |
| 159 | V105AF |
| 160 | V105AR |
| 161 | I132VF |
| 162 | I132VR |
| 163 | Variant 3 HBB5.3 Table VI |
| 164 to 177 | 14 variants HBB5 Table VII |
| 178 | I-*Cre*I 1g9y |
| 179 to 186 | 8 variants HBB5 Table IX |
| 187 | G19wtF |
| 188 | F54wtR |
| 189 | G19wtR |
| 190 | F54wtF |
| 191 | E80wtR |
| 192 | E80wtF |
| 193 | V105wtR |
| 194 | V105wtF |
| 195 | I132wtR |
| 196 | I132wtF |
| 197 to 220 | 24 variants HBB5 Table XI |
| 221 | attB1-ICreI For primer |
| 222 | attB2-ICreI Rev primer |
| 223 to 230 | Variants 2, 6, 8, 9, 11, 12, 14 and 17 HBB8.3 Table XVII |
| 231 to 255 | 25 variants HBB8 Table XVIII |
| 256 | Variant 10 HBB8.4 Table XIX |
| 257 to 276 | 20 variants HBB8 Table XX |
| 277 to 280 | Variants HBB5.3 Table XII |
| 281 | Variants HBB5.4 Table XII |
| 282 | HBB8.5 |
| 283 | HBB8.6 |
| 284 | HBB6.5 |
| 285 | HBB6.6 |
| 286 | Variant HBB8.4 Table XXI |
| 287 to 291 | Variant HBB8.3 Table XXI |
| 292 to 294 | Variant HBB5.4 figure 19B |
| 295 to 297 | Variant HBB5.4 figure 20B |
| 298 to 301 | Variant HBB5.3 figure 17A |
| 302 to 304 | Variant HBB5.3 figure 19A |
| 305,306 | Variant HBB5.3 figure 20A |

## Claims

1. An I-*Cre*I variant, **characterized in that** at least one of the two I-*Cre*I monomers has at least two substitutions, one in each of the two functional subdomains of the LAGLIDADG core domain situated respectively from positions 26 to 40 and 44 to 77 of I-*Cre*I, said variant being able to cleave a DNA target sequence from the human beta globin gene, and being obtainable by a method comprising at least the steps of:
(a) constructing a first series of I-*Cre*I variants having at least one substitution in a first functional subdomain of the LAGLIDADG core domain situated from positions 26 to 40 of I-*Cre*I,
(b) constructing a second series of I-*Cre*I variants having at least one substitution in a second functional subdomain of the LAGLIDADG core domain situated from positions 44 to 77 of I-*Cre*I,
(c) selecting and/or screening the variants from the first series of step (a) which are able to cleave a mutant I-*Cre*I site wherein at least (i) the nucleotide triplet at positions -10 to -8 of the I-*Cre*I site has been replaced with the nucleotide triplet which is present at positions -10 to -8 of said DNA target sequence from the human beta globin gene and (ii) the nucleotide triplet at positions +8 to +10 has been replaced with the reverse complementary sequence of the nucleotide triplet which is present at positions -10 to -8 of said DNA target sequence from the human beta globin gene,
(d) selecting and/or screening the variants from the second series of step (b) which are able to cleave a mutant I-*Cre*I site wherein at least (i) the nucleotide triplet at positions -5 to -3 of the I-*Cre*I site has been replaced with the nucleotide triplet which is present at positions -5 to -3 of said DNA target sequence from the human beta globin gene and (ii) the nucleotide triplet at positions +3 to +5 has been replaced with the reverse complementary sequence of the nucleotide triplet which is present at positions -5 to -3 of said DNA target sequence from the human beta globin gene,
(e) selecting and/or screening the variants from the first series of step (a) which are able to cleave a mutant I-*Cre*I site wherein at least (i) the nucleotide triplet at positions +8 to +10 of the I-*Cre*I site has been replaced with the nucleotide triplet which is present at positions +8 to +10 of said DNA target sequence from the human beta globin gene and (ii) the nucleotide triplet at positions -10 to -8 has been replaced with the reverse complementary sequence of the nucleotide triplet which is present at position +8 to +10 of said DNA target sequence from the human beta globin gene,
(f) selecting and/or screening the variants from the second series of step (b) which are able to cleave a mutant I-*Cre*I site wherein at least (i) the nucleotide triplet at positions +3 to +5 of the I-*Cre*I site has been replaced with the nucleotide triplet which is present at positions +3 to +5 of said DNA target sequence from the human beta globin gene and (ii) the nucleotide triplet at positions -5 to -3 has been replaced with the reverse complementary sequence of the nucleotide triplet which is present at position +3 to +5 of said DNA target sequence from the human beta globin gene,
(g) combining in a single variant, the mutation(s) at positions 26 to 40 and 44 to 77 of two variants from step (c) and step (d), to obtain a novel homodimeric I-*Cre*I variant which cleaves a sequence wherein (i) the nucleotide triplet at positions -10 to -8 is identical to the nucleotide triplet which is present at positions -10 to -8 of said DNA target sequence from the human beta globin gene, (ii) the nucleotide triplet at positions +8 to +10 is identical to the reverse complementary sequence of the nucleotide triplet which is present at positions -10 to -8 of said DNA target sequence from the human beta globin gene, (iii) the nucleotide triplet at positions -5 to -3 is identical to the nucleotide triplet which is present at positions -5 to -3 of said DNA target sequence from the human beta globin gene and (iv) the nucleotide triplet at positions +3 to +5 is identical to the reverse complementary sequence of the nucleotide triplet which is present at positions -5 to -3 of said DNA target sequence from the human beta globin gene, and/or
(h) combining in a single variant, the mutation(s) at positions 26 to 40 and 44 to 77 of two variants from step (e) and step (f), to obtain a novel homodimeric I-*Cre*I variant which cleaves a sequence wherein (i) the nucleotide triplet at positions +3 to +5 is identical to the nucleotide triplet which is present at positions +3 to +5 of said DNA target sequence from the human beta globin gene, (ii) the nucleotide triplet at positions -5 to -3 is identical to the reverse complementary sequence of the nucleotide triplet which is present at positions +3 to +5 of said DNA target sequence from the human beta globin gene, (iii) the nucleotide triplet at positions +8 to +10 of the I-*Cre*I site has been replaced with the nucleotide triplet which is present at positions +8 to +10 of said DNA target sequence from the human beta globin gene and (iv) the nucleotide triplet at positions -10 to -8 is identical to the reverse complementary sequence of the nucleotide triplet at positions +8 to +10 of said DNA target sequence from the human beta globin gene,
(i) combining the variants obtained in steps (g) and/or (h) to form heterodimers, and
(j) selecting and/or screening the heterodimers from step (i) which are able to cleave said DNA target sequence from the human beta globin gene.

2. The variant of claim 1, wherein said substitution(s) in the subdomain situated from positions 44 to 77 of I-*Cre*I are at positions 44, 68, 70, 75 and/or 77 and, wherein said substitution(s) in the subdomain situated from positions 26 to 40 of I-*Cre*I are at positions 26, 28, 30, 32, 33, 38 and/or 40.

3. The variant of anyone of claims 1 to 2, wherein said substitutions are replacement of the initial amino acids with amino acids selected in the group consisting of A, D, E, G, H, K, N, P, Q, R, S, T , Y, C, W, L and V.

4. The variant of anyone of claims 1 to 3, which is an heterodimer, resulting from the association of a first and a second monomer having different mutations at positions 26 to 40 and/or 44 to 77 of I-*Cre*I, said heterodimer being able to cleave a non-palindromic DNA target sequence from the human beta globin gene, preferably wherein said DNA target is selected from the group consisting of the sequences SEQ ID NO: 6 to 19.

5. The variant of claim 4, wherein the first and the second monomer, respectively, have amino acids at positions 28, 30, 32, 33, 38, 40, 44, 68, 70, 75 and 77 which are seleted from the group consisting of: KSSGQS/DNSNI and KNSTAS/QRSYR, KDSRQS/QRSNI and KNGTQS/ARGNI, KNDYCS/QRSDK and KNDYCS/QRSNI, KNSTAS/ARHDI and KNSHSS/YDSRY, KNGTQS/KESYV and KHSHQS/PRHNI, KDTYQS/QYSYI and KNDYCS/QYSRQ, KTSGQS/QHHNI and KNSRTS/ARHDI, KHSSQS/QYSYI and KDSRQS/QHHDI, KNWTQS/ARHDI and KNTCQS/ARGNI, KNTYWS/NYSRV and KNTTQS/ARSER, KQSHQS/ARSER and KNNGYS/QRSRQ, preferably, wherein the first monomer has amino acids at positions 28, 30, 32, 33, 38, 40, 44, 68, 70, 75 and 77 which are seleted from the group consisting of:KNSTSS/RYSNQ, KNTCQS/RYSNQ, KNTCQS/HYSNR, KNSCHS/RYSNQ, KNSVHS/RYSNQ, KNSTRQ/NESNR, KNETQS/DASKR, KNTCQS/DASKR, KNSCHS/DASKR, KNSCQS/DASKR, KNSSSS/KASDR, KNSSRS/DASKR, KNSTRQ/DASKR, KNSVRQ/DASKR, KNSTQS/DASKR and KNSVHQ/DASKR, and the second monomer has amino acids at positions 28, 30, 32, 33, 38, 40, 44, 68, 70, 75 and 77 which are seleted from the group consisting of: KNSGKS/QASNR, KTSHRS/KYSNY, KNSTRS/KYSNY, KNSGRS/KYSNY, KNSCRS/KYSNQ, KNSGKS/KYSNY, KNSGKS/QYSNR, KNSGKS/KYSNR, KNSGKS/KYSNQ, KQTYRS/KYSNI, KQTYRS/KYSNY, KQTYRS/QASNR, KQTYRS/KYSNQ, KNSRTS/QHHNI and KTSRQS/KSSNY or, wherein the first monomer has amino acids at positions 28, 30, 32, 33, 38, 40, 44, 68, 70, 75 and 77 which are seleted from the group consisting of: KNTYQS/ARSER, KNDYQS/ARSER, KNPYQS/ARSER, KNSPQS/ARSER and KNSYQS/ARSER, and the second monomer has amino acids at positions 28, 30, 32, 33, 38, 40, 44, 68, 70, 75 and 77 which are seleted from the group consisting of: KTSHRS/KYSDT, KNSRRS/KYSDT; KNSRRS/KYSDR, KNSRRS/KYSNQ, KNSRRS/RYSNQ, KSSHRS/KYSDT, KNSSKS/KYSNQ, KNSSKS/KYSDR, KNSRRS/KYSNQ, KSSHRS/KYSDQ, KSPHRS/KYSDT, KSSHRS/KYSNQ and KKSSQS/KESNR.

6. The variant of anyone of claims 1 to 5, which comprises one or more substitutions at positions 137 to 143 of I-*Cre*I that modify the specificity of the variant towards the nucleotide at positions ± 1 to 2, ± 6 to 7 and/or ± 11 to 12 of the I-*Cre*I site.

7. The variant of anyone of claims 1 to 6, which comprises one or more substitutions on the entire I-*Cre*I sequence that improve the binding and/or the cleavage properties of the variant towards said DNA target sequence from the human beta globin gene, preferably the variant comprises one or more substitutions selected from the group consisting of: K4E, K7R, Y12H, F16L, G19S, G19A, I24V, K34R, F43L, T49A, F54L, L58Q, D60N, D60G, V64I, Y66H, S79G, E80G, E80K, I81T, K82R, K82E, H85R, N86S, F87L, Q92R, P93A, F94L, K96R, Q99R, K100R, N103S, V105A, V105I, I109V, Q111H, E117G, D120G, K121R, K121E, V125A, V129A, Q131R, I132V, K139R, T140A, T147A, V151M, L152Q, L155P, K159R, K159E, K160E, S161P, S162P and P163S.

8. The variant of anyone of claims 5 to 7, wherein the first and the second monomer, respectively, have amino acids at positions 28, 30, 32, 33, 38, 40, 44, 68, 70, 75 and 77 and at additional positions, which are seleted from the group consisting of:
- KNSVHQ/DASKR, 87L and 131R (first monomer) and KNSGKS/KYSNY, 19S and 117G, KQTYRS/KYSNY, 19S and 641, KNSGKS/KYSNY, 19S and 132V, KQTYRS/KYSNY and 54L, KQTYRS/KYSNQ and 19S (second monomer),
- KNSTRQ/DASKR and 19S (first monomer) and KQTYRS/KYSNY, 105A and 152Q, KQTYRS/KYSNY and 54L, KQTYRS/QASNR and 87L, KQTYRS/KYSNI and 19S (second monomer),
- KNSVRQ/DASKR and 19S (first monomer) and KQTYRS/QASNR, 87L and 58Q, KQTYRS/KYSNY, 105A and 152Q, KNSGKS/KYSNY and 132V, KQTYRS/KYSNY and 54L, KQTYRS/QASNR and 87L (second monomer),
- KNSVHQ/DASKR and 87L (first monomer) and KNSGKS/KYSNY and 132V, KNSGKS/KYSNY, 19S and 117G, KNSGKS/KYSNY, 19S and 132V, KQTYRS/KYSNY and 54L, KQTYRS/KYSNI and 19S (second monomer), preferably wherein the first and the second monomer, respectively, have amino acids at positions 28, 30, 32, 33, 38, 40, 44, 68, 70, 75 and 77 and at additional positions, which are seleted from the group consisting of: KNTYQS/ARSER, 19S, 80K, 85R, 87L, 96R and 139R (first monomer) and KSPHRS/KYSDT and 81T or KTSHRS/KYSDT, 66H, 82R, 86S, 99R, 132V, 139R and 140A (second monomer).

9. The variant of anyone of claims 5 to 8, wherein the first monomer and the second monomer are selected from the following pairs of sequences:SEQ ID NO: 123 to 135 (first monomer) and SEQ ID NO: 136 to 148, respectively (second monomer); SEQ ID NO: 79 (first monomer) and any of SEQ ID NO: 45, 56, 164 to 177, 298 to 301 (second monomer); SEQ ID NO: 179 to 186 (first monomer) and SEQ ID NO: 57 (second monomer); SEQ ID NO: 179 (first monomer) and any of SEQ ID NO: 197 to 208, 302 to 306 (second monomer); SEQ ID NO: 209 to 220, 292 to 297 (first monomer) and SEQ ID NO: 164 (second monomer); SEQ ID NO: 213, 214, 281 (first monomer) and SEQ ID NO: 277, 278, 279 or 280 (second monomer); SEQ ID NO: 104 or 114 (first monomer) and SEQ ID NO: 82 (second monomer); SEQ ID NO: 105 or 118 (first monomer) and any of SEQ ID NO: 84, 85, 88, 94 and 103 (second monomer); SEQ ID NO: 113 or 111 (first monomer) and SEQ ID NO: 103 (second monomer); SEQ ID NO: 121 (first monomer) and SEQ ID NO: 85 (second monomer); SEQ ID NO: 118 (first monomer) and SEQ ID NO: 231 to 255 (second monomer); SEQ ID NO: 257 to 274 (first monomer) and SEQ ID NO: 103 (second monomer); SEQ ID NO: 286 (first monomer) and any of SEQ ID NO: 250, 236, 287, 288 and 289 (second monomer); SEQ ID NO: 269 (first monomer) and any of SEQ ID NO: 290, 236, 237 and 248 (second monomer); SEQ ID NO: 273 (first monomer) and any of SEQ ID NO: 291, 290, 242, 236 and 237 (second monomer); SEQ ID NO: 261 (first monomer) and any of SEQ ID NO: 242, 289, 287, 236 and 248 (second monomer), and variants which have at least 95 % sequence identity with one of these sequences.

10. The variant of anyone of claims 4 to 9, which is an obligate heterodimer, wherein the first monomer further comprises the D137R mutation and the second monomer further comprises the R51D mutation or wherein the first monomer further comprises the E8R or E8K and E61R mutations and the second monomer further comprises the K7E and K96E mutations.

11. A single-chain meganuclease comprising two monomers or core domains of one variant of anyone of claims 1 to 10, or a combination of both.

12. A polynucleotide fragment encoding the variant of anyone of claims 1 to 10 or the single-chain meganuclease of claim 11.

13. An expression vector comprising at least one polynucleotide fragment of claim 12, preferably the expression vector comprises two different polynucleotide fragments, each encoding one of the monomers of an heterodimeric variant of anyone of claims 4 to 10 and which includes a targeting construct comprising a sequence to be introduced in the human beta globin gene and a sequence homologous to the sequence of the human beta globin gene flanking the genomic DNA cleavage site of the I-*Cre*I variant as defined in anyone of claims 1, 5 and 6.

14. The vector of claim 13, wherein the sequence homologous to the sequence of the human beta globin flanking the genomic DNA cleavage site of the I-*Cre*I variant is a fragment of the human beta globin gene comprising positions: 508 to 707, 651 to 850, 701 to 900, 1048 to 1247, 1147 to 1346, 1524 to 1723, 1599 to 1798, 1808 to 2007, 2084 to 2283, 2094 to 2293, 2245 to 2444, 2323 to 2522 and 2523 to 2722 of SEQ ID NO: 4.

15. A composition comprising at least one variant of anyone of claims 1 to 10, one single-chain meganuclease of claim 11, and/or one expression vector of anyone of claims 13 to 14, preferably further comprising a targeting DNA construct as defined in anyone of claims 13 to 14.

16. A host cell which is modified by at least one polynucleotide fragment as defined in claim 12 or one vector of anyone of claims 13 to 14.

17. A non-human transgenic animal comprising one or two polynucleotide fragments as defined in claim 12.

18. A transgenic plant comprising one or two polynucleotide fragments as defined in claim 12.

19. Use of at least one variant of anyone of claims 1 to 10, one single-chain meganuclease of claim 11, and/or one expression vector according to anyone of claims 13 to 14, for the preparation of a medicament for preventing, improving or curing a pathological condition caused by a mutation in the beta-globin gene, preferably wherein said pathological condition is selected from the group consisting of: sickle cell disease and beta-thalassemia.

20. Use of at least one variant of anyone of claims 1 to 10, one single-chain meganuclease of claim 11, and/or one expression vector according to anyone of claims 13 to 14 for genome engineering, for non-therapeutic purposes.

21. The use of claim 20, for making animal models of inherited hemoglobin disorders caused by a mutation in the beta globin gene.


SEQUENCE LISTING

<110>  CELLECTIS
       PEREZ-MICHAUT, Christophe

<120>  Meganuclease variants cleaving a DNA target sequence from the
       human hemoglobin beta gene and uses thereof

<130>  1546PCT19

<160>  306

<170>  PatentIn version 3.3

<210>  1
<211>  163
<212>  PRT
<213>  Chlamydomonas reinhardtii

<400>  1

Met Asn Thr Lys Tyr Asn Lys Glu Phe Leu Leu Tyr Leu Ala Gly Phe
1               5                   10                  15

Val Asp Gly Asp Gly Ser Ile Ile Ala Gln Ile Lys Pro Asn Gln Ser
            20                  25                  30

Tyr Lys Phe Lys His Gln Leu Ser Leu Ala Phe Gln Val Thr Gln Lys
        35                  40                  45

Thr Gln Arg Arg Trp Phe Leu Asp Lys Leu Val Asp Glu Ile Gly Val
    50                  55                  60

Gly Tyr Val Arg Asp Arg Gly Ser Val Ser Asp Tyr Ile Leu Ser Glu
65                  70                  75                  80

Ile Lys Pro Leu His Asn Phe Leu Thr Gln Leu Gln Pro Phe Leu Lys
                85                  90                  95

Leu Lys Gln Lys Gln Ala Asn Leu Val Leu Lys Ile Ile Trp Arg Leu
            100                 105                 110

Pro Ser Ala Lys Glu Ser Pro Asp Lys Phe Leu Glu Val Cys Thr Trp
        115                 120                 125

Val Asp Gln Ile Ala Ala Leu Asn Asp Ser Lys Thr Arg Lys Thr Thr
    130                 135                 140

Ser Glu Thr Val Arg Ala Val Leu Asp Ser Leu Ser Glu Lys Lys Lys
145                 150                 155                 160

Ser Ser Pro

<210>  2
<211>  24
<212>  DNA
<213>  artificial sequence

<220>
<223>  I-CreI C1221 DNA target

<400>  2
tcaaaacgtc gtacgacgtt ttga                                              24


<210>  3
<211>  1606
<212>  DNA
<213>  Homo sapiens

<400>  3
acatttgctt ctgacacaac tgtgttcact agcaacctca aacagacacc atggtgcatc      60

tgactcctga ggagaagtct gccgttactg ccctgtgggg caaggtgaac gtggatgaag     120

ttggtggtga ggccctgggc aggttggtat caaggttaca agacaggttt aaggagacca     180

atagaaactg ggcatgtgga gacagagaag actcttgggt ttctgatagg cactgactct     240

ctctgcctat tggtctattt tcccaccctt aggctgctgg tggtctaccc ttggacccag     300

aggttctttg agtcctttgg ggatctgtcc actcctgatg ctgttatggg caaccctaag     360

gtgaaggctc atggcaagaa agtgctcggt gcctttagtg atggcctggc tcacctggac     420

aacctcaagg gcacctttgc cacactgagt gagctgcact gtgacaagct gcacgtggat     480

cctgagaact tcagggtgag tctatgggac gcttgatgtt ttctttcccc ttcttttcta     540

tggttaagtt catgtcatag gaaggggata agtaacaggg tacagtttag aatgggaaac     600

agacgaatga ttgcatcagt gtggaagtct caggatcgtt ttagtttctt ttatttgctg     660

ttcataacaa ttgttttctt ttgtttaatt cttgctttct ttttttttct tctccgcaat     720

ttttactatt atacttaatg ccttaacatt gtgtataaca aaaggaaata tctctgagat     780

acattaagta acttaaaaaa aaactttaca cagtctgcct agtacattac tatttggaat     840

atatgtgtgc ttatttgcat attcataatc tccctacttt attttctttt atttttaatt     900

gatacataat cattatacat atttatgggt taaagtgtaa tgttttaata tgtgtacaca     960

tattgaccaa atcagggtaa ttttgcattt gtaattttaa aaaatgcttt cttcttttaa    1020

tatacttttt tgtttatctt atttctaata ctttccctaa tctctttctt tcagggcaat    1080

aatgatacaa tgtatcatgc ctctttgcac cattctaaag aataacagtg ataatttctg    1140

ggttaaggca atagcaatat ctctgcatat aaatatttct gcatataaat tgtaactgat    1200

gtaagaggtt tcatattgct aatagcagct acaatccagc taccattctg cttttatttt    1260

atggttggga taaggctgga ttattctgag tccaagctag gcccttttgc taatcatgtt    1320

catacctctt atcttcctcc cacagctcct gggcaacgtg ctggtctgtg tgctggccca    1380

tcactttggc aaagaattca ccccaccagt gcaggctgcc tatcagaaag tggtggctgg    1440

tgtggctaat gccctggccc acaagtatca ctaagctcgc tttcttgctg tccaatttct 1500

attaaaggtt cctttgttcc ctaagtccaa ctactaaact gggggatatt atgaagggcc 1560

ttgagcatct ggattctgcc taataaaaaa catttatttt cattgc 1606

<210> 4
<211> 4080
<212> DNA
<213> Homo sapiens

<220>
<221> exon
<222> (1079)..(1220)

<220>
<221> exon
<222> (1351)..(1573)

<220>
<221> exon
<222> (2424)..(2684)

<400> 4
tcacgttggg aagctataga gaaagaagag taaattttag taaaggaggt ttaaacaaac 60
aaaatataaa gagaaatagg aacttgaatc aaggaaatga ttttaaaacg cagtattctt 120
agtggactag aggaaaaaaa taatctgagc caagtagaag accttttccc ctcctacccc 180
tactttctaa gtcacagagg ctttttgttc ccccagacac tcttgcagat tagtccaggc 240
agaaacagtt agatgtcccc agttaacctc ctatttgaca ccactgatta ccccattgat 300
agtcacactt tgggttgtaa gtgacttttt atttatttgt atttttgact gcattaagag 360
gtctctagtt ttttatctct tgtttcccaa aacctaataa gtaactaatg cacagagcac 420
attgatttgt atttattcta tttttagaca taatttatta gcatgcatga gcaaattaag 480
aaaaacaaca acaaatgaat gcatatatat gtatatgtat gtgtgtatat atacacacat 540
atatatatat attttttctt ttcttaccag aaggttttaa tccaaataag gagaagatat 600
gcttagaacc gaggtagagt tttcatccat tctgtcctgt aagtattttg catattctgg 660
agacgcagga agagatccat ctacatatcc caaagctgaa ttatggtaga caaaactctt 720
ccacttttag tgcatcaact tcttatttgt gtaataagaa aattgggaaa acgatcttca 780
atatgcttac caagctgtga ttccaaatat tacgtaaata cacttgcaaa ggaggatgtt 840
tttagtagca atttgtactg atggtatggg gccaagagat atatcttaga gggagggctg 900
agggtttgaa gtccaactcc taagccagtg ccagaagagc caaggacagg tacggctgtc 960
atcacttaga cctcaccctg tggagccaca ccctagggtt ggccaatcta ctcccaggag 1020
cagggagggc aggagccagg gctgggcata aaagtcaggg cagagccatc tattgctt 1078
aca ttt gct tct gac aca act gtg ttc act agc aac ctc aaa cag aca 1126
cca tgg tgc atc tga ctc ctg agg aga agt ctg ccg tta ctg ccc tgt     1174
ggg gca agg tga acg tgg atg aag ttg gtg gtg agg ccc tgg gca g       1220
gttggtatca aggttacaag acaggtttaa ggagaccaat agaaactggg catgtggaga   1280
cagagaagac tcttgggttt ctgataggca ctgactctct ctgcctattg gtctattttc   1340
ccacccttag gc  tgc tgg tgg tct acc ctt gga ccc aga ggt tct ttg      1388
agt cct ttg ggg atc tgt cca ctc ctg atg ctg tta tgg gca acc cta     1436
agg tga agg ctc atg gca aga aag tgc tcg gtg cct tta gtg atg gcc     1484
tgg ctc acc tgg aca acc tca agg gca cct ttg cca cac tga gtg agc     1532
tgc act gtg aca agc tgc acg tgg atc ctg aga act tca gg              1573
gtgagtctat gggacgcttg atgttttctt tccccttctt ttctatggtt aagttcatgt   1633
cataggaagg ggataagtaa cagggtacag tttagaatgg gaaacagacg aatgattgca   1693
tcagtgtgga agtctcagga tcgttttagt ttcttttatt tgctgttcat aacaattgtt   1753
ttcttttgtt taattcttgc tttctttttt tttcttctcc gcaattttta ctattatact   1813
taatgcctta acattgtgta taacaaaagg aaatatctct gagatacatt aagtaactta   1873
aaaaaaaact ttacacagtc tgcctagtac attactattt ggaatatatg tgtgcttatt   1933
tgcatattca taatctccct actttatttt cttttatttt taattgatac ataatcatta   1993
tacatattta tgggttaaag tgtaatgttt taatatgtgt acacatattg accaaatcag   2053
ggtaattttg catttgtaat tttaaaaaat gctttcttct tttaatatac ttttttgttt   2113
atcttatttc taatactttc cctaatctct ttctttcagg gcaataatga tacaatgtat   2173
catgcctctt tgcaccattc taaagaataa cagtgataat ttctgggtta aggcaatagc   2233
aatatctctg catataaata tttctgcata taaattgtaa ctgatgtaag aggtttcata   2293
ttgctaatag cagctacaat ccagctacca ttctgctttt attttatggt tgggataagg   2353
ctggattatt ctgagtccaa gctaggccct tttgctaatc atgttcatac ctcttatctt   2413
cctcccacag c tcc tgg gca acg tgc tgg tct gtg tgc tgg ccc atc act    2463
ttg gca aag aat tca ccc cac cag tgc agg ctg cct atc aga aag tgg     2511
tgg ctg gtg tgg cta atg ccc tgg ccc aca agt atc act aag ctc gct     2559
ttc ttg ctg tcc aat ttc tat taa agg ttc ctt tgt tcc cta agt cca     2607
act act aaa ctg ggg gat att atg aag ggc ctt gag cat ctg gat tct     2655
gcc taa taa aaa aca ttt att ttc att gc aatgatgtat ttaaattatt     2704
tctgaatatt ttactaaaaa gggaatgtgg gaggtcagtg catttaaaac ataaagaaat     2764
gaagagctag ttcaaacctt gggaaaatac actatatctt aaactccatg aaagaaggtg     2824
aggctgcaaa cagctaatgc acattggcaa cagcccctga tgcatatgcc ttattcatcc     2884
ctcagaaaag gattcaagta gaggcttgat ttggaggtta aagttttgct atgctgtatt     2944
ttacattact tattgtttta gctgtcctca tgaatgtctt ttcactaccc atttgcttat     3004
cctgcatctc tcagccttga ctccactcag ttctcttgct tagagatacc acctttcccc     3064
tgaagtgttc cttccatgtt ttacggcgag atggtttctc ctcgcctggc cactcagcct     3124
tagttgtctc tgttgtctta tagaggtcta cttgaagaag gaaaaacagg ggtcatggtt     3184
tgactgtcct gtgagccctt cttccctgcc tcccccactc acagtgaccc ggaatctgca     3244
gtgctagtct cccggaacta tcactctttc acagtctgct ttggaaggac tgggcttagt     3304
atgaaaagtt aggactgaga agaatttgaa aggcggcttt ttgtagcttg atattcacta     3364
ctgtcttatt accctgtcat aggcccaccc caaatggaag tcccattctt cctcaggatg     3424
tttaagatta gcattcagga agagatcaga ggtctgctgg ctcccttatc atgtccctta     3484
tggtgcttct ggctctgcag ttattagcat agtgttacca tcaaccacct taacttcatt     3544
tttcttattc aatacctagg taggtagatg ctagattctg gaaataaaat atgagtctca     3604
agtggtcctt gtcctctctc ccagtcaaat tctgaatcta gttggcaaga ttctgaaatc     3664
aaggcatata atcagtaata agtgatgata gaagggtata tagaagaatt ttattatatg     3724
agagggtgaa accctcaaaa tgaaatgaaa tcagaccctt gtcttacacc ataaacaaaa     3784
ataaatttga atgggttaaa gaattaaact aagacctaaa accataaaaa tttttaaaga     3844
aatcaaaaga agaaaattct aatattcacg ttgcagccgt tttttgaatt tgatatgaga     3904
agcaaaggca acaaaaggaa aaataaagaa gtgaggctac atcaaactaa aaaatttcca     3964
cacaaaaaac aaaacaatga acaaatgaaa ggtgaaccat gaaatggcat atttgcaaac     4024
caaatatttc ttaaatattt tggttaatat ccaaaatata taagaaacac agatga     4080

<210>  5
<211>  167
<212>  PRT
<213>  artificial sequence

<220>
<223>  I-CreI N75 scaffold protein

<400>  5

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Tyr Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Arg Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  6
<211>  24
<212>  DNA
<213>  artificial sequence

<220>
<223>  human HBB gene target

<400>  6
atatgcttag aaccgaggta gagt                                              24


<210>  7
<211>  24
<212>  DNA
<213>  artificial sequence

<220>
<223>  human HBB gene target

<400>  7
ttcttatttg tgtaataaga aaat                                              24


<210>  8
<211>  24
<212>  DNA
<213>  artificial sequence

<220>
<223>  human HBB gene target

<400>  8
ccaagctgtg attccaaata ttac                                              24


<210>  9
<211>  24
<212>  DNA
<213>  artificial sequence

<220>
<223>  human HBB gene target

<400>  9
tctgactcct gaggagaagt ctgc                                              24


<210>  10
<211>  24
<212>  DNA
<213>  artificial sequence

<220>
<223>  human HBB gene target

<400>  10
tctgactcct gtggagaagt ctgc                                              24


<210>  11
<211>  24
<212>  DNA
<213>  artificial sequence

<220>
<223>  human HBB gene target

<400>  11
acaagacagg tttaaggaga ccaa                                              24


<210>  12

<211>  24
<212>  DNA
<213>  artificial sequence

<220>
<223>  human HBB gene target

<400>  12
tttctatggt taagttcatg tcat                                              24


<210>  13
<211>  24
<212>  DNA
<213>  artificial sequence

<220>
<223>  human HBB gene target

<400>  13
attgcatcag tgtggaagtc tcag                                              24


<210>  14
<211>  24
<212>  DNA
<213>  artificial sequence

<220>
<223>  human HBB gene target

<400>  14
ctagtacatt actatttgga atat                                              24


<210>  15
<211>  24
<212>  DNA
<213>  artificial sequence

<220>
<223>  human HBB gene target

<400>  15
tcatgcctct ttgcaccatt ctaa                                              24


<210>  16
<211>  24
<212>  DNA
<213>  artificial sequence

<220>
<223>  human HBB gene target

<400>  16
ttgcaccatt ctaaagaata acag                                              24


<210>  17
<211>  24
<212>  DNA
<213>  artificial sequence

<220>

<223>  human HBB gene target

<400>  17
attttatggt tgggataagg ctgg                                              24


<210>  18
<211>  24
<212>  DNA
<213>  artificial sequence

<220>
<223>  human HBB gene target

<400>  18
ttcctcccac agctcctggg caac                                              24


<210>  19
<211>  24
<212>  DNA
<213>  artificial sequence

<220>
<223>  human HBB gene target

<400>  19
ctaaactggg ggatattatg aagg                                              24


<210>  20
<211>  24
<212>  DNA
<213>  artificial sequence

<220>
<223>  10 GGT_P target

<400>  20
tcggtacgtc gtacgacgta ccga                                              24


<210>  21
<211>  24
<212>  DNA
<213>  artificial sequence

<220>
<223>  10AAG_P target

<400>  21
tcaagacgtc gtacgacgtc ttga                                              24


<210>  22
<211>  24
<212>  DNA
<213>  artificial sequence

<220>
<223>  5CCT_P target

<400>  22
tcaaaaccct gtacagggtt ttga                                              24

<210>  23
<211>  24
<212>  DNA
<213>  artificial sequence

<220>
<223>  5AAG_P target

<400>  23
tcaaaacagg gtaccctgtt ttga                                              24


<210>  24
<211>  22
<212>  DNA
<213>  artificial sequence

<220>
<223>  HBB5.2 target

<400>  24
caagacaggg tacaggagac ca                                                22


<210>  25
<211>  22
<212>  DNA
<213>  artificial sequence

<220>
<223>  HBB5.3 target

<400>  25
tggtctcctg tacaggagac ca                                                22


<210>  26
<211>  22
<212>  DNA
<213>  artificial sequence

<220>
<223>  HBB5.4 target

<400>  26
caagacaggg taccctgact tg                                                22


<210>  27
<211>  24
<212>  DNA
<213>  artificial sequence

<220>
<223>  10AGA_P target

<400>  27
tcagaacgtc gtacgacgtt ctga                                              24


<210>  28
<211>  24
<212>  DNA
<213>  artificial sequence

<220>
<223>  10TGA_P target

<400>  28
tctgaacgtc gtacgacgtt caga                                              24


<210>  29
<211>  24
<212>  DNA
<213>  artificial sequence

<220>
<223>  5TCT_P target

<400>  29
tcaaaactct gtacagagtt ttga                                              24


<210>  30
<211>  22
<212>  DNA
<213>  artificial sequence

<220>
<223>  HBB8.2 target

<400>  30
ctgactcctg tacagaagtc tg                                                22


<210>  31
<211>  22
<212>  DNA
<213>  artificial sequence

<220>
<223>  HBB8.3 target

<400>  31
cagacttctg tacagaagtc tg                                                22


<210>  32
<211>  22
<212>  DNA
<213>  artificial sequence

<220>
<223>  HBB8.4 target

<400>  32
ctgactcctg tacaggagtc ag                                                22


<210>  33
<211>  50
<212>  DNA
<213>  artificial sequence

<220>
<223>  target oligonucleotide

<400>  33

tggcatacaa gttttggtct cctgtacagg agaccaacaa tcgtctgtca 50

<210> 34
<211> 26
<212> DNA
<213> artificial sequence

<220>
<223> Gal10F primer

<400> 34
gcaactttag tgctgacaca tacagg 26

<210> 35
<211> 24
<212> DNA
<213> artificial sequence

<220>
<223> Gal10R primer

<400> 35
acaaccttga ttggagactt gacc 24

<210> 36
<211> 15
<212> DNA
<213> artificial sequence

<220>
<223> AssF primer

<220>
<221> misc_feature
<222> (4)..(6)
<223> n is a, c, g, or t

<400> 36
ctannnttga ccttt 15

<210> 37
<211> 15
<212> DNA
<213> artificial sequence

<220>
<223> AssR primer

<220>
<221> misc_feature
<222> (10)..(12)
<223> n is a, c, g, or t

<400> 37
aaaggtcaan nntag 15

<210> 38
<211> 167

<212>  PRT
<213>  artificial sequence

<220>
<223>  I-CreI variant/HBB5.3

<400>  38

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Cys Gln
            20                  25                  30

Ser Arg Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  39
<211>  167
<212>  PRT
<213>  artificial sequence

<220>
<223>  I-CreI variant/HBB5.3

<400>  39

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Lys Gln
            20                  25                  30

Ser Ala Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  40
<211>  167
<212>  PRT
<213>  artificial sequence

<220>
<223>  I-CreI variant/HBB5.3

<400>  40

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Lys Gln
            20                  25                  30

Ser Ala Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  41
<211>  167
<212>  PRT
<213>  artificial sequence

<220>
<223>  I-CreI variant/HBB5.3

<400>  41

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Gly His Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln

            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 42
<211> 167
<212> PRT
<213> artificial sequence

<220>
<223> I-CreI variant/HBB5.3 target

<400> 42

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser His Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  43
<211>  167
<212>  PRT
<213>  artificial sequence

<220>
<223>  I-CreI variant/HBB5.3 target

<400>  43

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asp Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  44
<211>  167
<212>  PRT

<213>  artificial sequence

<220>
<223>  I-CreI variant HBB5.3 target

<400>  44

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  45
<211>  167
<212>  PRT
<213>  artificial sequence

<220>
<223>  I-CreI variant/HBB5.3 target

<400>  45

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 46
<211> 167
<212> PRT
<213> artificial sequence

<220>
<223> I-CreI variant/HBB5.3 target

<400> 46

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  47
<211>  167
<212>  PRT
<213>  artificial sequence

<220>
<223>  I-CreI variant/HBB5.3 target

<400>  47

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asp Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  48
<211>  167
<212>  PRT
<213>  artificial sequence

<220>
<223>  I-CreI variant/HBB5.3 target

<400>  48

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys

145                 150                 155                 160

Lys Ser Ser Pro Ala Ala Asp
                165

<210>  49
<211>  167
<212>  PRT
<213>  artificial sequence

<220>
<223>  I-CreI variant/HBB5.3 target

<400>  49

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Asn Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  50
<211>  167
<212>  PRT
<213>  artificial sequence

<220>
<223>  I-CreI variant/HBB5.3 target

<400>  50

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Tyr Tyr Asn Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  51
<211>  167
<212>  PRT
<213>  artificial sequence

<220>
<223>  I-CreI variant/HBB5.3 target

<400>  51

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln

            20                  25                  30

Ser Ser Lys Phe Lys His Thr Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ser Asp Ser Gly Ser Val Ser Asn Tyr Leu Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 52
<211> 167
<212> PRT
<213> artificial sequence

<220>
<223> I-CreI variant/HBB5.3 target

<400> 52

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Arg Gln
            20                  25                  30

Asp Tyr Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  53
<211>  167
<212>  PRT
<213>  artificial sequence

<220>
<223>  I-CreI variant/HBB5.3 target

<400>  53

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Arg Gln
            20                  25                  30

Asp Tyr Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 54
<211> 167
<212> PRT
<213> artificial sequence

<220>
<223> I-CreI variant/HBB5.3 target

<400> 54

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  55
<211>  167
<212>  PRT
<213>  artificial sequence

<220>
<223>  I-CreI variant/HBB5.3 target

<400>  55

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  56
<211>  167
<212>  PRT
<213>  artificial sequence

<220>
<223>  I-CreI variant/HBB5.3 target

<400>  56

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  57
<211>  167
<212>  PRT
<213>  artificial sequence

<220>
<223>  I-CreI variant/HBB5.3 target

<400>  57

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser Arg Lys Phe Lys His His Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ser Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  58
<211>  167
<212>  PRT
<213>  artificial sequence

<220>
<223>  I-CreI variant/HBB5.4

<400>  58

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gly Gln
            20                  25                  30

Ser Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser

65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  59
<211>  167
<212>  PRT
<213>  artificial sequence

<220>
<223>  I-CreI variant/HBB5.4 target

<400>  59

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gly Gln
            20                  25                  30

Ser Tyr Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Glu Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  60
<211>  167
<212>  PRT
<213>  artificial sequence

<220>
<223>  I-CreI variant/HBB5.4 target

<400>  60

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gly Gln
            20                  25                  30

Ser Tyr Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Glu Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  61
<211>  167
<212>  PRT
<213>  artificial sequence

<220>
<223>  I-CreI variant/HBB5.4 target

<400>  61

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gly Gln
            20                  25                  30

Ser Tyr Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  62
<211>  167
<212>  PRT
<213>  artificial sequence

<220>

<223>  I-CreI variant/HBB5.4 target

<400>  62

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Asp Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  63
<211>  167
<212>  PRT
<213>  artificial sequence

<220>
<223>  I-CreI variant/HBB5.4 target

<400>  63

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Gln Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  64
<211>  167
<212>  PRT
<213>  artificial sequence

<220>
<223>  I-CreI variant/HBB5.4 target

<400>  64

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  65
<211>  167
<212>  PRT
<213>  artificial sequence

<220>
<223>  I-CreI variant/HBB5.4 target

<400>  65

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Tyr Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Pro Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr

115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  66
<211>  167
<212>  PRT
<213>  artificial sequence

<220>
<223>  I-CreI variant/HBB5.4 target

<400>  66

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Tyr Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  67
<211>  167
<212>  PRT
<213>  artificial sequence

<220>
<223>  I-CreI variant/HBB5.4 target

<400>  67

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  68
<211>  167
<212>  PRT
<213>  artificial sequence

<220>
<223>  I-CreI variant/HBB5.4 target

<400>  68

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Glu Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  69
<211>  167
<212>  PRT
<213>  artificial sequence

<220>
<223>  I-CreI variant/HBB5.4 target

<400>  69

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Glu Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  70
<211>  167
<212>  PRT
<213>  artificial sequence

<220>
<223>  I-CreI variant/HBB5.4 target

<400>  70

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  71
<211>  167
<212>  PRT
<213>  artificial sequence

<220>
<223>  I-CreI variant/HBB5.4 target

<400>  71

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  72
<211>  167
<212>  PRT
<213>  artificial sequence

<220>
<223>  I-CreI variant/HBB5.4 target

<400>  72

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Ser Arg Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp

                165

<210> 73
<211> 167
<212> PRT
<213> artificial sequence

<220>
<223> I-CreI variant/HBB5.4 target

<400> 73

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Tyr Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 74
<211> 167
<212> PRT
<213> artificial sequence

<220>
<223> I-CreI variant/HBB5.4 target

<400>  74

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser Arg Lys Phe Lys His Gln Leu Ser Leu Thr Phe Thr Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  75
<211>  167
<212>  PRT
<213>  artificial sequence

<220>
<223>  I-CreI variant/HBB5.4 target

<400>  75

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Ser Pro Asn Gln
            20                  25                  30

Ser Tyr Lys Phe Lys His Gln Leu Lys Leu Thr Phe Thr Val Thr Gln

        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 76
<211> 167
<212> PRT
<213> artificial sequence

<220>
<223> I-CreI variant/HBB5.4 target

<400> 76

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Ser Pro Asn Gln
            20                  25                  30

Ser Tyr Lys Phe Lys His Gln Leu Lys Leu Thr Phe Tyr Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Gln Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  77
<211>  59
<212>  DNA
<213>  artificial sequence

<220>
<223>  preATGF primer

<400>  77
gcataaatta ctatacttct atagacacgc aaacacaaat acacagcggc cttgccacc      59

<210>  78
<211>  48
<212>  DNA
<213>  artificial sequence

<220>
<223>  I-CreIpostRev primer

<400>  78
ggctcgagga gctcgtctag aggatcgctc gagttatcag tcggccgc                  48

<210>  79
<211>  167
<212>  PRT
<213>  artificial sequence

<220>
<223>  optimized I-CreI variant/HBB5.4 target

<400>  79

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  80
<211>  167
<212>  PRT
<213>  artificial sequence

<220>
<223>  I-CreI variant HBB8.3 target

<400>  80

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asp Gln
            20                  25                  30

Ser Arg Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  81
<211>  167
<212>  PRT
<213>  artificial sequence

<220>
<223>  I-CreI variant/HBB8.3 target

<400>  81

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asp Gln
            20                  25                  30

Ser Arg Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr

        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 82
<211> 167
<212> PRT
<213> artificial sequence

<220>
<223> I-CreI variant/HBB8.3 target

<400> 82

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  83
<211>  167
<212>  PRT
<213>  artificial sequence

<220>
<223>  I-CreI variant/HBB8.3 target

<400>  83

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  84
<211>  167
<212>  PRT
<213>  artificial sequence

<220>
<223>  I-CreI variant/HBB8.3 target

<400>  84

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  85
<211>  167
<212>  PRT
<213>  artificial sequence

<220>
<223>  I-CreI variant/HBB8.3 target

<400>  85

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  86
<211>  167
<212>  PRT
<213>  artificial sequence

<220>
<223>  I-CreI variant/HBB8.3 target

<400>  86

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Asn Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  87
<211>  167
<212>  PRT
<213>  artificial sequence

<220>
<223>  I-CreI variant/HBB8.4 target

<400>  87

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  88
<211>  167
<212>  PRT
<213>  artificial sequence

<220>
<223>  I-CreI variant/HBB8.4 target

<400>  88

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp

                165

<210> 89
<211> 166
<212> PRT
<213> artificial sequence

<220>
<223> I-CreI variant/HBB8.3 target

<400> 89

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Lys Lys Phe Lys His Arg Leu Ser Leu Thr Phe Lys Val Thr Gln Lys
        35                  40                  45

Thr Gln Arg Arg Trp Phe Leu Asp Lys Leu Val Asp Glu Ile Gly Val
    50                  55                  60

Gly Tyr Val Tyr Asp Ser Gly Ser Val Ser Asn Tyr Tyr Leu Ser Glu
65                  70                  75                  80

Ile Lys Pro Leu His Asn Phe Leu Thr Gln Leu Gln Pro Phe Leu Lys
                85                  90                  95

Leu Lys Gln Lys Gln Ala Asn Leu Val Leu Lys Ile Ile Glu Gln Leu
            100                 105                 110

Pro Ser Ala Lys Glu Ser Pro Asp Lys Phe Leu Glu Val Cys Thr Trp
        115                 120                 125

Val Asp Gln Ile Ala Ala Leu Asn Asp Ser Lys Thr Arg Lys Thr Thr
    130                 135                 140

Ser Glu Thr Val Arg Ala Val Leu Asp Ser Leu Ser Glu Lys Lys Lys
145                 150                 155                 160

Ser Ser Pro Ala Ala Asp
                165

<210> 90
<211> 167
<212> PRT
<213> artificial sequence

<220>
<223> I-CreI variant/HBB8.3 target

<400>  90

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Asn Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  91
<211>  167
<212>  PRT
<213>  artificial sequence

<220>
<223>  I-CreI variant/HBB8.3 target

<400>  91

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Asn Leu Ser Leu Thr Phe Lys Val Thr Gln

35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  92
<211>  167
<212>  PRT
<213>  artificial sequence

<220>
<223>  I-CreI variant/HBB8.3 target

<400>  92

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Thr Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  93
<211>  167
<212>  PRT
<213>  artificial sequence

<220>
<223>  I-CreI variant/HBB8.3 target

<400>  93

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  94
<211>  167
<212>  PRT
<213>  artificial sequence

<220>
<223>  I-CreI variant/HBB8.3 target

<400>  94

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  95
<211>  167
<212>  PRT
<213>  artificial sequence

<220>
<223>  I-CreI variant/HBB8.3 target

<400>  95

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  96
<211>  167
<212>  PRT
<213>  artificial sequence

<220>
<223>  I-CreI variant/HBB8.3 target

<400>  96

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  97
<211>  167
<212>  PRT
<213>  artificial sequence

<220>
<223>  I-CreI variant/HBB8.3 target

<400>  97

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Arg Gln
            20                  25                  30

Asp Tyr Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  98
<211>  167
<212>  PRT
<213>  artificial sequence

<220>
<223>  I-CreI variant/HBB8.3 target

<400>  98

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Arg Gln
            20                  25                  30

Asp Tyr Lys Phe Lys His Ser Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu

85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  99
<211>  167
<212>  PRT
<213>  artificial sequence

<220>
<223>  I-CreI variant/HBB8.3 target

<400>  99

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser Gly Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Leu Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  100
<211>  167
<212>  PRT
<213>  artificial sequence

<220>
<223>  I-CreI variant/HBB8.3 target

<400>  100

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser Gly Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  101
<211>  167
<212>  PRT
<213>  artificial sequence

<220>
<223>  I-CreI variant/HBB8.3 target

<400>  101

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser Gly Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Asn Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  102
<211>  167
<212>  PRT
<213>  artificial sequence

<220>
<223>  I-CreI variant/HBB8.3 target

<400>  102

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  103
<211>  167
<212>  PRT
<213>  artificial sequence

<220>
<223>  I-CreI variant/HBB8.3 target

<400>  103

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  104
<211>  167
<212>  PRT
<213>  artificial sequence

<220>
<223>  I-CreI variant/HBB8.4 target

<400>  104

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Glu Thr Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  105
<211>  167
<212>  PRT
<213>  artificial sequence

<220>
<223>  I-CreI variant/HBB8.4 target

<400>  105

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His His Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr

130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  106
<211>  167
<212>  PRT
<213>  artificial sequence

<220>
<223>  I-CreI variant/HBB8.4 target

<400>  106

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His Thr Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ser Asp Ser Gly Ser Val Ser Asn Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 107
<211> 167
<212> PRT
<213> artificial sequence

<220>
<223> I-CreI variant/HBB8.4 target

<400> 107

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His Thr Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 108
<211> 167
<212> PRT
<213> artificial sequence

<220>
<223> I-CreI variant/HBB8.4 target

<400> 108

Met Ala Asn Thr Lys Tyr Asn Lys Glu Phe Leu Leu Tyr Leu Ala Gly

1               5                   10                  15

Phe Val Asp Gly Asp Gly Ser Ile Ile Ala Gln Ile Lys Pro Asn Gln
            20                  25                  30

Ser Cys Lys Phe Lys His Thr Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  109
<211>  167
<212>  PRT
<213>  artificial sequence

<220>
<223>  I-CreI variant/HBB8.4 target

<400>  109

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Thr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  110
<211>  167
<212>  PRT
<213>  artificial sequence

<220>
<223>  I-CreI variant/HBB8.4 target

<400>  110

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Thr Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Thr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  111
<211>  167
<212>  PRT
<213>  artificial sequence

<220>
<223>  I-CreI variant/HBB8.4 target

<400>  111

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Ser Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  112
<211>  167
<212>  PRT
<213>  artificial sequence

<220>
<223>  I-CreI variant/HBB8.4 target

<400>  112

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  113

<211>  167
<212>  PRT
<213>  artificial sequence

<220>
<223>  I-CreI variant/HBB8.4 target

<400>  113

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Gln Leu Thr Phe Asn Val Thr Gln
        35                  40                  45

Lys Thr Gln Arg Arg Trp Phe Leu Asp Lys Leu Val Asp Glu Ile Gly
    50                  55                  60

Val Gly Tyr Val Glu Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  114
<211>  167
<212>  PRT
<213>  artificial sequence

<220>
<223>  I-CreI variant/HBB8.4 target

<400>  114

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Ser Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 115
<211> 167
<212> PRT
<213> artificial sequence

<220>
<223> I-CreI variant/HBB8.4 target

<400> 115

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Val Lys Phe Lys His His Leu Ser Leu Thr Phe Lys Val Thr Gln
        35                  40                  45

Lys Thr Gln Arg Arg Trp Phe Leu Asp Lys Leu Val Asp Glu Ile Gly

50                  55                  60

Val Gly Tyr Val Ser Asp Ser Gly Ser Val Ser Asn Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  116
<211>  167
<212>  PRT
<213>  artificial sequence

<220>
<223>  I-CreI variant/HBB8.4 target

<400>  116

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Val Lys Phe Lys His His Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Tyr Tyr Asn Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  117
<211>  167
<212>  PRT
<213>  artificial sequence

<220>
<223>  I-CreI variant/HBB8.4 target

<400>  117

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Val Lys Phe Lys His His Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  118
<211>  167
<212>  PRT
<213>  artificial sequence

<220>
<223>  I-CreI variant/HBB8.4 target

<400>  118

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  119
<211>  167

<212>  PRT
<213>  artificial sequence

<220>
<223>  I-CreI variant/HBB8.4 target

<400>  119

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Glu Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  120
<211>  167
<212>  PRT
<213>  artificial sequence

<220>
<223>  I-CreI variant/HBB8.4 target

<400>  120

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Asn Tyr Asp Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  121
<211>  167
<212>  PRT
<213>  artificial sequence

<220>
<223>  I-CreI variant/HBB8.4 target

<400>  121

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  122
<211>  167
<212>  PRT
<213>  artificial sequence

<220>
<223>  I-CreI variant/HBB8.4 target

<400>  122

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Thr Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Thr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln

            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 123
<211> 167
<212> PRT
<213> artificial sequence

<220>
<223> I-CreI variant/HBB target

<400> 123

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser Gly Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Asn Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  124
<211>  167
<212>  PRT
<213>  artificial sequence

<220>
<223>  I-CreI variant/HBB target

<400>  124

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asp Gln
            20                  25                  30

Ser Arg Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  125
<211>  167
<212>  PRT

<213>  artificial sequence

<220>
<223>  I-CreI variant/HBB target

<400>  125

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Asp Tyr Lys Phe Lys His Cys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Asp Tyr Lys Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  126
<211>  167
<212>  PRT
<213>  artificial sequence

<220>
<223>  I-CreI variant/HBB target

<400>  126

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  127
<211>  167
<212>  PRT
<213>  artificial sequence

<220>
<223>  I-CreI variant/HBB target

<400>  127

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Pro Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  128
<211>  167
<212>  PRT
<213>  artificial sequence

<220>
<223>  I-CreI variant/HBB target

<400>  128

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Ala Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp His Gly Ser Val Ser Asp Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  129
<211>  167
<212>  PRT
<213>  artificial sequence

<220>
<223>  I-CreI variant/HBB target

<400>  129

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Gly Thr Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Glu Asp Ser Gly Ser Val Ser Tyr Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys

145                 150                 155                 160

Lys Ser Ser Pro Ala Ala Asp
                165

<210>  130
<211>  167
<212>  PRT
<213>  artificial sequence

<220>
<223>  I-CreI variant/HBB target

<400>  130

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asp Gln
            20                  25                  30

Thr Tyr Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Tyr Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  131
<211>  167
<212>  PRT
<213>  artificial sequence

<220>
<223>  I-CreI variant/HBB target

<400>  131

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser Gly Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val His Asp His Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  132
<211>  167
<212>  PRT
<213>  artificial sequence

<220>
<223>  I-CreI variant/HBB target

<400>  132

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro His Gln

20                  25                  30

Ser Ser Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Tyr Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  133
<211>  167
<212>  PRT
<213>  artificial sequence

<220>
<223>  I-CreI variant/HBB target

<400>  133

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Trp Thr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp His Gly Ser Val Ser Asp Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  134
<211>  167
<212>  PRT
<213>  artificial sequence

<220>
<223>  I-CreI variant/HBB target

<400>  134

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Trp Leu Ser Leu Thr Phe Asn Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Arg Tyr Val Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  135
<211>  167
<212>  PRT
<213>  artificial sequence

<220>
<223>  I-CreI variant/HBB target

<400>  135

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Ser His Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  136
<211>  167
<212>  PRT
<213>  artificial sequence

<220>
<223>  I-CreI variant/HBB target

<400>  136

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Ala Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Tyr Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  137
<211>  167
<212>  PRT
<213>  artificial sequence

<220>
<223>  I-CreI variant/HBB target

<400>  137

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Gly Thr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Gly Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  138
<211>  167
<212>  PRT
<213>  artificial sequence

<220>
<223>  I-CreI variant/HBB target

<400>  138

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Asp Tyr Lys Phe Lys His Cys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  139
<211>  167
<212>  PRT
<213>  artificial sequence

<220>
<223>  I-CreI variant/HBB target

<400>  139

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Thr Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val His Asp His Gly Ser Val Ser Asn Tyr Ile Leu Ser

65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  140
<211>  167
<212>  PRT
<213>  artificial sequence

<220>
<223>  I-CreI variant/HBB target

<400>  140

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Lys Gln
            20                  25                  30

Ser Ser Lys Phe Lys His Gln Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Glu Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  141
<211>  167
<212>  PRT
<213>  artificial sequence

<220>
<223>  I-CreI variant/HBB target

<400>  141

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser His Lys Phe Lys His Ser Leu Ser Leu Thr Phe Tyr Val Thr Gln
        35                  40                  45

Lys Thr Gln Arg Arg Trp Phe Leu Asp Lys Leu Val Asp Glu Ile Gly
    50                  55                  60

Val Gly Tyr Val Asp Asp Ser Gly Ser Val Ser Arg Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  142
<211>  167
<212>  PRT
<213>  artificial sequence

<220>
<223>  I-CreI variant/HBB target

<400>  142

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro His Gln
            20                  25                  30

Ser His Lys Phe Lys His Gln Leu Ser Leu Thr Phe Pro Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp His Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  143
<211>  167
<212>  PRT
<213>  artificial sequence

<220>

<223>  I-CreI variant/HBB target

<400>  143

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Asp Tyr Lys Phe Lys His Cys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Arg Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  144
<211>  167
<212>  PRT
<213>  artificial sequence

<220>
<223>  I-CreI variant/HBB target

<400>  144

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Thr Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp His Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  145
<211>  167
<212>  PRT
<213>  artificial sequence

<220>
<223>  I-CreI variant/HBB target

<400>  145

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asp Gln
            20                  25                  30

Ser Arg Lys Phe Lys His Gln Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val His Asp His Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  146
<211>  167
<212>  PRT
<213>  artificial sequence

<220>
<223>  I-CreI variant/HBB target

<400>  146

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Gly Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr

115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  147
<211>  167
<212>  PRT
<213>  artificial sequence

<220>
<223>  I-CreI variant/HBB target

<400>  147

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Thr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  148
<211>  167
<212>  PRT
<213>  artificial sequence

<220>
<223>  I-CreI variant/HBB target

<400>  148

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Asn Gly Lys Phe Lys His Tyr Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Arg Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  149
<211>  626
<212>  DNA
<213>  Homo sapiens

<220>

<221>  CDS
<222>  (51)..(494)

<400>  149
acatttgctt ctgacacaac tgtgttcact agcaacctca aacagacacc atg gtg      56
cat ctg act cct gag gag aag tct gcc gtt act gcc ctg tgg ggc aag     104
gtg aac gtg gat gaa gtt ggt ggt gag gcc ctg ggc agg ctg ctg gtg     152
gtc tac cct tgg acc cag agg ttc ttt gag tcc ttt ggg gat ctg tcc     200
act cct gat gct gtt atg ggc aac cct aag gtg aag gct cat ggc aag     248
aaa gtg ctc ggt gcc ttt agt gat ggc ctg gct cac ctg gac aac ctc     296
aag ggc acc ttt gcc aca ctg agt gag ctg cac tgt gac aag ctg cac     344
gtg gat cct gag aac ttc agg ctc ctg ggc aac gtg ctg gtc tgt gtg     392
ctg gcc cat cac ttt ggc aaa gaa ttc acc cca cca gtg cag gct gcc     440
tat cag aaa gtg gtg gct ggt gtg gct aat gcc ctg gcc cac aag tat     488
cac taa gctcgctttc ttgctgtcca atttctatta aaggttcctt tgttccctaa      544
gtccaactac taaactgggg gatattatga agggccttga gcatctggat tctgcctaat   604
aaaaaacatt tattttcatt gc                                            626


<210>  150
<211>  147
<212>  PRT
<213>  Homo sapiens

<400>  150

<210>  151
<211>  33
<212>  DNA
<213>  artificial sequence

<220>
<223>  G19SF primer

<400>  151
gccggctttg tggactctga cggtagcatc atc                                33

<210>  152
<211>  33
<212>  DNA
<213>  artificial sequence

<220>
<223>  G19SR primer

<400>  152
gatgatgcta ccgtcagagt ccacaaagcc ggc                                33

<210>  153
<211>  33
<212>  DNA
<213>  artificial sequence

<220>

<223>  F54LF primer

<400>  153
acccagcgcc gttggctgct ggacaaacta gtg                                    33


<210>  154
<211>  33
<212>  DNA
<213>  artificial sequence

<220>
<223>  F54LR primer

<400>  154
cactagtttg tccagcagcc aacggcgctg ggt                                    33


<210>  155
<211>  33
<212>  DNA
<213>  artificial sequence

<220>
<223>  E80KF primer

<400>  155
ttaagcaaaa tcaagccgct gcacaacttc ctg                                    33


<210>  156
<211>  33
<212>  DNA
<213>  artificial sequence

<220>
<223>  E80KR primer

<400>  156
caggaagttg tgcagcggct tgattttgct taa                                    33


<210>  157
<211>  33
<212>  DNA
<213>  artificial sequence

<220>
<223>  F87LF primer

<400>  157
aagccgctgc acaacctgct gactcaactg cag                                    33


<210>  158
<211>  33
<212>  DNA
<213>  artificial sequence

<220>
<223>  F87LR primer

<400>  158
ctgcagttga gtcagcaggt tgtgcagcgg ctt                                    33

<210>  159
<211>  33
<212>  DNA
<213>  artificial sequence

<220>
<223>  V105AF primer

<400>  159
aaacaggcaa acctggctct gaaaattatc gaa                                    33


<210>  160
<211>  33
<212>  DNA
<213>  artificial sequence

<220>
<223>  V105AR primer

<400>  160
ttcgataatt ttcagagcca ggtttgcctg ttt                                    33


<210>  161
<211>  33
<212>  DNA
<213>  artificial sequence

<220>
<223>  I132VF primer

<400>  161
acctgggtgg atcaggttgc agctctgaac gat                                    33


<210>  162
<211>  33
<212>  DNA
<213>  artificial sequence

<220>
<223>  I132VR primer

<400>  162
atcgttcaga gctgcaacct gatccaccca ggt                                    33


<210>  163
<211>  167
<212>  PRT
<213>  artificial sequence

<220>
<223>  I-CreI variant/HBB5.3 target

<400>  163

Met Ala Asn Thr Lys Tyr Asn Lys Glu Phe Leu Leu Tyr Leu Ala Gly
1               5                   10                  15


Phe Val Asp Gly Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Asn Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Thr Asp
                165

<210>  164
<211>  167
<212>  PRT
<213>  artificial sequence

<220>
<223>  I-CreI variant/HBB5.3 target

<400>  164

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser

65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  165
<211>  167
<212>  PRT
<213>  artificial sequence

<220>
<223>  I-CreI variant/HBB5.3 target

<400>  165

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Gly Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  166
<211>  167
<212>  PRT
<213>  artificial sequence

<220>
<223>  I-CreI variant/HBB5.3 target

<400>  166

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Ser Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Glu
145                 150                 155                 160

Glu Ser Ser Pro Ala Ala Asp
                165

<210>  167
<211>  167
<212>  PRT
<213>  artificial sequence

<220>
<223>  I-CreI variant/HBB5.3 target

<400>  167

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Pro Pro Ala Ala Asp
                165

<210>  168
<211>  167
<212>  PRT
<213>  artificial sequence

<220>

<223>  I-CreI variant/HBB5.3 target

<400>  168

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Arg Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Met Leu Asp Ser Leu Ser Glu Lys Lys
145                 150                 155                 160

Lys Ser Ser Pro Ala Ala Asp
                165


<210>  169
<211>  167
<212>  PRT
<213>  artificial sequence

<220>
<223>  I-CreI variant/HBB5.3 target

<400>  169

Met Ala Asn Thr Glu Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Ala Arg Lys Thr
    130                 135                 140

Thr Ser Glu Thr Val Arg Ala Val Leu Asp Ser Leu Ser Glu Lys Lys
145                 150                 155                 160

Lys Ser Ser Ser Ala Ala Asp
                165

<210>  170
<211>  167
<212>  PRT
<213>  artificial sequence

<220>
<223>  I-CreI variant/HBB5.3 target

<400>  170

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly His Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Leu Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Arg
145                 150                 155                 160

Lys Ser Ser Pro Ala Ala Asp
                165

<210>  171
<211>  167
<212>  PRT
<213>  artificial sequence

<220>
<223>  I-CreI variant/HBB5.3 target

<400>  171

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu His
            100                 105                 110

Leu Pro Ser Ala Lys Glu Ser Pro Asp Lys Phe Leu Glu Val Cys Thr

115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  172
<211>  167
<212>  PRT
<213>  artificial sequence

<220>
<223>  I-CreI variant/HBB5.3 target

<400>  172

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  173
<211>  167
<212>  PRT
<213>  artificial sequence

<220>
<223>  I-CreI variant/HBB5.3 target

<400>  173

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Glu Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  174
<211>  167
<212>  PRT
<213>  artificial sequence

<220>
<223>  I-CreI variant/HBB5.3 target

<400>  174

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  175
<211>  167
<212>  PRT
<213>  artificial sequence

<220>
<223>  I-CreI variant/HBB5.3 target

<400>  175

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  176
<211>  167
<212>  PRT
<213>  artificial sequence

<220>
<223>  I-CreI variant/HBB5.3 target

<400>  176

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  177
<211>  167
<212>  PRT
<213>  artificial sequence

<220>
<223>  I-CreI variant/HBB5.3 target

<400>  177

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  178
<211>  163
<212>  PRT
<213>  Chlamydomonas reinhardtii

<400>  178

Met Asn Thr Lys Tyr Asn Lys Glu Phe Leu Leu Tyr Leu Ala Gly Phe
1               5                   10                  15

Val Asp Gly Asp Gly Ser Ile Ile Ala Gln Ile Lys Pro Asn Gln Ser
            20                  25                  30

Tyr Lys Phe Lys His Gln Leu Ser Leu Thr Phe Gln Val Thr Gln Lys
        35                  40                  45

Thr Gln Arg Arg Trp Phe Leu Asp Lys Leu Val Asp Glu Ile Gly Val
    50                  55                  60

Gly Tyr Val Arg Asp Arg Gly Ser Val Ser Asp Tyr Ile Leu Ser Glu
65                  70                  75                  80

Ile Lys Pro Leu His Asn Phe Leu Thr Gln Leu Gln Pro Phe Leu Lys
                85                  90                  95

Leu Lys Gln Lys Gln Ala Asn Leu Val Leu Lys Ile Ile Glu Gln Leu
            100                 105                 110

Pro Ser Ala Lys Glu Ser Pro Asp Lys Phe Leu Glu Val Cys Thr Trp
        115                 120                 125

Val Asp Gln Ile Ala Ala Leu Asn Asp Ser Lys Thr Arg Lys Thr Thr
    130                 135                 140

Ser Glu Thr Val Arg Ala Val Leu Asp Ser Leu Ser Glu Lys Lys Lys
145                 150                 155                 160

Ser Ser Pro

<210>  179
<211>  167
<212>  PRT
<213>  artificial sequence

<220>
<223>  optimized I-CreI variant/HBB5.4 target

<400>  179

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Ser Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  180
<211>  167
<212>  PRT
<213>  artificial sequence

<220>
<223>  optimized I-CreI variant/HBB5.4 target

<400>  180

Met Ala Asn Thr Lys Tyr Asn Lys Glu Phe Leu Leu Tyr Leu Ala Gly

1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  181
<211>  167
<212>  PRT
<213>  artificial sequence

<220>
<223>  optimized I-CreI variant/HBB5.4 target

<400>  181

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  182
<211>  167
<212>  PRT
<213>  artificial sequence

<220>
<223>  optimized I-CreI variant/HBB5.4 target

<400>  182

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  183
<211>  167
<212>  PRT
<213>  artificial sequence

<220>
<223>  optimized I-CreI variant/HBB5.4 target

<400>  183

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Asp Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  184
<211>  167
<212>  PRT
<213>  artificial sequence

<220>
<223>  optimized I-CreI variant/HBB5.4 target

<400>  184

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Pro Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  185

<211>  167
<212>  PRT
<213>  artificial sequence

<220>
<223>  optimized I-CreI variant/HBB5.4 target

<400>  185

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  186
<211>  167
<212>  PRT
<213>  artificial sequence

<220>
<223>  optimized I-CreI variant/HBB5.4 target

<400>  186

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  187
<211>  33
<212>  DNA
<213>  artificial sequence

<220>
<223>  G19wtF primer

<400>  187
gccggctttg tggacggtga cggtagcatc atc                                33

<210>  188
<211>  33
<212>  DNA
<213>  artificial sequence

<220>
<223>  F54wtR primer

<400>  188
cactagtttg tccagaaacc aacggcgctg ggt                                33

<210>  189
<211>  33
<212>  DNA
<213>  artificial sequence

<220>
<223>  G19wtR primer

<400>  189
gatgatgcta ccgtcaccgt ccacaaagcc ggc                                    33


<210>  190
<211>  33
<212>  DNA
<213>  artificial sequence

<220>
<223>  F54wtF primer

<400>  190
acccagcgcc gttggtttct ggacaaacta gtg                                    33


<210>  191
<211>  33
<212>  DNA
<213>  artificial sequence

<220>
<223>  E80wtR primer

<400>  191
caggaagttg tgcagcggct tgatttcgct taa                                    33


<210>  192
<211>  33
<212>  DNA
<213>  artificial sequence

<220>
<223>  E80wtF primer

<400>  192
ttaagcgaaa tcaagccgct gcacaacttc ctg                                    33


<210>  193
<211>  33
<212>  DNA
<213>  artificial sequence

<220>
<223>  V105wtR primer

<400>  193
ttcgataatt ttcagaacca ggtttgcctg ttt                                    33


<210>  194
<211>  33
<212>  DNA
<213>  artificial sequence

<220>
<223>  V105wtF primer

<400>  194
aaacaggcaa acctggttct gaaaattatc gaa                                    33


<210>  195
<211>  33
<212>  DNA
<213>  artificial sequence

<220>
<223>  I132wtR primer

<400>  195
atcgttcaga gctgcaatct gatccaccca ggt                                    33


<210>  196
<211>  33
<212>  DNA
<213>  artificial sequence

<220>
<223>  I132wtF primer

<400>  196
acctgggtgg atcagattgc agctctgaac gat                                    33


<210>  197
<211>  167
<212>  PRT
<213>  artificial sequence

<220>
<223>  optimized I-CreI variant/HBB5.3 target

<400>  197

Met Ala Asn Thr Lys Tyr Asn Lys Glu Phe Leu Leu Tyr Leu Ala Gly
1               5                   10                  15


Phe Val Asp Gly Asp Gly Ser Ile Ile Ala Gln Ile Lys Pro Ser Gln
            20                  25                  30


Ser His Lys Phe Lys His Arg Leu Ser Leu Thr Phe Lys Val Thr Gln
        35                  40                  45


Lys Thr Gln Arg Arg Trp Phe Leu Asp Lys Leu Val Asp Glu Ile Gly
    50                  55                  60


Val Gly Tyr Val Tyr Asp Ser Gly Ser Val Ser Asp Tyr Thr Leu Ser
65                  70                  75                  80


Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 198
<211> 167
<212> PRT
<213> artificial sequence

<220>
<223> optimized I-CreI variant/HBB5.3 target

<400> 198

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Pro His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Thr Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Glu
145                 150                 155                 160

Lys Ser Ser Pro Ala Ala Asp
                165

<210>  199
<211>  167
<212>  PRT
<213>  artificial sequence

<220>
<223>  optimized I-CreI variant/HBB5.3 target

<400>  199

Met Ala Asn Thr Lys Tyr Asn Arg Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly His Val Tyr Asp Ser Gly Ser Val Ser Asp Tyr Thr Leu Ser
65                  70                  75                  80

Glu Ile Arg Pro Leu His Ser Phe Leu Thr Gln Leu Gln Pro Phe Leu
                85                  90                  95

Lys Leu Lys Arg Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Arg Ala Arg Lys Thr
    130                 135                 140

Thr Ser Glu Thr Val Arg Ala Val Leu Asp Ser Leu Ser Glu Lys Arg
145                 150                 155                 160

Lys Ser Ser Pro Ala Ala Asp
                165

<210>  200

<211>  167
<212>  PRT
<213>  artificial sequence

<220>
<223>  optimized I-CreI variant/HBB5.3 target

<400>  200

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  201
<211>  167
<212>  PRT
<213>  artificial sequence

<220>
<223>  optimized I-CreI variant/HBB5.3 target

<400>  201

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Ser Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Glu
145                 150                 155                 160

Glu Ser Ser Pro Ala Ala Asp
                165

<210>  202
<211>  167
<212>  PRT
<213>  artificial sequence

<220>
<223>  optimized I-CreI variant/HBB5.3 target

<400>  202

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Gly Glu Ile Gly

50                  55                  60

Val Gly Tyr Val Tyr Asp Ser Gly Ser Val Ser Asn Tyr Gln Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Ser Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  203
<211>  167
<212>  PRT
<213>  artificial sequence

<220>
<223>  optimized I-CreI variant/HBB5.3 target

<400>  203

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Ser Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Lys Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Ala Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  204
<211>  167
<212>  PRT
<213>  artificial sequence

<220>
<223>  optimized I-CreI variant/HBB5.3 target

<400>  204

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Glu
145                 150                 155                 160

Glu Ser Ser Pro Ala Ala Asp
                165

<210>  205
<211>  167
<212>  PRT
<213>  artificial sequence

<220>
<223>  optimized I-CreI variant/HBB5.3 target

<400>  205

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Arg
145                 150                 155                 160

Lys Ser Ser Pro Ala Ala Asp
                165

<210>  206
<211>  167

<212>  PRT
<213>  artificial sequence

<220>
<223>  optimized I-CreI variant/HBB5.3 target

<400>  206

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Glu
145                 150                 155                 160

Glu Ser Ser Pro Ala Ala Asp
                165


<210>  207
<211>  167
<212>  PRT
<213>  artificial sequence

<220>
<223>  optimized I-CreI variant/HBB5.3 target

<400>  207

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  208
<211>  167
<212>  PRT
<213>  artificial sequence

<220>
<223>  optimized I-CreI variant/HBB5.3 target

<400>  208

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly His Val Tyr Asp Ser Gly Ser Val Ser Asp Tyr Thr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Glu
145                 150                 155                 160

Glu Ser Ser Pro Ala Ala Asp
                165

<210>  209
<211>  167
<212>  PRT
<213>  artificial sequence

<220>
<223>  optimized I-CreI variant/HBB5.4 target

<400>  209

Met Ala Asn Thr Lys Tyr Asn Lys Glu Phe Leu Leu Tyr Leu Ala Gly
1               5                   10                  15

Phe Val Asp Ser Asp Gly Ser Ile Val Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln

                100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Glu Ser Ser Pro Ala Ala Asp
                165

<210>  210
<211>  167
<212>  PRT
<213>  artificial sequence

<220>
<223>  optimized I-CreI variant/HBB5.4 target

<400>  210

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Ser Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Arg Leu Lys Gln Arg Gln Ala Asn Leu Ile Leu Lys Ile Val Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Pro Ser Pro Ala Ala Asp
                165

<210>  211
<211>  167
<212>  PRT
<213>  artificial sequence

<220>
<223>  optimized I-CreI variant/HBB5.4 target

<400>  211

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Ser Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Glu Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Arg
145                 150                 155                 160

Lys Ser Ser Pro Ala Ala Asp
                165

<210>  212
<211>  167
<212>  PRT

<213>  artificial sequence

<220>
<223>  optimized I-CreI variant/HBB5.4 target

<400>  212

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Ser Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  213
<211>  167
<212>  PRT
<213>  artificial sequence

<220>
<223>  optimized I-CreI variant/HBB5.4 target

<400>  213

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Arg Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 214
<211> 167
<212> PRT
<213> artificial sequence

<220>
<223> optimized I-CreI variant/HBB5.4 target

<400> 214

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu Arg Asn Leu Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Arg Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  215
<211>  167
<212>  PRT
<213>  artificial sequence

<220>
<223>  optimized I-CreI variant/HBB5.4 target

<400>  215

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  216
<211>  167
<212>  PRT
<213>  artificial sequence

<220>
<223>  optimized I-CreI variant/HBB5.4 target

<400>  216

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys

145                 150                 155                 160

Lys Ser Ser Pro Ala Ala Asp
                165

<210>  217
<211>  167
<212>  PRT
<213>  artificial sequence

<220>
<223>  optimized I-CreI variant/HBB5.4 target

<400>  217

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  218
<211>  167
<212>  PRT
<213>  artificial sequence

<220>
<223>  optimized I-CreI variant/HBB5.4 target

<400>  218

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Ser Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  219
<211>  167
<212>  PRT
<213>  artificial sequence

<220>
<223>  optimized I-CreI variant/HBB5.4 target

<400>  219

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln

            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 220
<211> 167
<212> PRT
<213> artificial sequence

<220>
<223> optimized I-CreI variant/HBB5.4 target

<400> 220

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Arg Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  221
<211>  77
<212>  DNA
<213>  artificial sequence

<220>
<223>  attB1-ICreIFor primer

<400>  221
ggggacaagt ttgtacaaaa aagcaggctt cgaaggagat agaaccatgg ccaataccaa      60

atataacaaa gagttcc                                                     77

<210>  222
<211>  64
<212>  DNA
<213>  artificial sequence

<220>
<223>  attB2-ICreIRev primer

<400>  222
ggggaccact ttgtacaaga aagctgggtt tagtcggccg ccggggagga tttcttcttc      60

tcgc                                                                   64

<210>  223
<211>  167
<212>  PRT
<213>  artificial sequence

<220>
<223>  I-CreI variant/HBB8.3 target

<400>  223

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  224
<211>  167
<212>  PRT
<213>  artificial sequence

<220>
<223>  I-CreI variant/HBB8.3 target

<400>  224

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Asn Asp Ser Gly Ser Val Ser Asn Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  225
<211>  167
<212>  PRT
<213>  artificial sequence

<220>
<223>  I-CreI variant/HBB8.3 target

<400>  225

Met Ala Asn Thr Lys Tyr Asn Lys Glu Phe Leu Leu His Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu

85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  226
<211>  167
<212>  PRT
<213>  artificial sequence

<220>
<223>  I-CreI variant/HBB8.3 target

<400>  226

Met Ala Asn Thr Glu Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  227
<211>  167
<212>  PRT
<213>  artificial sequence

<220>
<223>  I-CreI variant/HBB8.3 target

<400>  227

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  228
<211>  167
<212>  PRT
<213>  artificial sequence

<220>
<223>  I-CreI variant/HBB8.3 target

<400>  228

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Asn Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  229
<211>  167
<212>  PRT
<213>  artificial sequence

<220>
<223>  I-CreI variant/HBB8.3 target

<400>  229

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  230
<211>  167
<212>  PRT
<213>  artificial sequence

<220>
<223>  I-CreI variant/HBB8.3 target

<400>  230

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Asn Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  231
<211>  167
<212>  PRT
<213>  artificial sequence

<220>
<223>  optimized I-CreI variant/HBB8.3 target

<400>  231

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Leu Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  232
<211>  167
<212>  PRT
<213>  artificial sequence

<220>
<223>  optimized I-CreI variant/HBB8.3 target

<400>  232

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Leu Leu Asp Lys Leu Val Asp Glu Ile Gly
    50                  55                  60

Val Gly Tyr Val Tyr Asp Ser Gly Ser Val Ser Asn Tyr Leu Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr

130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  233
<211>  167
<212>  PRT
<213>  artificial sequence

<220>
<223>  optimized I-CreI variant/HBB8.3 target

<400>  233

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Leu Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  234
<211>  167
<212>  PRT
<213>  artificial sequence

<220>
<223>  optimized I-CreI variant/HBB8.3 target

<400>  234

Met Ala Asn Thr Glu Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Leu Leu Asp Lys Leu Val Asp Glu Ile Gly
    50                  55                  60

Val Gly Tyr Val Tyr Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  235
<211>  167
<212>  PRT
<213>  artificial sequence

<220>
<223>  optimized I-CreI variant/HBB8.3 target

<400>  235

Met Ala Asn Thr Lys Tyr Asn Lys Glu Phe Leu Leu Tyr Leu Ala Gly

1               5                   10                  15

Phe Val Asp Gly Asp Gly Ser Ile Ile Ala Gln Ile Lys Pro Thr Gln
            20                  25                  30

Ser Arg Lys Phe Lys His Gln Leu Ser Leu Thr Phe Lys Val Thr Gln
        35                  40                  45

Lys Thr Gln Arg Arg Trp Leu Leu Asp Lys Leu Val Asp Glu Ile Gly
    50                  55                  60

Val Gly Tyr Val Ser Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  236
<211>  167
<212>  PRT
<213>  artificial sequence

<220>
<223>  optimized I-CreI variant/HBB8.3 target

<400>  236

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Leu Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  237
<211>  167
<212>  PRT
<213>  artificial sequence

<220>
<223>  optimized I-CreI variant/HBB8.3 target

<400>  237

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  238
<211>  167
<212>  PRT
<213>  artificial sequence

<220>
<223>  optimized I-CreI variant/HBB8.3 target

<400>  238

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Leu Leu Thr Gln Leu Gln Ala Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  239
<211>  167
<212>  PRT
<213>  artificial sequence

<220>
<223>  optimized I-CreI variant/HBB8.3 target

<400>  239

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Arg Leu Ser
65                  70                  75                  80

Glu Ile Lys Pro Leu Arg Asn Phe Leu Thr Gln Leu Gln Pro Phe Leu
                85                  90                  95

Lys Leu Lys Arg Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  240

<211>  167
<212>  PRT
<213>  artificial sequence

<220>
<223>  optimized I-CreI variant/HBB8.3 target

<400>  240

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  241
<211>  167
<212>  PRT
<213>  artificial sequence

<220>
<223>  optimized I-CreI variant/HBB8.3 target

<400>  241

Met Ala Asn Thr Lys Tyr Asn Lys Glu Phe Leu Leu His Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  242
<211>  167
<212>  PRT
<213>  artificial sequence

<220>
<223>  optimized I-CreI variant/HBB8.3 target

<400>  242

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly

50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  243
<211>  167
<212>  PRT
<213>  artificial sequence

<220>
<223>  optimized I-CreI variant/HBB8.3 target

<400>  243

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Gln Val Thr Gln
        35                  40                  45

Lys Thr Gln Arg Arg Trp Phe Leu Asp Lys Leu Val Asn Glu Ile Gly
    50                  55                  60

Val Gly Tyr Val Tyr Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  244
<211>  167
<212>  PRT
<213>  artificial sequence

<220>
<223>  optimized I-CreI variant/HBB8.3 target

<400>  244

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  245
<211>  167
<212>  PRT
<213>  artificial sequence

<220>
<223>  optimized I-CreI variant/HBB8.3 target

<400>  245

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  246
<211>  167

<212>  PRT
<213>  artificial sequence

<220>
<223>  optimized I-CreI variant/HBB8.3 target

<400>  246

Met Ala Asn Thr Lys Tyr Asn Lys Glu Phe Leu Leu His Leu Ala Gly
1               5                   10                  15

Phe Val Asp Ser Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  247
<211>  167
<212>  PRT
<213>  artificial sequence

<220>
<223>  optimized I-CreI variant/HBB8.3 target

<400>  247

Met Ala Asn Thr Glu Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  248
<211>  167
<212>  PRT
<213>  artificial sequence

<220>
<223>  optimized I-CreI variant/HBB8.3 target

<400>  248

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Ile Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  249
<211>  167
<212>  PRT
<213>  artificial sequence

<220>
<223>  optimized I-CreI variant/HBB8.3 target

<400>  249

Met Ala Asn Thr Lys Tyr Asn Lys Glu Phe Leu Leu His Leu Ala Gly
1               5                   10                  15

Phe Val Asp Ser Asp Gly Ser Ile Ile Ala Gln Ile Lys Pro Asn Gln
            20                  25                  30

Ser Gly Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln

            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210> 250
<211> 167
<212> PRT
<213> artificial sequence

<220>
<223> optimized I-CreI variant/HBB8.3 target

<400> 250

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  251
<211>  167
<212>  PRT
<213>  artificial sequence

<220>
<223>  optimized I-CreI variant/HBB8.3 target

<400>  251

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Gln Gln
            20                  25                  30

Thr Tyr Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  252
<211>  167
<212>  PRT

<213>  artificial sequence

<220>
<223>  optimized I-CreI variant/HBB8.3 target

<400>  252

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  253
<211>  167
<212>  PRT
<213>  artificial sequence

<220>
<223>  optimized I-CreI variant/HBB8.3 target

<400>  253

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  254
<211>  167
<212>  PRT
<213>  artificial sequence

<220>
<223>  optimized I-CreI variant/HBB8.3 target

<400>  254

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Ala Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  255
<211>  167
<212>  PRT
<213>  artificial sequence

<220>
<223>  optimized I-CreI variant/HBB8.3 target

<400>  255

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Gln Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Asn Tyr Arg Leu Ser
65                  70                  75                  80

Lys Ile Lys Pro Leu His Asn Ile Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  256
<211>  167
<212>  PRT
<213>  artificial sequence

<220>
<223>  I-CreI variant/HBB8.4 target

<400>  256

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His His Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Arg Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys

145                 150                 155                 160

Lys Ser Ser Pro Ala Ala Asp
                165

<210>  257
<211>  167
<212>  PRT
<213>  artificial sequence

<220>
<223>  optimized I-CreI variant/HBB8.4 target

<400>  257

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Gln Leu Thr Phe Asp Val Thr Gln
        35                  40                  45

Lys Thr Gln Arg Arg Trp Leu Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  258
<211>  167
<212>  PRT
<213>  artificial sequence

<220>
<223>  optimized I-CreI variant/HBB8.4 target

<400>  258

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Arg Leu Ser Leu Thr Phe Asp Val Thr Gln
        35                  40                  45

Lys Thr Gln Arg Arg Trp Leu Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  259
<211>  167
<212>  PRT
<213>  artificial sequence

<220>
<223>  optimized I-CreI variant/HBB8.4 target

<400>  259

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln

20                  25                  30

Ser Thr Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  260
<211>  167
<212>  PRT
<213>  artificial sequence

<220>
<223>  optimized I-CreI variant/HBB8.4 target

<400>  260

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Arg Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  261
<211>  167
<212>  PRT
<213>  artificial sequence

<220>
<223>  optimized I-CreI variant/HBB8.4 target

<400>  261

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Val Lys Phe Lys His His Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  262
<211>  167
<212>  PRT
<213>  artificial sequence

<220>
<223>  optimized I-CreI variant/HBB8.4 target

<400>  262

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  263
<211>  167
<212>  PRT
<213>  artificial sequence

<220>
<223>  optimized I-CreI variant/HBB8.4 target

<400>  263

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Arg Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  264
<211>  167
<212>  PRT
<213>  artificial sequence

<220>
<223>  optimized I-CreI variant/HBB8.4 target

<400>  264

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  265
<211>  167
<212>  PRT
<213>  artificial sequence

<220>
<223>  optimized I-CreI variant/HBB8.4 target

<400>  265

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Thr Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  266
<211>  167
<212>  PRT
<213>  artificial sequence

<220>
<223>  optimized I-CreI variant/HBB8.4 target

<400>  266

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Thr Cys Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser

65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  267
<211>  167
<212>  PRT
<213>  artificial sequence

<220>
<223>  optimized I-CreI variant/HBB8.4 target

<400>  267

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Ser Lys Phe Lys His Arg Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  268
<211>  167
<212>  PRT
<213>  artificial sequence

<220>
<223>  optimized I-CreI variant/HBB8.4 target

<400>  268

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Asn Gln
            20                  25                  30

Ser Cys Lys Phe Lys His His Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  269
<211>  167
<212>  PRT
<213>  artificial sequence

<220>
<223>  optimized I-CreI variant/HBB8.4 target

<400>  269

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Thr Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  270
<211>  167
<212>  PRT
<213>  artificial sequence

<220>

<223>  optimized I-CreI variant/HBB8.4 target

<400>  270

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Cys Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Arg Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  271
<211>  167
<212>  PRT
<213>  artificial sequence

<220>
<223>  optimized I-CreI variant/HBB8.4 target

<400>  271

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Cys Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  272
<211>  167
<212>  PRT
<213>  artificial sequence

<220>
<223>  optimized I-CreI variant/HBB8.4 target

<400>  272

Met Ala Asn Thr Lys Tyr Asn Arg Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Cys Lys Phe Lys His His Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  273
<211>  167
<212>  PRT
<213>  artificial sequence

<220>
<223>  optimized I-CreI variant/HBB8.4 target

<400>  273

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Val Lys Phe Lys His Arg Leu Gln Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr

115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  274
<211>  167
<212>  PRT
<213>  artificial sequence

<220>
<223>  optimized I-CreI variant/HBB8.4 target

<400>  274

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Val Lys Phe Lys His His Leu Ser Leu Thr Phe Arg Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  275
<211>  167
<212>  PRT
<213>  artificial sequence

<220>
<223>  optimized I-CreI variant/HBB8.4 target

<400>  275

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Cys Lys Phe Lys His Gln Leu Ser Leu Thr Phe Asp Val Thr Gln
        35                  40                  45

Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  276
<211>  167
<212>  PRT
<213>  artificial sequence

<220>
<223>  optimized I-CreI variant/HBB8.4 target

<400>  276

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Cys Lys Phe Lys His Gln Leu Ser Leu Thr Phe His Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  277
<211>  167
<212>  PRT
<213>  artificial sequence

<220>
<223>  optimized I-CreI variant/HBB5.3 target

<400>  277

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Pro His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Thr Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  278
<211>  167
<212>  PRT
<213>  artificial sequence

<220>
<223>  optimized I-CreI variant/HBB5.3 target

<400>  278

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Pro His Arg Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser
65                  70                  75                  80

Glu Thr Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  279
<211>  167
<212>  PRT
<213>  artificial sequence

<220>
<223>  optimized I-CreI variant/HBB5.3 target

<400>  279

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly His Val Tyr Asp Ser Gly Ser Val Ser Asp Tyr Thr Leu Ser
65                  70                  75                  80

Glu Ile Arg Pro Leu His Ser Phe Leu Thr Gln Leu Gln Pro Phe Leu
                85                  90                  95

Lys Leu Lys Arg Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Arg Ala Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  280
<211>  167
<212>  PRT
<213>  artificial sequence

<220>
<223>  optimized I-CreI variant/HBB5.3 target

<400>  280

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Thr Gln
            20                  25                  30

Ser His Lys Phe Lys His Arg Leu Ser Leu Thr Phe Lys Val Thr Gln
        35                  40                  45

Lys Ala Gln Arg Arg Trp Phe Leu Asp Lys Leu Val Asp Glu Ile Gly
    50                  55                  60

Val Gly His Val Tyr Asp Ser Gly Ser Val Ser Asp Tyr Thr Leu Ser
65                  70                  75                  80

Glu Ile Arg Pro Leu His Ser Phe Leu Thr Gln Leu Gln Pro Phe Leu
                85                  90                  95

Lys Leu Lys Arg Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Arg Ala Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp

                165

<210>  281
<211>  167
<212>  PRT
<213>  artificial sequence

<220>
<223>  optimized I-CreI variant/HBB5.4 target

<400>  281

Met Ala Asn Thr Lys Tyr Asn Lys Glu Phe Leu Leu Tyr Leu Ala Gly
1               5                   10                  15

Phe Val Asp Ser Asp Gly Ser Ile Val Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  282
<211>  22
<212>  DNA
<213>  artificial sequence

<220>
<223>  HBB8.5 target

<400>  282
cagacttctc cacagaagtc tg                                                22


<210>  283
<211>  22
<212>  DNA
<213>  artificial sequence

<220>
<223>  HBB8.6 target

<400>  283
ctgactcctc cacaggagtc ag                                                22


<210>  284
<211>  22
<212>  DNA
<213>  artificial sequence

<220>
<223>  HBB6.5 target

<400>  284
cagacttctc ctcagaagtc tg                                                22


<210>  285
<211>  22
<212>  DNA
<213>  artificial sequence

<220>
<223>  HBB6.6 target

<400>  285
ctgactcctc ctcaggagtc ag                                                22


<210>  286
<211>  167
<212>  PRT
<213>  artificial sequence

<220>
<223>  optimized I-CreI variant/HBB8.4 target

<400>  286

Met Ala Asn Thr Lys Tyr Asn Lys Glu Phe Leu Leu Tyr Leu Ala Gly
1               5                   10                  15


Phe Val Asp Gly Asp Gly Ser Ile Ile Ala Gln Ile Lys Pro Asn Gln
            20                  25                  30


Ser Val Lys Phe Lys His His Leu Gln Leu Thr Phe Asp Val Thr Gln
        35                  40                  45


Lys Thr Gln Arg Arg Trp Phe Leu Asp Lys Leu Val Asp Glu Ile Gly
    50                  55                  60

Val Gly Tyr Val Ala Asp Ser Gly Ser Val Ser Lys Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Arg Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  287
<211>  167
<212>  PRT
<213>  artificial sequence

<220>
<223>  optimized I-CreI variant/HBB8.3 target

<400>  287

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  288
<211>  167
<212>  PRT
<213>  artificial sequence

<220>
<223>  optimized I-CreI variant/HBB8.3 target

<400>  288

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Ile Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys

145                 150                 155                 160

Lys Ser Ser Pro Ala Ala Asp
                165

<210>  289
<211>  167
<212>  PRT
<213>  artificial sequence

<220>
<223>  optimized I-CreI variant/HBB8.3 target

<400>  289

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Gly Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Gly Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  290
<211>  167
<212>  PRT
<213>  artificial sequence

<220>
<223>  optimized I-CreI variant/HBB8.3 target

<400>  290

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln
            20                  25                  30

Thr Tyr Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Tyr Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Gln Asp Ser Leu Ser Glu Lys Lys
145                 150                 155                 160

Lys Ser Ser Pro Ala Ala Asp
                165


<210>  291
<211>  167
<212>  PRT
<213>  artificial sequence

<220>
<223>  optimized I-CreI variant/HBB8.3 target

<400>  291

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Gln Gln

20                  25                  30

Thr Tyr Lys Phe Lys His Arg Leu Ser Leu Thr Phe Gln Val Thr Gln
        35                  40                  45

Lys Thr Gln Arg Arg Trp Phe Leu Asp Lys Gln Val Asp Glu Ile Gly
    50                  55                  60

Val Gly Tyr Val Ala Asp Ser Gly Ser Val Ser Asn Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  292
<211>  167
<212>  PRT
<213>  artificial sequence

<220>
<223>  optimized I-CreI variant/HBB5.4 target

<400>  292

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Arg Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  293
<211>  167
<212>  PRT
<213>  artificial sequence

<220>
<223>  optimized I-CreI variant/HBB5.4 target

<400>  293

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Thr Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Arg Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Pro Ser Glu Lys Lys
145                 150                 155                 160

Lys Ser Ser Pro Ala Ala Asp
                165

<210>  294
<211>  167
<212>  PRT
<213>  artificial sequence

<220>
<223>  optimized I-CreI variant/HBB5.4 target

<400>  294

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Arg Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Leu Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  295
<211>  167
<212>  PRT
<213>  artificial sequence

<220>
<223>  optimized I-CreI variant/HBB5.4 target

<400>  295

Met Ala Asn Thr Lys Tyr Asn Lys Glu Phe Leu Leu Tyr Leu Ala Gly
1               5                   10                  15

Phe Val Asp Gly Asp Gly Ser Ile Val Ala Gln Ile Lys Pro Asn Gln
            20                  25                  30

Ser Tyr Lys Phe Lys His Gln Leu Ser Leu Thr Leu Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  296
<211>  167
<212>  PRT
<213>  artificial sequence

<220>
<223>  optimized I-CreI variant/HBB5.4 target

<400>  296

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Thr Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165


<210>  297
<211>  167
<212>  PRT
<213>  artificial sequence

<220>
<223>  optimized I-CreI variant/HBB5.4 target

<400>  297

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Asp Tyr Lys Phe Lys His Gln Leu Ser Leu Thr Phe Ala Val Thr Gln
        35                  40                  45

Lys Thr Gln Arg Arg Trp Phe Leu Asp Lys Leu Val Asp Glu Ile Gly
    50                  55                  60

Val Gly Tyr Val Arg Asp Ser Gly Ser Val Ser Glu Tyr Arg Leu Ser
65                  70                  75                  80

Lys Ile Lys Pro Leu His Asn Phe Leu Thr Gln Leu Gln Pro Phe Leu
                85                  90                  95

Arg Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  298
<211>  167
<212>  PRT
<213>  artificial sequence

<220>
<223>  optimized I-CreI variant/HBB5.3 target

<400>  298

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Thr Leu Ser

65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  299
<211>  167
<212>  PRT
<213>  artificial sequence

<220>
<223>  optimized I-CreI variant/HBB5.3 target

<400>  299

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  300
<211>  167
<212>  PRT
<213>  artificial sequence

<220>
<223>  optimized I-CreI variant/HBB5.3 target

<400>  300

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Lys Pro Leu His Asn Phe Leu Thr Gln Leu Arg Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  301
<211>  167
<212>  PRT
<213>  artificial sequence

<220>
<223>  optimized I-CreI variant/HBB5.3 target

<400>  301

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Ser Lys Phe Lys His Lys Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  302
<211>  167
<212>  PRT
<213>  artificial sequence

<220>

<223>  optimized I-CreI variant/HBB5.3 target

<400>  302

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Ser Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Ala Val Arg Ala Val Leu Asp Ser Leu Ser Glu Lys Lys
145                 150                 155                 160

Lys Ser Ser Pro Ala Ala Asp
                165


<210>  303
<211>  167
<212>  PRT
<213>  artificial sequence

<220>
<223>  optimized I-CreI variant/HBB5.3 target

<400>  303

Met Ala Asn Thr Lys Tyr Asn Lys Glu Phe Leu Leu Tyr Leu Ala Gly
1               5                   10                  15

Leu Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Arg Leu Ser
65                  70                  75                  80

Glu Ile Lys Pro Leu His Asn Leu Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Arg
145                 150                 155                 160

Lys Ser Ser Pro Ala Ala Asp
                165

<210>  304
<211>  167
<212>  PRT
<213>  artificial sequence

<220>
<223>  optimized I-CreI variant/HBB5.3 target

<400>  304

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Asn Gln
            20                  25                  30

Ser Arg Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Glu Ile Glu Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Pro Pro Ala Ala Asp
                165

<210>  305
<211>  167
<212>  PRT
<213>  artificial sequence

<220>
<223>  optimized I-CreI variant/HBB5.3 target

<400>  305

Met Ala Asn Thr Lys Tyr Asn Lys Glu Phe Leu Leu Tyr Leu Ala Gly
1               5                   10                  15

Phe Val Asp Gly Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asp Tyr Gln Leu Ser
65                  70                  75                  80

Glu Ile Lys Pro Leu His Asn Phe Leu Thr Gln Leu Gln Pro Phe Leu
                85                  90                  95

Lys Leu Lys Gln Lys Gln Ala Asn Leu Ala Leu Lys Ile Ile Glu Gln
            100                 105                 110

Leu Pro Ser Ala Lys Glu Ser Pro Asp Lys Phe Leu Glu Val Cys Thr

115                 120                 125

Trp Val Asp Gln Val Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165

<210>  306
<211>  167
<212>  PRT
<213>  artificial sequence

<220>
<223>  optimized I-CreI variant/HBB5.3 target

<400>  306

Met Ala Asn Thr Lys Tyr Asn Lys Glu Phe Leu Leu Tyr Leu Ala Gly
1               5                   10                  15

Phe Val Asp Ser Asp Gly Ser Ile Ile Ala Gln Ile Lys Pro Ser Gln
            20                  25                  30

Ser His Lys Phe Lys His Arg Leu Ser Leu Thr Phe Lys Val Thr Gln
        35                  40                  45

Lys Thr Gln Arg Arg Trp Phe Leu Asp Lys Leu Val Asp Glu Ile Gly
    50                  55                  60

Val Gly Tyr Val Tyr Asp Ser Gly Ser Val Ser Asn Tyr Gln Leu Ser
65                  70                  75                  80

Lys Ile Lys Pro Leu His Asn Phe Leu Thr Gln Leu Gln Pro Phe Leu
                85                  90                  95

Lys Leu Lys Gln Lys Gln Ala Asn Leu Val Leu Lys Ile Ile Glu Gln
            100                 105                 110

Leu Pro Ser Ala Lys Glu Ser Pro Asp Lys Phe Leu Glu Val Cys Thr
        115                 120                 125

Trp Val Asp Gln Ile Ala Ala Leu Asn Asp Ser Lys Thr Arg Lys Thr
    130                 135                 140

Thr Ser Glu Thr Val Arg Ala Val Leu Asp Ser Leu Ser Glu Lys Lys
145                 150                 155                 160

Lys Ser Ser Pro Ala Ala Asp
                165